(19) Europäisches Patentamt
European Patent Office
Office européen des brevets

(11) EP 4 421 103 A1

(12) # EUROPEAN PATENT APPLICATION
published in accordance with Art. 153(4) EPC

(43) Date of publication:
**28.08.2024 Bulletin 2024/35**

(21) Application number: **22883324.0**

(22) Date of filing: **30.09.2022**

(51) International Patent Classification (IPC):
***C08G 61/12*** *(2006.01)* ***C07B 61/00*** *(2006.01)*
***C07D 513/04*** *(2006.01)* ***C07F 7/10*** *(2006.01)*
***C07F 7/22*** *(2006.01)* ***H10K 30/50*** *(2023.01)*

(52) Cooperative Patent Classification (CPC):
**C07B 61/00; C07D 513/04; C07F 7/10; C07F 7/22; C08G 61/12; H10K 30/50;** Y02E 10/549

(86) International application number:
**PCT/JP2022/036770**

(87) International publication number:
**WO 2023/068017 (27.04.2023 Gazette 2023/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.10.2021 JP 2021170410**

(71) Applicant: **Toyobo Co., Ltd.**
**Kita-ku**
**Osaka-shi, Osaka 530-0001 (JP)**

(72) Inventors:
- **HIRATA, Akari**
  **Otsu-shi, Shiga 520-0292 (JP)**
- **IMANISHI, Yoshiki**
  **Otsu-shi, Shiga 520-0292 (JP)**
- **HAGIYA, Kazutake**
  **Otsu-shi, Shiga 520-0292 (JP)**
- **TANAKA, Hikaru**
  **Otsu-shi, Shiga 520-0292 (JP)**
- **SAKAMOTO, Yasuhiro**
  **Otsu-shi, Shiga 520-0292 (JP)**

(74) Representative: **Müller-Boré & Partner Patentanwälte PartG mbB**
**Friedenheimer Brücke 21**
**80639 München (DE)**

(54) **ORGANIC SEMICONDUCTOR MATERIAL**

(57) Providing a macromolecular compound for providing an organic semiconductor material exhibiting excellent conversion efficiency; a starting-material compound having high material design freedom; and methods for producing thereof.

The macromolecular compound comprising a benzobisthiazole structural unit represented by the formula (1):

[Chemical Formula 1]

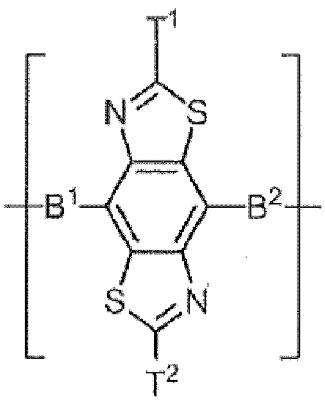

(1)



EP 4 421 103 A1

[in the formula (1), $T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

## Description

### TECHNICAL FIELD

The present invention relates to: a macromolecular compound comprising a structural unit having a specific benzobisthiazole backbone and a hetero ring to which one or more halogen atoms have been bound; an organic semiconductor material; a method for production thereof; an organic electronic device including the organic semiconductor material; and a solar cell module including the organic electronic device.

### BACKGROUND ART

Organic semiconductor materials are one of the most important materials in the field of organic electronics, and can be classified into electron-donating p-type organic semiconductor materials and electron-accepting n-type organic semiconductor materials. Various semiconductor elements can be produced by appropriately combining p-type organic semiconductor materials and n-type organic semiconductor materials, and these elements are applied to, for example, organic electroluminescences which emit light under the action of excitons formed by recombination of electrons and holes, organic thin-film solar cells which convert light into electric power, and organic thin-film transistors which control an amperage and a voltage.

Among them, organic thin-film solar cells are useful for environmental conservation because they do not release carbon dioxide into the air, and also, organic thin-film solar cells are easily produced because they have a simple structure. Therefore, the demand of organic thin-film solar cells is increasing. However, the photoelectric conversion efficiency of the organic thin-film solar cell is not sufficient yet. The photoelectric conversion efficiency $\eta$ is a value calculated as a product of a short-circuit current density (Jsc), an open circuit voltage (Voc) and a fill factor (FF) "$\eta$ = open circuit voltage (Voc) $\times$ short circuit current density (Jsc) $\times$ fill factor (FF)", and for improving the photoelectric conversion efficiency, it is necessary to improve the short-circuit current density (Jsc) and the fill factor (FF) as well as the open circuit voltage (Voc).

The open circuit voltage (Voc) is proportional to a difference in energy between the HOMO (highest occupied molecular orbital) level of a p-type organic semiconductor and the LUMO (lowest unoccupied molecular orbital) level of a n-type organic semiconductor, and therefore for improving the open circuit voltage (Voc), it is necessary to deepen (lower) the HOMO level of the p-type organic semiconductor.

The short-circuit current density (Jsc) correlates to the amount of energy received by an organic semiconductor material, and for improving the short-circuit current density (Jsc) of the organic semiconductor material, it is necessary for the organic semiconductor material to absorb light in a wide wavelength range extending from a visible region to a near-infrared region. The wavelength of a light having the lowest energy in the light that can be absorbed by the organic semiconductor material (the longest wavelength) is an absorption edge wavelength, and the energy corresponding to this wavelength is equal to band gap energy. Accordingly, for the organic semiconductor material to absorb light in a wider wavelength range, it is necessary to narrow the band gap (difference in energy between the HOMO level and the LUMO level of the p-type organic semiconductor).

On the other hand, in Patent Document 1, a compound having a benzobisthiazole backbone is proposed, but the conversion efficiency (PCE) is 2.8% at most and is not sufficient.

### PRIOR ART DOCUMENT

### PATENT DOCUMENT

Patent Document 1: JP-A-2017-157782

### SUMMARY OF THE INVENTION

### PROBLEMS TO BE SOLVED BY THE INVENTION

An object of the present invention is to provide an organic semiconductor material having excellent conversion efficiency. Since in an organic semiconductor material, the chemical structure and the conversion efficiency are closely related to each other, another object of the present invention is to provide a raw material compound capable of introducing more diverse backbones and substituents. Still another object of the present invention is to provide a method for producing the organic semiconductor material and the raw material compound for the organic semiconductor material.

SOLUTIONS TO THE PROBLEMS

The present inventors have found that for improving the conversion efficiency, i.e. improving both the open circuit voltage (Voc) and the short-circuit current density (Jsc), it is useful to moderately deepen the HOMO level while causing a p-type organic semiconductor to absorb light in a wide wavelength range. The present inventors have extensively conducted studies with attention paid to a correlation between the conversion efficiency and the chemical structure in the p-type organic semiconductor material, and resultantly learned that by using an organic semiconductor polymer having a specific structure, light with a wide range of wavelengths in the whole visible light region is absorbed, and the HOMO level and the LUMO level can be adjusted to be in an appropriate range, so that both the open circuit voltage (Voc) and the short-circuit current density (Jsc) can be improved. The present inventors have found that when the organic semiconductor polymer having good conversion efficiency (PCE) is used, charge separation can easily occur between a p-type organic semiconductor and a n-type organic semiconductor, leading to completion of the present invention.

Thus, a macromolecular compound according to the present invention comprising a benzobisthiazole structural unit represented by the formula (1):

[Chemical Formula 1]

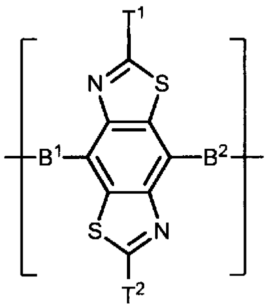

(1)

[in the formula (1),

$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

In the formula (1), $T^1$ and $T^2$ are each preferably a group represented by the following formula (t1).

[Chemical Formula 2]

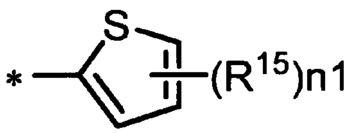

(t1)

[in the formula (t1),

$R^{15}$ represents a hydrogen atom, a hydrocarbon group with a carbon number of 6 to 30, a halogen atom, or a group represented by *-Si($R^{18}$)$_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different;
n1 represents an integer of 1 to 3;
a plurality of $R^{15}$s may be same or different; and
asterisk symbol (*) represents a bond].

In the formula (1), $B^1$ and $B^2$ are each preferably a group represented by the following formula (b1).

[Chemical Formula 3]

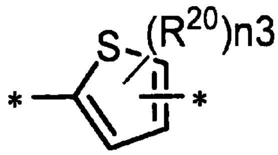

(b1)

[in the formula (b1),

$R^{20}$ represents a halogen atom or a hydrocarbon group with a carbon number of 6 to 30;
n3 represents an integer of 0 to 2;
a plurality of $R^{20}$s may be same or different; and
asterisk symbols (*) each represent a bond, and in particular, asterisk symbol (*) on the left side represents a bond to a benzene ring of a benzobisthiazole compound].

In the formula (1), the present invention includes the macromolecular compound, wherein the heteroaryl groups represented by $T^1$ and $T^2$ are each substituted by one or more halogen atoms.

In the formula (1), the present invention includes the macromolecular compound, wherein the heteroaryl groups represented by $B^1$ and $B^2$ are each substituted by one or more halogen atoms.

The macromolecular compound according to the present invention is preferably a donor-acceptor-type semiconductor polymer. An organic semiconductor material comprising the macromolecular compound according to the present invention is also encompassed in the technical scope of the present invention.

The present invention includes a benzobisthiazole compound represented by the formula (5):

[Chemical Formula 4]

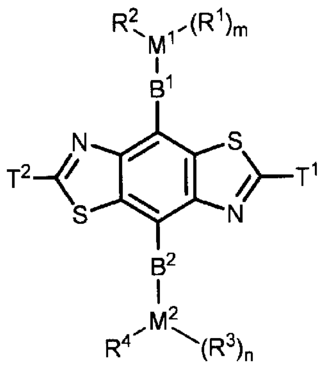

(5)

[in the formula (5),

$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom;
$R^1$ to $R^4$ each independently represent an aliphatic hydrocarbon group with a carbon number of 1 to 6, a hydroxy group, an alkoxy group with a carbon number of 1 to 6, or an aryloxy group with a carbon number of 6 to 10;
$M^1$ and $M^2$ each independently represent a boron atom or a tin atom;
$R^1$ and $R^2$ may form a ring with $M^1$, and $R^3$ and $R^4$ may form a ring with $M^2$;
m and n each represent an integer of 1 or 2; and
when m is 2, a plurality of $R^1$s may be same or different, and when n is 2, a plurality of $R^3$s may be same or different].

The present invention also includes a benzobisthiazole compound represented by the formula (4):

[Chemical Formula 5]

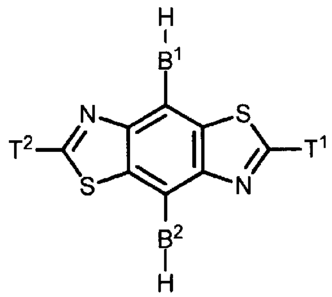

(4)

[in the formula (4),
$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

The present invention furthermore includes a benzobisthiazole compound represented by the formula (3):

[Chemical Formula 6]

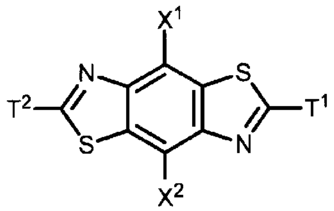

(3)

[in the formula (3),

$T^1$ and $T^2$ represent heteroaryl groups, the heteroaryl groups are optionally each independently substituted by an organosilyl group, a halogen atom, or a hydrocarbon group, and at least one of the heteroaryl groups represented by $T^1$ and $T^2$ is substituted by a halogen atom; and
$X^1$ and $X^2$ each represent a halogen atom].

The present invention includes a benzobisthiazole compound represented by the formula (2):

[Chemical Formula 7]

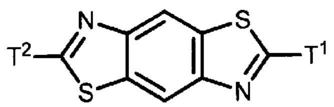

(2)

[in the formula (2),
$T^1$ and $T^2$ represent heteroaryl groups, the heteroaryl groups are optionally each independently substituted by an organosilyl group, a halogen atom, or a hydrocarbon group, and at least one of the heteroaryl groups represented by $T^1$ and $T^2$ is substituted by a halogen atom].

The present invention includes a thiophene compound represented by the formula (20):

[Chemical Formula 8]

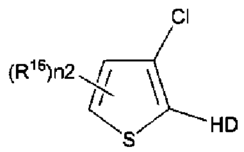

(20)

[in the formula (20),

HD represents a hexyldecyl group;
$R^{16}$s each independently represent a hydrogen atom, a hydrocarbon group with a carbon number of 6 to 30, a halogen atom, or a group represented by *-Sn($R^{18}$)$_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different;
n2 represents 1 or 2;
a plurality of $R^{16}$s may be same or different; and
asterisk symbol (*) represents a bond].

The present invention includes a thiophene compound represented by the formula (21):

[Chemical Formula 9]

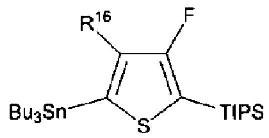

(21)

[in the formula (21),

TIPS represents a triisopropylsilyl group;
BusSn represents a tributyltin group;
$R^{16}$ represents a hydrogen atom, a hydrocarbon group with a carbon number of 6 to 30, a halogen atom, or a group represented by *-Sn($R^{18}$)$_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different; and
asterisk symbol (*) represents a bond].

A production method for the macromolecular compound according to the present invention comprising:

using one or more compounds selected from the group consisting of 2,6-diiodobenzo[1,2-d:4,5-d']bisthiazole and 2,6-dibromobenzo[1,2-d:4,5-d']bisthiazole as starting materials; and
going through
a compound represented by the formula (22):

[Chemical Formula 10]

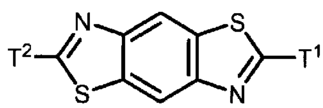

(22)

[in the formula (22),

$T^1$ and $T^2$ represent heteroaryl groups, and the heteroaryl groups are optionally each independently substituted by

an organosilyl group, a halogen atom, or a hydrocarbon group];
a compound represented by the formula (23):

[Chemical Formula 11]

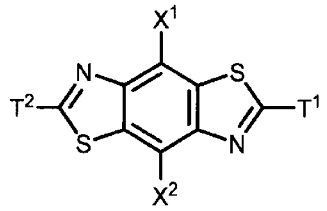

(2 3)

[in the formula (23),

$T^1$ and $T^2$ represent heteroaryl groups, and the heteroaryl groups are optionally each independently substituted by an organosilyl group, a halogen atom, or a hydrocarbon group; and
$X^1$ and $X^2$ each represent a halogen atom]; and
a compound represented by the formula (4):

[Chemical Formula 5]

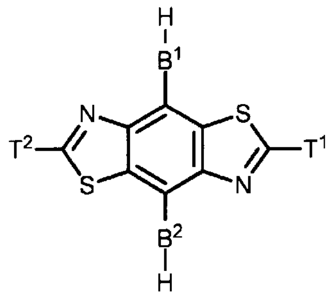

(4)

[in the formula (4),
$T^1$, $T^2$, $B^1$, and $B^2$ each represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

The production method for the macromolecular compound according to the present invention includes preferably the following first step, second step (a), and third step (hereinafter referred to as route A), or the following first step, second step (b-1), second step (b-2), and third step (hereinafter referred to as route B). The macromolecular compound of the present invention can be synthesized by either the route A or the route B. It is preferably the route B when at least one of the heteroaryl groups represented by $T^1$ and $T^2$ is substituted by a halogen atom, and it is preferably the route A when neither the heteroaryl groups represented by $T^1$ and $T^2$ is substituted by a halogen atom.

First, the route A is described. The route A includes the following first step, second step (a), and third step:

the first step: a step of reacting a compound represented by the formula (6) and/or the formula (7) with one compound selected from the group consisting of 2,6-diiodobenzo[1,2-d:4,5-d']bisthiazole and 2,6-dibromobenzo[1,2-d:4,5-d']bisthiazole in the presence of a metal catalyst to prepare a compound represented by the formula (22):
[Chemical Formula 12]

$T^1$-$R^5$ (6)

$T^2$-$R^6$ (7)

[in the formulae (6) and (7),
$T^1$ and $T^2$ each represent heteroaryl groups, and the heteroaryl groups are optionally each independently substituted by an organosilyl group, a halogen atom, or a hydrocarbon group;

$R^5$ and $R^6$ each independently represent a hydrogen atom or *-$M^3(R^7)_kR^8$;
$R^7$ and $R^8$ each independently represent an aliphatic hydrocarbon group with a carbon number of 1 to 6, a hydroxy group, an alkoxy group with a carbon number of 1 to 6, or an aryloxy group with a carbon number of 6 to 10;
$M^3$ represents a boron atom or a tin atom;
asterisk symbol (*) represents a bond;
$R^7$ and $R^8$ may form a ring with $M^3$;
k represents an integer of 1 or 2; and
when k is 2, a plurality of $R^7$s may be same or different];
the second step (a): a step of reacting a base and a halogenation reagent with the compound represented by the formula (22) to prepare a compound represented by the formula (23); and
the third step: a step of reacting a compound represented by the following formula (8) and/or formula (9) with the compound represented by the formula (23) in the presence of a metal catalyst to prepare a compound represented by the formula (4):

[Chemical Formula 13]

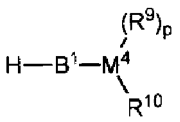

(8)

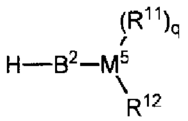

(9)

[in the formulae (8) and (9),
$B^1$ and $B^2$ represent heteroaryl groups, and the heteroaryl groups are optionally each independently substituted by an organosilyl group, a halogen atom, or a hydrocarbon group;
$R^9$ to $R^{12}$ each independently represent an aliphatic hydrocarbon group with a carbon number of 1 to 6, a hydroxy group, an alkoxy group with a carbon number of 1 to 6, an aryl group with a carbon number of 6 to 10, or an aryloxy group with a carbon number of 6 to 10;
$M^4$ and $M^5$ each represent a boron atom, a tin atom, or a silicon atom;
$R^9$ and $R^{10}$ may form a ring with $M^4$, and $R^{11}$ and $R^{12}$ may form a ring with $M^5$;
p and q each represent an integer of 1 or 2; and
when p is 2, a plurality of $R^9$s may be same or different, and when q is 2, a plurality of $R^{11}$s may be same or different].

Next, the route B is described. The route B includes the first step, second step (b-1), second step (b-2), and the third step. The first step is the same first step of the route A and the third step is the same third step of the route A:

the second step (b-1): a step of reacting a base and an organosilylation reagent with the compound represented by the formula (22);
the second step (b-2): a step of reacting a base and a halogenation reagent with the product resulting from the second step (b) to prepare a compound represented by the formula (3).

Preferably, the production method for the macromolecular compound according to the present invention further goes through a compound represented by the formula (5):

[Chemical Formula 4]

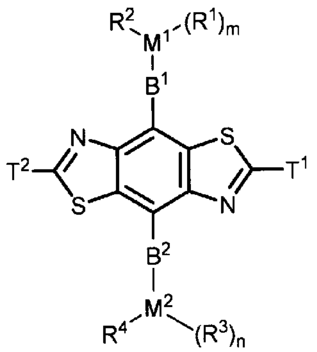

(5)

[in the formula (5),
$T^1$, $T^2$, $B^1$, $B^2$, $R^1$ to $R^4$, $M^1$, $M^2$, m, and n each represent the same groups as above. However, at least one of the heteroaryl groups represented by $T^1$, $T^2$, $B^1$, and $B^2$ is substituted by a halogen atom].

Preferably, the production method for the macromolecular compound according to the present invention further includes the following fourth step:
the fourth step: a step of reacting a base and a tin halide compound with the compound represented by the formula (4) to prepare a compound represented by the formula (5).

EFFECTS OF THE INVENTION

The halogen-containing benzobisthiazole compound according to the present invention can form a planar cross-shaped backbone under intramolecular S-N and halogen atom-heteroatom interactions. As a result, a π-conjugation is extended in the planar cross-shaped backbone, and therefore the benzobisthiazole compound has multi-band light absorptions derived from a plurality of π-π* transitions, and can absorb light in a wide wavelength range extending from a visible region to a near-infrared region. In addition, the HOMO level and the LUMO level can each be adjusted to be in an appropriate range owing to the halogen atoms. Accordingly, both a high open circuit voltage (Voc) and a high short-circuit current density (Jsc) can be achieved, so that a high conversion efficiency (PCE) can be achieved. According to a production method according to the present invention, various substituents can be introduced into the benzobisthiazole backbone, so that the properties (e.g. crystallinity, film formability and absorption wavelength) of a material can be controlled. Furthermore, the present invention makes it possible to provide: an organic semiconductor material including the above macromolecular compound; an organic electronic device including the organic semiconductor material; and a solar cell module including the organic electronic device.

BRIEF DESCRIPTION OF THE DRAWINGS

Fig. 1 shows an ultraviolet visible absorption spectrum of a macromolecular compound in Example 1.
Fig. 2 shows an ultraviolet visible absorption spectrum of a macromolecular compound in Example 2.
Fig. 3 shows an ultraviolet visible absorption spectrum of a macromolecular compound in Example 5.

MODE FOR CARRYING OUT THE INVENTION

1. Macromolecular compound

The macromolecular compound according to the present invention comprises a benzobisthiazole structural unit represented by the formula (1).

[Chemical Formula 1]

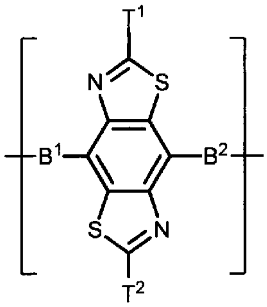

(1)

[in the formula (1),
$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by T', $T^2$, $B^1$, and $B^2$ is substituted by a halogen atom].

The macromolecular compound according to the present invention has a benzobisthiazole structural unit represented by the formula (1), so that the band gap can be narrowed while the HOMO level is deepened. Thus, the macromolecular compound is advantageous for improving the conversion efficiency (PCE). The macromolecular compound according to the present invention is preferably a donor-acceptor-type semiconductor polymer. The donor-acceptor-type semiconductor macromolecular compound means a macromolecular compound in which donor units and acceptor units are alternately arranged. The donor unit means an electron-donating structural unit, and the acceptor unit means an electron-accepting structural unit. The donor-acceptor-type semiconductor polymer is preferably a macromolecular compound in which structural units represented by the formula (1) and other structural units are alternately arranged. By forming such a structure, the donor units and the acceptor units can form regular bonds in the main chain of the macromolecule, and thus a charge-transfer $\pi$-conjugated system in which charge transfer between the units is spread throughout the entire main chain via a $\pi$-conjugated chain can be formed. Charge transfer occurring in molecules is considered to be more advantageous than charge transfer occurring between the molecules. Thus, if charge transfer can be caused to occur in molecules, the power generation efficiency can be expected to be further improved. In addition, owing to such intramolecular charge transfer, the absorption wavelength band can be widened, and the macromolecular compound can be suitably used as a p-type semiconductor compound. In this specification, the organosilyl group means a monovalent group in which a Si atom is substituted with one or more hydrocarbon groups, and the number of hydrocarbon groups with which the Si atom is substituted is preferably 2 or more, further preferably 3.

In the formula (1), at least one of the heteroaryl groups represented by $T^1$, $T^2$, $B^1$, and $B^2$ is substituted by a halogen atom. It is preferable that the heteroaryl groups represented by $T^1$ and $T^2$ are each substituted by one or more halogen atoms. It is also preferable that the heteroaryl groups represented by $B^1$ and $B^2$ are each substituted by one or more halogen atoms. The upper limits of the numbers of the halogen atoms are not particularly limited. However, the numbers of the halogen atoms by which $T^1$ and $T^2$ are substituted are each independently preferably 3 or smaller, more preferably 2 or smaller, and further preferably 1. The numbers of the halogen atoms by which $B^1$ and $B^2$ are substituted are each independently preferably 2 or smaller and more preferably 1. By halogen atoms, both $T^1$ and $T^2$ may be substituted alone, both $B^1$ and $B^2$ may be substituted alone, or all of $T^1$, $T^2$, $B^1$, and $B^2$ may be substituted. It is preferable that: both $T^1$ and $T^2$ are substituted alone; or both $B^1$ and $B^2$ are substituted alone. Specific examples of such macromolecular compounds include: a macromolecular compound in which $T^1$ and $T^2$ are each substituted by one halogen atom, and neither $B^1$ nor $B^2$ is substituted by any halogen atom; a macromolecular compound in which $B^1$ and $B^2$ are each substituted by one halogen atom, and neither $T^1$ nor $T^2$ is substituted by any halogen atom; and a macromolecular compound in which all of $T^1$, $T^2$, $B^1$, and $B^2$ are each substituted by one halogen atom. The halogen atoms by which the respective heteroaryl groups are substituted may be equal to one another or different from one another. However, from the viewpoint of facilitating production, the halogen atoms by which $T^1$ and $T^2$ are substituted are preferably equal to each other, and the halogen atoms by which $B^1$ and $B^2$ are substituted are preferably equal to each other.

In the benzobisthiazole structural unit represented by the formula (1), $T^1$ and $T^2$ may be mutually same or different, and they are preferably the same from the viewpoint of ease of production.

In the benzobisthiazole structural unit represented by the formula (1), $T^1$ and $T^2$ are each preferably thiophene rings, and the thiophene rings may be optionally substituted by a hydrocarbon group, a halogen atom, or an organosilyl group. Specifically, $T^1$ and $T^2$ are each preferably a group represented by the following formula (t1). When $T^1$ and $T^2$ are a group represented by the following formula (t1), light having a short wavelength can be absorbed, and high flatness is achieved, so that a $\pi$-$\pi$ stacking is efficiently formed, and therefore the conversion efficiency (PCE) can be further improved.

[Chemical Formula 2]

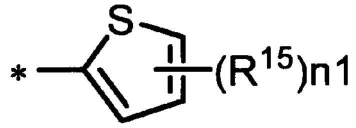

(t1)

[in the formula (t1),

$R^{15}$ represents a hydrogen atom, a hydrocarbon group with a carbon number of 6 to 30, a halogen atom, or a group represented by *-Si($R^{18}$)$_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different;
n1 represents an integer of 1 to 3;
a plurality of $R^{15}$s may be same or different; and
asterisk symbol (*) represents a bond].

In the above formula (t1), the hydrocarbon groups with a carbon number of 6 to 30 as $R^{15}$ are preferably a branched hydrocarbon group, more preferably a branched chain saturated hydrocarbon group. When the hydrocarbon groups of $R^{15}$ are branched, the solubility in an organic solvent can be increased, so that moderate crystallinity can be imparted to the macromolecular compound according to the present invention. The solubility in an organic solvent can be increased as the carbon number of the hydrocarbon groups of $R^{15}$ becomes larger, but when the carbon number is excessively large, reactivity in a coupling reaction as described later is reduced, and therefore it is difficult to synthesize a macromolecular compound. Accordingly, the carbon number of the hydrocarbon groups of $R^{15}$ is preferably 8 to 25, more preferably 8 to 20, further preferably 8 to 16.

Examples of the hydrocarbon groups with a carbon number of 6 to 30, which are represented by $R^{15}$ include alkyl groups with the carbon number of 6 such as n-hexyl group; alkyl groups with the carbon number of 7 such as n-heptyl group; alkyl groups with the carbon number of 8 such as n-octyl group, 1-n-butylbutyl group, 1-n-propylpentyl group, 1-ethylhexyl group, 2-ethylhexyl group, 3-ethylhexyl group, 4-ethylhexyl group, 1-methylheptyl group, 2-methylheptyl group, 6-methylheptyl group, 2,4,4-trimethylpentyl group and 2,5-dimethylhexyl group; alkyl groups with the carbon number of 9 such as n-nonyl group, 1-n-propylhexyl group, 2-n-propylhexyl group, 1-ethylheptyl group, 2-ethylheptyl group, 1-methyloctyl group, 2-methyloctyl group, 6-methyloctyl group, 2,3,3,4-tetramethylpentyl group and 3,5,5-trimethylhexyl group; alkyl groups with the carbon number of 10 such as n-decyl group, 1-n-pentylpentyl group, 1-n-butylhexyl group, 2-n-butylhexyl group, 1-n-propylheptyl group, 1-ethyloctyl group, 2-ethyloctyl group, 1-methylnonyl group, 2-methylnonyl group and 3,7-dimethyloctyl group; alkyl groups with the carbon number of 11 such as n-undecyl group, 1-n-butylheptyl group, 2-n-butylheptyl group, 1-n-propyloctyl group, 2-n-propyloctyl group, 1-ethylnonyl group and 2-ethylnonyl group; alkyl groups with the carbon number of 12 such as n-dodecyl group, 1-n-pentylheptyl group, 2-n-pentylheptyl group, 1-n-butyloctyl group, 2-n-butyloctyl group, 1-n-propylnonyl group and 2-n-propylnonyl group; alkyl groups with the carbon number of 13 such as n-tridecyl group, 1-n-pentyloctyl group, 2-n-pentyloctyl group, 1-n-butylnonyl group, 2-n-butylnonyl group, 1-methyldodecyl group and 2-methyldodecyl group; alkyl groups with the carbon number of 14 such as n-tetradecyl group, 1-n-heptylheptyl group, 1-n-hexyloctyl group, 2-n-hexyloctyl group, 1-n-pentylnonyl group and 2-n-pentylnonyl group; alkyl groups with the carbon number of 15 such as n-pentadecyl group, 1-n-heptyloctyl group, 1-n-hexylnonyl group and 2-n-hexylnonyl group; alkyl groups with the carbon number of 16 such as n-hexadecyl group, 2-n-hexyldecyl group, 1-n-octyloctyl group, 1-n-heptylnonyl group and 2-n-heptylnonyl group; alkyl groups with the carbon number of 17 such as n-heptadecyl group and 1-n-octylnonyl group; alkyl groups with the carbon number of 18 such as n-octadecyl group and 1-n-nonylnonyl group; alkyl groups with the carbon number of 19 such as n-nonadecyl group; alkyl groups with the carbon number of 20 such as n-eicosyl group and 2-n-octyldodecyl group; alkyl groups with the carbon number of 21 such as n-heneicosyl group; alkyl groups with the carbon number of 22 such as n-docosyl group; alkyl groups with the carbon number of 23 such as n-tricosyl group; and alkyl groups with the carbon number of 24 such as n-tetracosyl group and 2-n-desyltetradesyl group. Alkyl groups with a carbon number of 8 to 20 are preferable, alkyl groups with a carbon number of 8 to 16 are more preferable, branched chain alkyl groups with a carbon number of 8 to 16 are further preferable, and 2-ethylhexyl group, 3,7-dimethyloctyl group, 2-n-butyloctyl group, 2-n-hexyldecyl group, 2-n-octyldodecyl group and 2-n-decyltetradecyl group are especially preferable. When $R^{15}$ is the above-mentioned group, the macromolecular compound according to the present invention has an increased solubility in an organic solvent,

and has moderate crystallinity.

In the groups represented by *-Si($R^{18}$)$_3$ as $R^{15}$ in the above formula (t1), the carbon number of the aliphatic hydrocarbon group of $R^{18}$ is preferably 1 to 18, more preferably 1 to 8. Examples of the aliphatic hydrocarbon group of $R^{18}$ include methyl group, ethyl group, isopropyl group, and tert-butyl group. The carbon number of the aromatic hydrocarbon group of $R^{18}$ is preferably 6 to 8, more preferably 6 or 7, especially preferably 6. The aromatic hydrocarbon group of $R^{18}$ is, for example, phenyl group. In particular, $R^{18}$ is preferably an aliphatic hydrocarbon group, especially preferably an methyl group. A plurality of $R^{18}$s may be same or different, and they are preferably the same. When $R^{15}$ is the group represented by *-Si($R^{18}$)$_3$, the macromolecular compound according to the present invention has an increased solubility in an organic solvent.

Specific examples of the groups represented by *-Si($R^{18}$)$_3$ as $R^{15}$ in the above formula (t1) include alkylsilyl groups such as trimethylsilyl group, ethyldimethylsilyl group, isopropyldimethylsilyl group, triisopropylsilyl group, tertbutyldimethylsilyl group, triethylsilyl group, triisobutylsilyl group, tripropylsilyl group, tributylsilyl group, dimethylphenylsilyl group and methyldiphenylsilyl group; and arylsilyl groups such as triphenylsilyl group and tert-butylchlorodiphenylsilyl group. Among them, alkylsilyl groups are preferable, and trimethylsilyl group and triisopropylsilyl group are especially preferable.

When $R^{15}$ is a halogen atom in the above formula (t1), any of fluorine, chlorine, bromine and iodine may be used. Among them, fluorine or chlorine is preferable.

The electron-donating groups of $T^1$ and $T^2$ are each more preferably a group represented by one of the formula (t1), especially preferably a group represented by one of the following formulae (t1-1) to (t1-11) from the viewpoint of excellent flatness as the whole of the structural unit represented by the formula (1). In the formulae, $R^{T'}$ and $R^{T''}$ may be the same or different and represent halogen atom, hydrogen atom or organosilyl group. In the formulae, asterisk symbol (*) represents a bond.

[Chemical Formula 14]

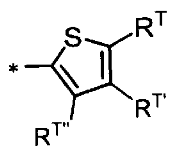

$R^T$ =

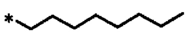 (t1-1)

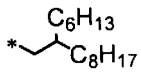 (t1-5)

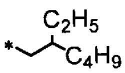 (t1-2)

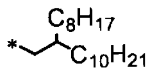 (t1-6)

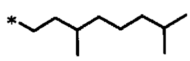 (t1-3)

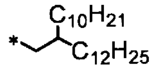 (t1-7)

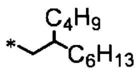 (t1-4)

[Chemical Formula 15]

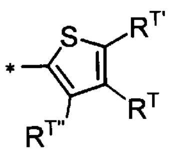

$R^T$ =

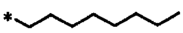 (t1-8)

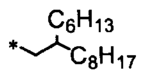 (t1-12)

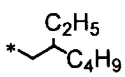 (t1-9)

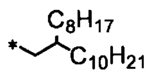 (t1-13)

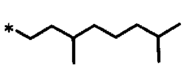 (t1-10)

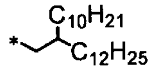 (t1-14)

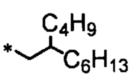 (t1-11)

In the benzobisthiazole structural unit represented by the formula (1), $B^1$ and $B^2$ may be mutually same or different, and they are preferably the same from the viewpoint of ease of the production. In the structural unit represented by the formula (1), $B^1$ and $B^2$ are each preferably thiophene rings, and the thiophene rings may be optionally substituted by a hydrocarbon group, a halogen atom, or an organosilyl group. Specifically, $B^1$ and $B^2$ are each preferably a group represented by the following formula (b1). When $B^1$ and $B^2$ are each a group represented by the following formula (b1), the resulting macromolecular compound has proper flatness, so that the conversion efficiency (PCE) can be further improved.

[Chemical Formula 3]

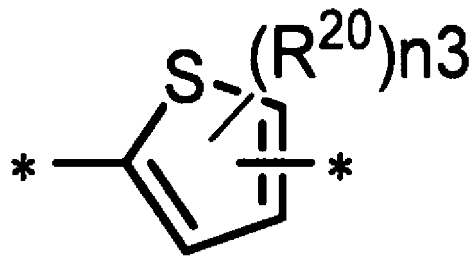

(b1)

[in the formula (b1),

$R^{20}$ represents a halogen atom or a hydrocarbon group with a carbon number of 6 to 30;
n3 represents an integer of 0 to 2;
a plurality of $R^{20}$s may be same or different; and
asterisk symbols (*) each represent a bond, and in particular, asterisk symbol (*) on the left side represents a bond to a benzene ring of a benzobisthiazole compound].

As the hydrocarbon groups with a carbon number of 6 to 30 as $R^{20}$, the groups shown as examples of hydrocarbon groups with a carbon number of 6 to 30 as $R^{15}$ may be preferably used.

It is preferable that $R^{20}$ is hydrogen atoms in that a donor-acceptor-type semiconductor polymer is easily formed. It is preferable that $R^{20}$ is hydrocarbon groups with a carbon number of 6 to 30 in that the conversion efficiency (PCE) may be further improved.

In the benzobisthiazole structural unit represented by the formula (1), $B^1$ and $B^2$ are each more preferably a group represented by one of the formula (b1) from the viewpoint of excellent flatness as the whole of the structural unit represented by the formula (1) as well as excellent flatness as the whole of the resulting macromolecular compound. When $B^1$ and $B^2$ are each a group represented by one of the formula (b1), the S atom and the N atom interact with each other in the benzobisthiazole structural unit (1), so that flatness is further improved. Specifically, $B^1$ and $B^2$ are each preferably a group represented by the following formula. However, in the formula, $R^B$ and $R^{B'}$ may be the same or different and represent a hydrocarbon group with 6 to 30 carbons, a halogen atom or a hydrogen atom. In the formula, asterisk symbol (*) represents a bond, and asterisk symbol (*) on the left side represents a bond to a benzene ring of a benzobisthiazole compound.

[Chemical Formula 16]

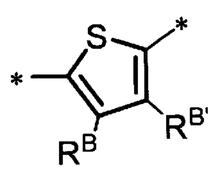

(b1-1)

Examples of the structural unit represented by the formula (1) include groups represented by the following formulae (1-1) to (1-42).

[Chemical Formula 17]

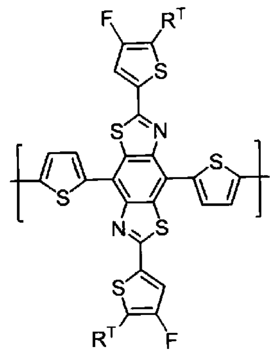

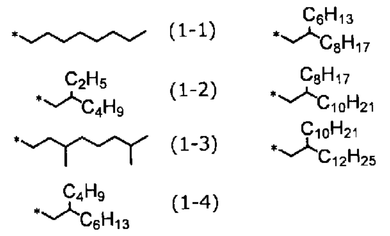

$R^T$ = (1-1) (1-2) (1-3) (1-4) (1-5) (1-6) (1-7)

[Chemical Formula 18]

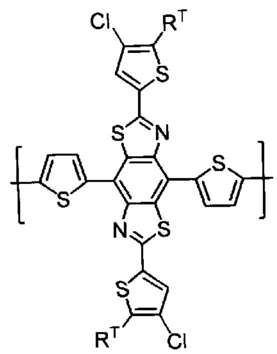

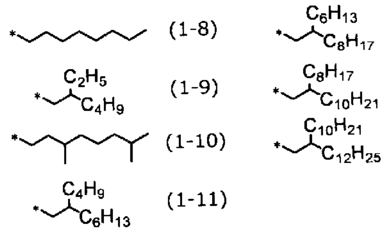

$R^T$ = (1-8) (1-9) (1-10) (1-11) (1-12) (1-13) (1-14)

[Chemical Formula 19]

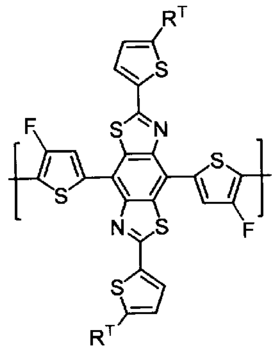

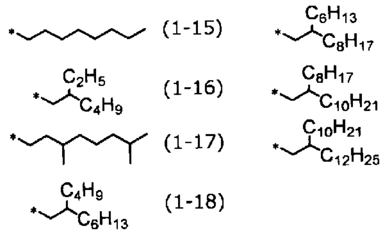

$R^T$ = (1-15) (1-16) (1-17) (1-18) (1-19) (1-20) (1-21)

[Chemical Formula 20]

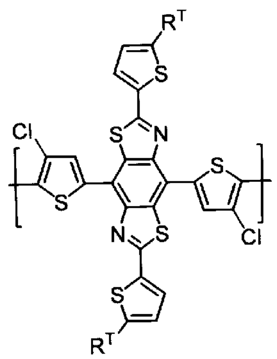

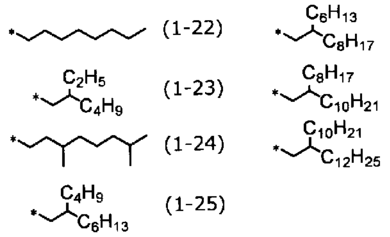

$R^T$ = (1-22) (1-23) (1-24) (1-25) (1-26) (1-27) (1-28)

[Chemical Formula 21]

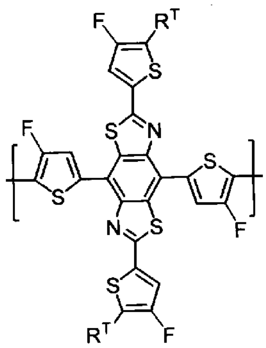

$R^T$ =

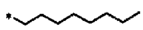 (1-29)
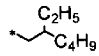 (1-30)
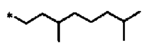 (1-31)
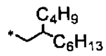 (1-32)
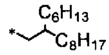 (1-33)
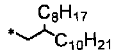 (1-34)
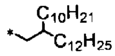 (1-35)

[Chemical Formula 22]

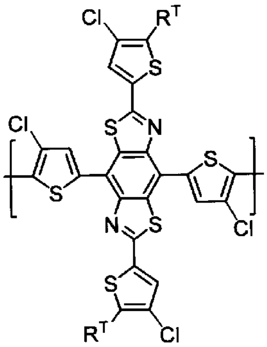

$R^T$ =

 (1-36)
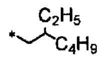 (1-37)
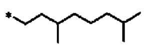 (1-38)
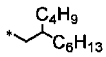 (1-39)
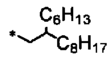 (1-40)
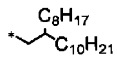 (1-41)
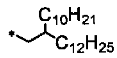 (1-42)

As a structural unit that is combined with a structural unit represented by the formula (1) to form a donor-acceptor-type semiconductor polymer (donor unit or acceptor unit), specific examples thereof may include the following structural units.

[Chemical Formula 23]

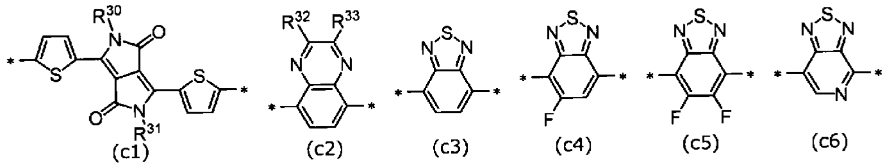

(c1) (c2) (c3) (c4) (c5) (c6)

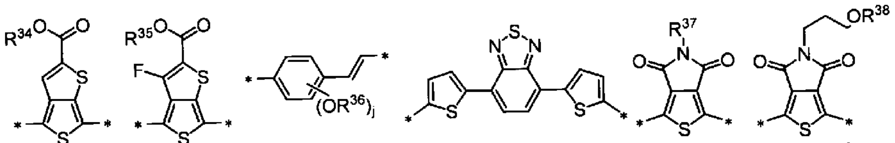

(c7) (c8) (c9) (c10) (c11) (c12)

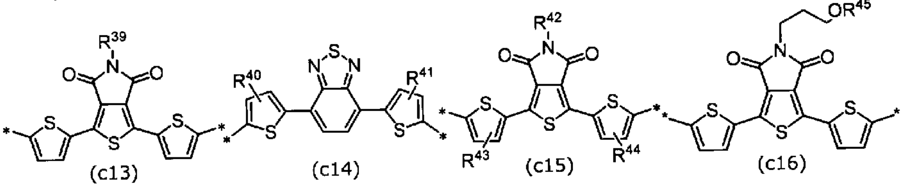

(c13) (c14) (c15) (c16)

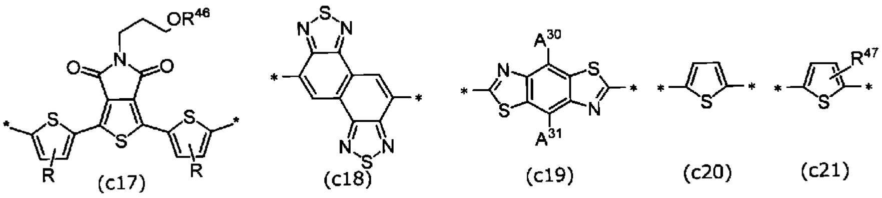

(c17) (c18) (c19) (c20) (c21)

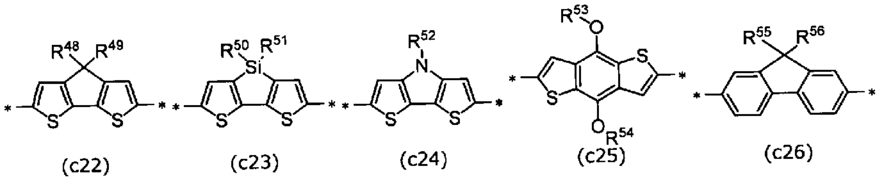

(c22) (c23) (c24) (c25) (c26)

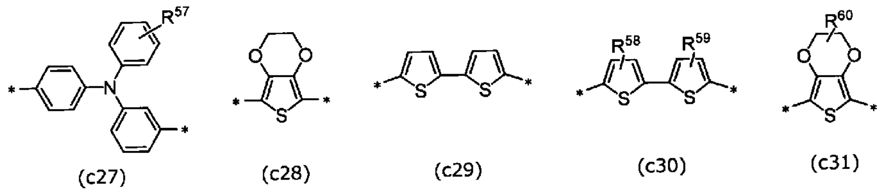

(c27) (c28) (c29) (c30) (c31)

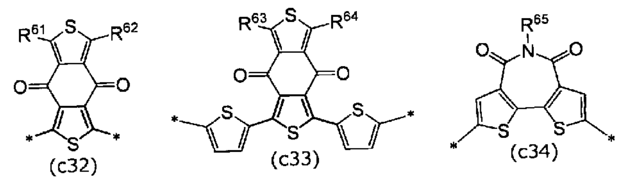

(c32) (c33) (c34)

[in the formulae (c1) to (c34), R and $R^{30}$ to $R^{65}$ each independently represent the same group as $R^{15}$; $A^{30}$ and $A^{31}$ each independently represent an alkoxy group, a thioalkoxy group, a thiophene ring optionally substituted by a hydrocarbon group or an organosilyl group, a thiazole ring optionally substituted by a hydrocarbon group or an organosilyl group, or a phenyl group optionally substituted by a hydrocarbon group, an alkoxy group, a thioalkoxy group, an organosilyl group, a halogen atom, or a trifluoromethyl group; and j represents an integer of 0 to 4].

The groups represented by the above formulae (c1) to (c18) and (c32) to (c34) are groups which serve as an acceptor unit, and the groups represented by the formulae (c20) to (c31) are groups which serve as a donor unit. The group represented by the formula (c19) may serve as an acceptor unit or serve as a donor unit depending on the type of $A^{30}$ and $A^{31}$.

The structural unit to be combined with the structural unit represented by the formula (1) is preferably a structure resulting from fusing heteroaryl groups and is further preferably a group that acts as an acceptor unit. Among them, a structure resulting from binding and/or fusing thiophene rings is more preferable, and a structure represented by the formula (c1), (c7), (c8), (c10) to (c17), or (c32) to (c34) is particularly preferable.

The weight average molecular weight and number average molecular weight of the macromolecular compound

according to the present invention are generally 2,000 or more and 500,000 or less, more preferably 3,000 or more and 200,000 or less. The weight average molecular weight and number average molecular weight of the macromolecular compound according to the present invention can be calculated based on a calibration curve prepared with polystyrene as a standard sample using gel permeation chromatography.

The ionization potential of the macromolecular compound according to the present invention is preferably 4 eV or more, more preferably 4.5 eV or more, further preferably 5 eV or more, especially preferably 5.1 eV or more. While the upper limit of the ionization potential is not particularly limited, it is, for example, 7 eV or less, preferably 6.5 eV or less, more preferably 6.2 eV or less. When the ionization potential of the macromolecular compound according to the present invention is in the above-mentioned range, the HOMO level is moderately deepened (lowered), and therefore both a high open circuit voltage (Voc) and a high short-circuit current density (Jsc) can be achieved, so that a higher conversion efficiency (PCE) can be achieved.

2. Compound 2-1. (Compound represented by formula (5))

The present invention includes a compound represented by the following formula (5) (hereinafter, sometimes referred to as a "compound (5)").

[Chemical Formula 4]

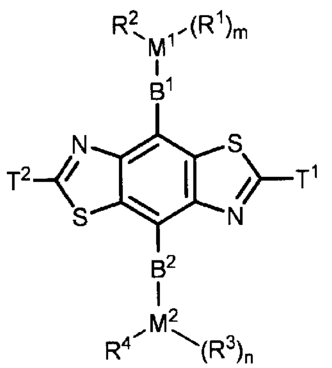

(5)

[in the formula (5),

$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom;
$R^1$ to $R^4$ each independently represent an aliphatic hydrocarbon group with a carbon number of 1 to 6, a hydroxy group, an alkoxy group with a carbon number of 1 to 6, or an aryloxy group with a carbon number of 6 to 10;
$M^1$ and $M^2$ each independently represent a boron atom or a tin atom;
$R^1$ and $R^2$ may form a ring with $M^1$, and $R^3$ and $R^4$ may form a ring with $M^2$;
m and n each represent an integer of 1 or 2; and
when m is 2, a plurality of $R^1$s may be same or different, and when n is 2, a plurality of $R^3$s may be same or different].

In the formula (5), $T^1$ and $T^2$ may be mutually same or different, and they are preferably the same from the viewpoint of ease of production. In the formula (5), $T^1$ and $T^2$ are each preferably a group represented by the above formula (t1).

In the formula (5), $B^1$ and $B^2$ may be mutually same or different, and they are preferably the same from the viewpoint of ease of production. In the formula (5), $B^1$ and $B^2$ are each preferably a group represented by the above formula (b1).

Regarding the halogen atoms by which the heteroaryl groups represented by $T^1$, $T^2$, $B^1$, and $B^2$ are substituted in the formula (5), see description about the halogen atoms in the formula (1).

In the formula (5), the carbon number of the aliphatic hydrocarbon groups of $R^1$ to $R^4$ is preferably 1 to 5, more preferably 1 to 4. The aliphatic hydrocarbon groups of $R^1$ to $R^4$ are each preferably methyl group, ethyl group, propyl group or butyl group, more preferably methyl group or butyl group. The carbon number of the alkoxy groups of $R^1$ to $R^4$ is preferably 1 to 3, more preferably 1 or 2. The alkoxy groups of $R^1$ to $R^4$ are each preferably methoxy group, ethoxy group, propoxy group or the like, more preferably methoxy group or ethoxy group. The carbon number of the aryloxy groups of $R^1$ to $R^4$ is preferably 6 to 9, more preferably 6 to 8. The aryloxy groups of $R^1$ to $R^4$ are each preferably

phenyloxy group, benzyloxy group and phenylenebis(methyleneoxy) group.

When $M^1$ and $M^2$ are boron atoms, $R^1$ to $R^4$ are each preferably hydroxyl group, an alkoxy group with a carbon number of 1 to 6, or an aryloxy group with a carbon number of 6 to 10, and m and n are each preferably 1. When $M^1$ and $M^2$ are boron atoms, examples of $*\text{-}M^1(R^1)_mR^2$ and $*\text{-}M^2(R^3)_nR^4$ include groups represented by the following formulae. Asterisk symbol (*) represents a bond.

[Chemical Formula 24]

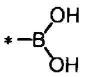 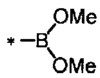 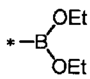 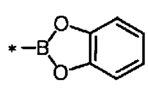 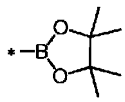 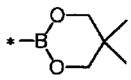

When $M^1$ and $M^2$ are tin atoms, $R^1$ to $R^4$ are each preferably an aliphatic hydrocarbon group with a carbon number of 1 to 6, m and n are each preferably 2. When $M^1$ and $M^2$ are tin atoms, examples of $*\text{-}M^1(R^1)_mR^2$ and $*\text{-}M^2(R^3)_nR^4$ include groups represented by the following formulae. Asterisk symbol (*) represents a bond.

[Chemical Formula 25]

$*-Sn(Me)_3$ $*-Sn(Bu)_3$

The compound (5) is an intermediate compound to be used for synthesis of the macromolecular compound according to the present invention. Since the compound (5) has the predetermined group described above, it has high temporal stability, and can efficiently react to form the macromolecular compound according to the present invention. Examples of the compound (5) may include compounds represented by the following formulae.

[Chemical Formula 28]

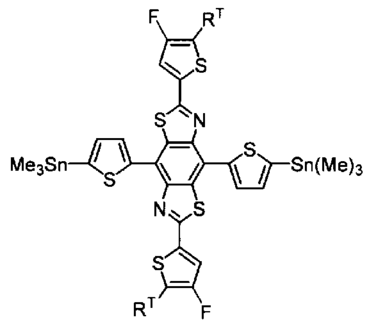

$R^T$ =  (5-1) 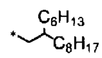 (5-5)

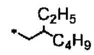 (5-2) 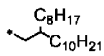 (5-6)

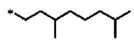 (5-3) 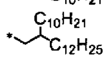 (5-7)

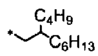 (5-4)

[Chemical Formula 29]

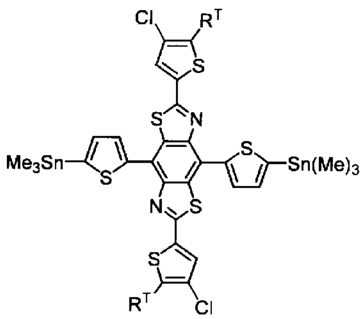

$R^T$ = 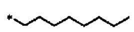 (5-8) 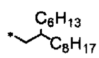 (5-12)

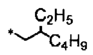 (5-9) 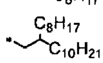 (5-13)

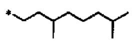 (5-10) 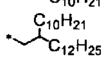 (5-14)

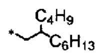 (5-11)

[Chemical Formula 30]

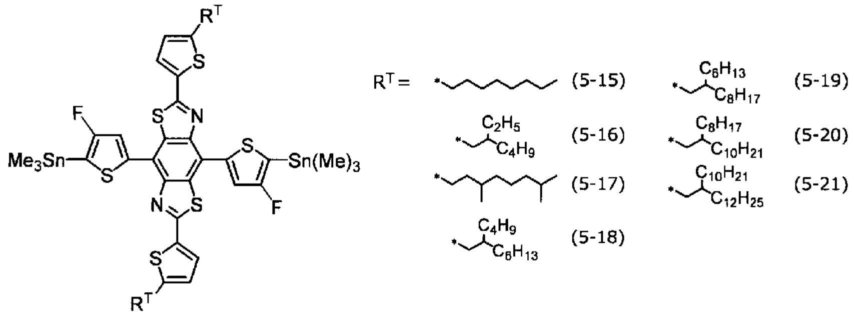

(5-15) (5-16) (5-17) (5-18) (5-19) (5-20) (5-21)

[Chemical Formula 31]

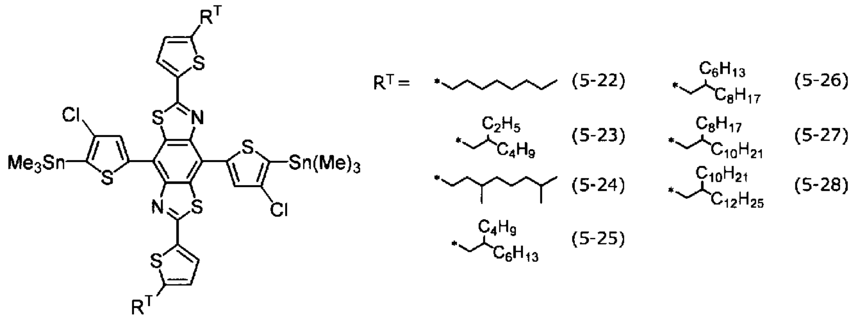

(5-22) (5-23) (5-24) (5-25) (5-26) (5-27) (5-28)

[Chemical Formula 32]

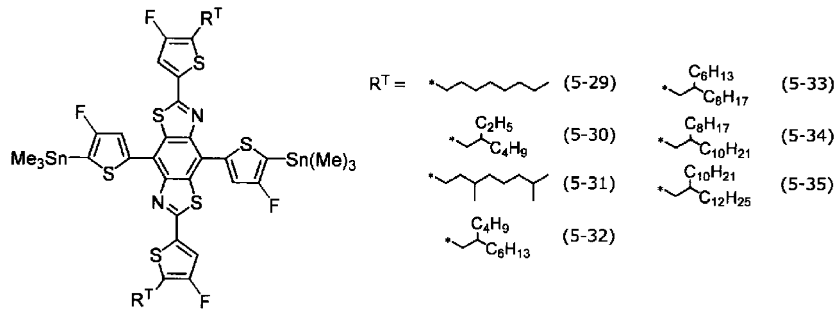

(5-29) (5-30) (5-31) (5-32) (5-33) (5-34) (5-35)

[Chemical Formula 33]

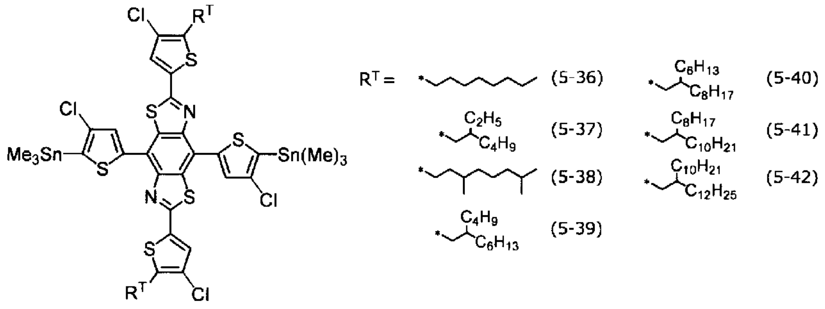

(5-36) (5-37) (5-38) (5-39) (5-40) (5-41) (5-42)

[Chemical Formula 34]

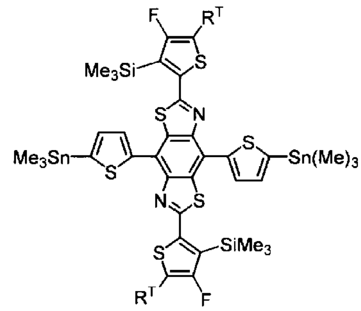

$R^T$ =

 (5-43) 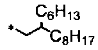 (5-47)

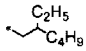 (5-44) 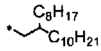 (5-48)

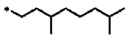 (5-45) 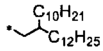 (5-49)

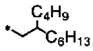 (5-46)

[Chemical Formula 35]

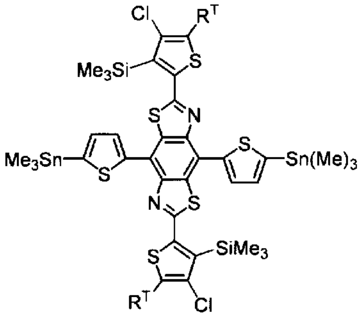

$R^T$ =

 (5-50) 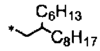 (5-54)

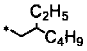 (5-51) 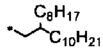 (5-55)

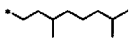 (5-52) 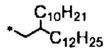 (5-56)

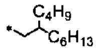 (5-53)

[Chemical Formula 36]

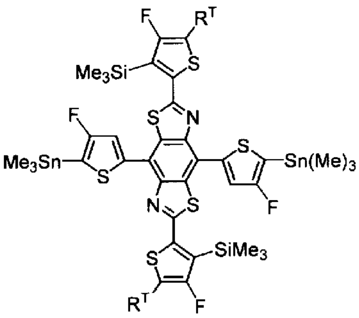

$R^T$ =

 (5-57) 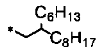 (5-61)

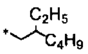 (5-58) 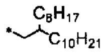 (5-62)

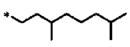 (5-59) 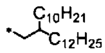 (5-63)

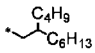 (5-60)

[Chemical Formula 37]

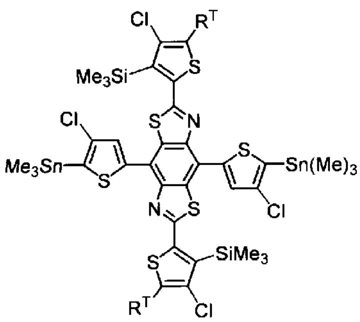

$R^T$ =

 (5-64) 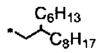 (5-68)

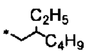 (5-65) 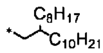 (5-69)

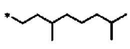 (5-66) 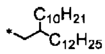 (5-70)

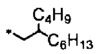 (5-67)

2-2. Compound represented by formula (4)

The present invention includes a compound represented by the following formula (4) (hereinafter, sometimes referred to as a "compound (4)").

[Chemical Formula 5]

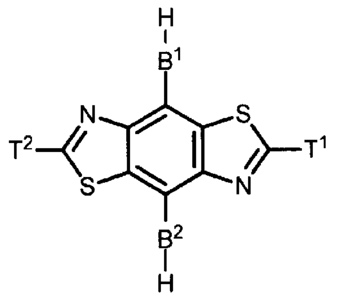

(4)

[in the formula (4),
$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

The compound (4) is a raw material for the compound (5). In other words, the compound (4) is an intermediate of the compound (5). Since the compound (4) has the predetermined group described above, it has high temporal stability, and efficient reactivity. Examples of the compound (4) may include the following compounds.

[Chemical Formula 38]

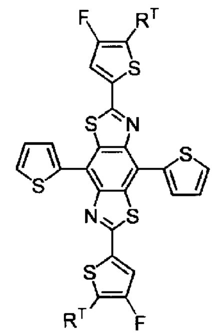

$R^T$ =

 (4-1)

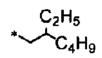 (4-2)

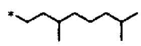 (4-3)

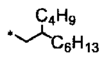 (4-4)

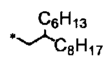 (4-5)

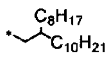 (4-6)

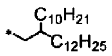 (4-7)

[Chemical Formula 39]

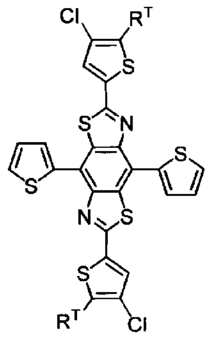

$R^T$ =

 (4-8)

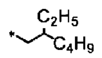 (4-9)

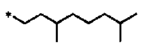 (4-10)

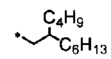 (4-11)

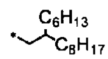 (4-12)

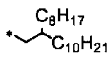 (4-13)

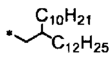 (4-14)

[Chemical Formula 40]

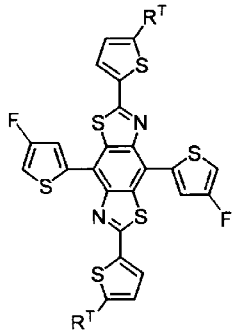

$R^T$ =  (4-15) 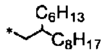 (4-19)

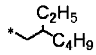 (4-16) 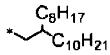 (4-20)

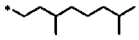 (4-17) 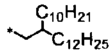 (4-21)

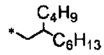 (4-18)

[Chemical Formula 41]

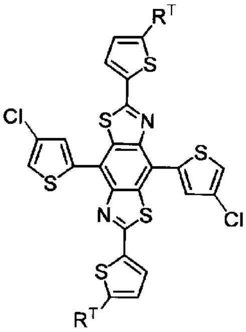

$R^T$ = 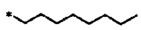 (4-22) 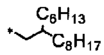 (4-26)

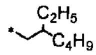 (4-23) 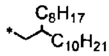 (4-27)

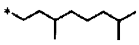 (4-24) 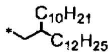 (4-28)

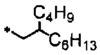 (4-25)

[Chemical Formula 42]

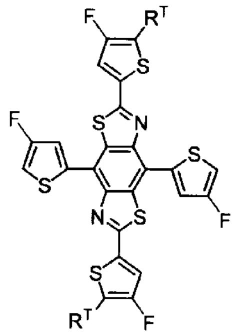

$R^T$ = 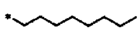 (4-29) 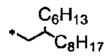 (4-33)

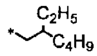 (4-30) 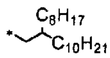 (4-34)

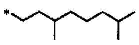 (4-31) 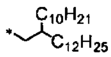 (4-35)

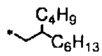 (4-32)

[Chemical Formula 43]

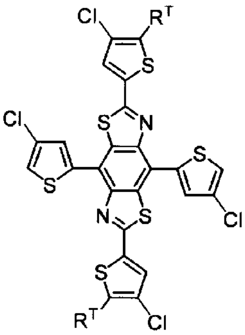

$R^T$ =  (4-36) 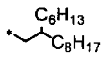 (4-40)

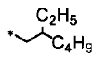 (4-37) 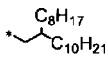 (4-41)

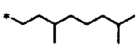 (4-38) 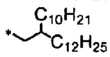 (4-42)

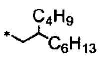 (4-39)

[Chemical Formula 44]

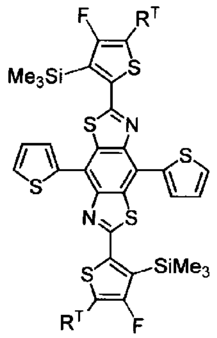

$R^T$ =

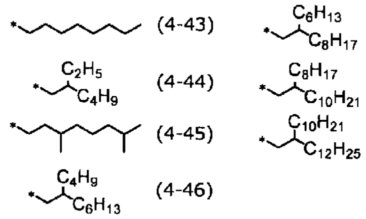

(4-43) (4-44) (4-45) (4-46) (4-47) (4-48) (4-49)

[Chemical Formula 45]

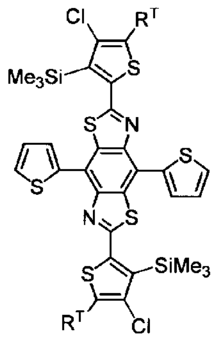

$R^T$ =

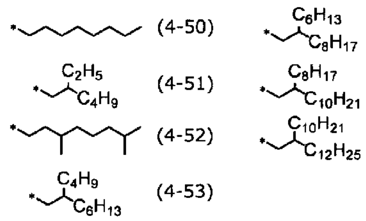

(4-50) (4-51) (4-52) (4-53) (4-54) (4-55) (4-56)

[Chemical Formula 46]

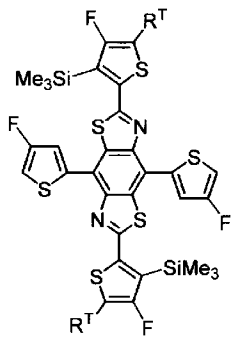

$R^T$ =

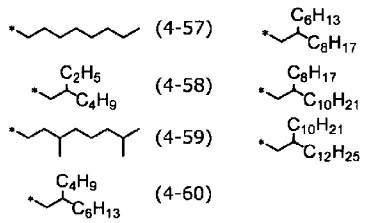

(4-57) (4-58) (4-59) (4-60) (4-61) (4-62) (4-63)

[Chemical Formula 47]

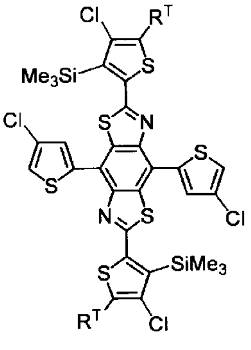

$R^T$ =

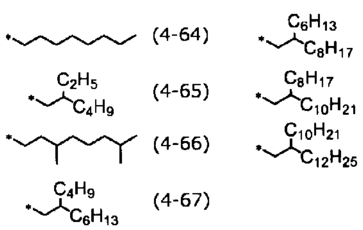

(4-64) (4-65) (4-66) (4-67) (4-68) (4-69) (4-70)

2-3. Compound represented by formula (3)

The present invention includes a compound represented by the following formula (3) (hereinafter, sometimes referred to as a "compound (3)").

[Chemical Formula 6]

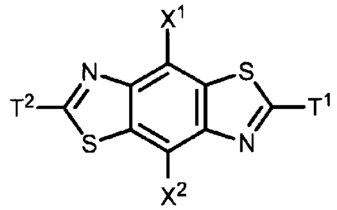

(3)

[in the formula (3),

$T^1$ and $T^2$ represent heteroaryl groups, the heteroaryl groups are optionally each independently substituted by an organosilyl group, a halogen atom, or a hydrocarbon group, and at least one of the heteroaryl groups represented by $T^1$ and $T^2$ is substituted by a halogen atom; and
$X^1$ and $X^2$ each represent a halogen atom].

Examples of the halogen atom of $X^1$ and $X^2$ include chlorine, bromine and iodine. While any of these halogen atoms may be used, iodine is especially preferable from the viewpoint of the balance between reactivity and stability.

The compound (3) is a raw material for the compound (4). In other words, the compound (3) is an intermediate of the compound (6). Since the compound (3) has the predetermined group described above, it has high temporal stability, and has a high solubility in an organic solvent, and hence efficient reactivity. Further, by using the compound (3), a variety of backbones and substituents can be introduced into the macromolecular compound according to the present invention. Examples of the compound (3) may include the following compounds.

[Chemical Formula 48]

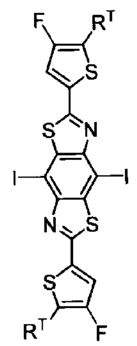

$R^T$ =  (3-1) 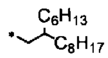 (3-5)

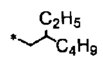 (3-2) 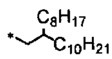 (3-6)

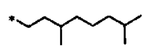 (3-3) 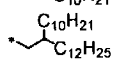 (3-7)

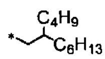 (3-4)

[Chemical Formula 49]

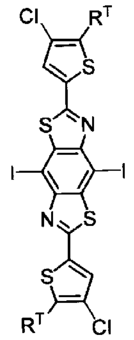

$R^T$ =

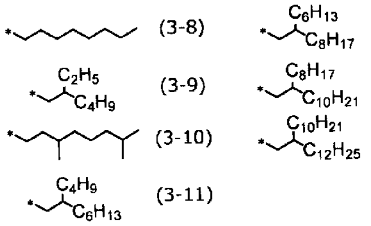

(3-8) (3-9) (3-10) (3-11) (3-12) (3-13) (3-14)

[Chemical Formula 50]

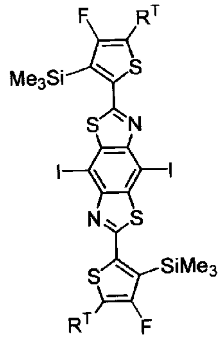

$R^T$ =

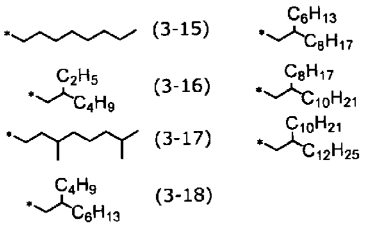

(3-15) (3-16) (3-17) (3-18) (3-19) (3-20) (3-21)

[Chemical Formula 51]

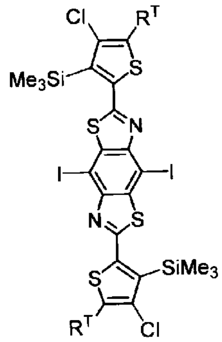

$R^T$ =

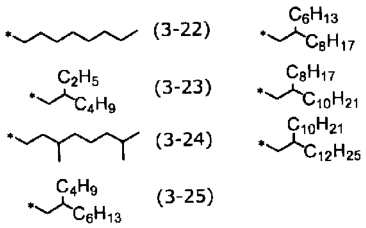

(3-22) (3-23) (3-24) (3-25) (3-26) (3-27) (3-28)

In the production method according to the present invention, a compound represented by the following formula (3') (hereinafter, sometimes referred to as a "compound (3')") is also generated. By using the compound (3'), a compound substituted with a group represented by $B^1$ or $B^2$ at only one of two substitutable positions present on the benzene ring of benzobisthiazole in the formulae (1), (4), and (5) can be obtained. The compound is useful as, for example, an end part of the macromolecular compound according to the present invention.

[Chemical Formula 26]

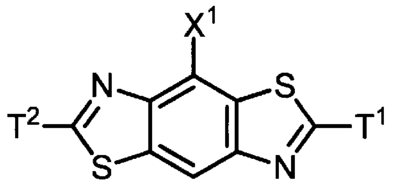

(3')

[in the formula (3'),
$T^1$, $T^2$ and $X^1$ can be referred to the description of formula (3) described above.]

Examples of the compound (3') may include compounds represented by the following formulae.

[Chemical Formula 52]

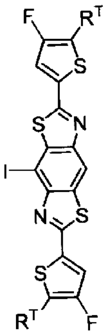

$R^T$ = 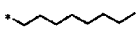 (3'-1) 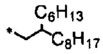 (3'-5)

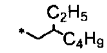 (3'-2) 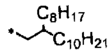 (3'-6)

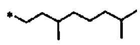 (3'-3) 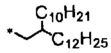 (3'-7)

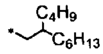 (3'-4)

[Chemical Formula 53]

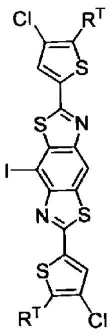

$R^T$ =  (3'-8) 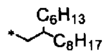 (3'-12)

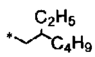 (3'-9) 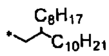 (3'-13)

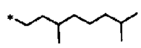 (3'-10) 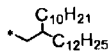 (3'-14)

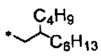 (3'-11)

[Chemical Formula 54]

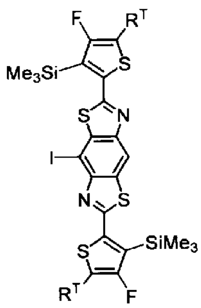

$R^T$ =  (3'-15) 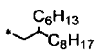 (3'-19)

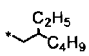 (3'-16) 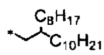 (3'-20)

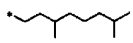 (3'-17) 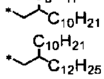 (3'-21)

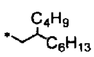 (3'-18)

[Chemical Formula 55]

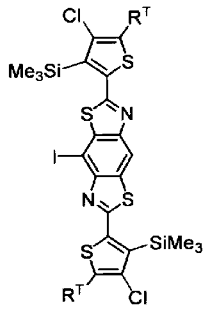

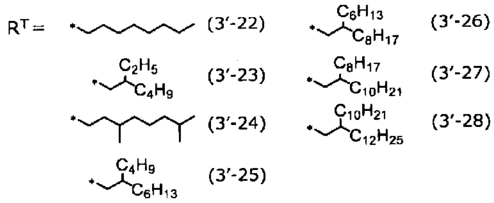

(3'-22) (3'-23) (3'-24) (3'-25) (3'-26) (3'-27) (3'-28)

2-4. Compound represented by formula (2)

The present invention includes a compound represented by the following formula (2) (hereinafter, sometimes referred to as a "compound (2)").

[Chemical Formula 7]

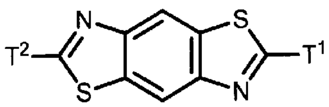

(2)

[in the formula (2),

$T^1$ and $T^2$ represent heteroaryl groups, the heteroaryl groups are optionally each independently substituted by an organosilyl group, a halogen atom, or a hydrocarbon group, and at least one of the heteroaryl groups represented by $T^1$ and $T^2$ is substituted by a halogen atom].

The compound (2) is a raw material for the compound (3). In other words, the compound (2) is an intermediate of the compound (5). Since the compound (2) has the predetermined group described above, it has high temporal stability, and efficient reactivity. Further, by using the compound (2), a variety of backbones and substituents can be introduced into the macromolecular compound according to the present invention. Examples of the compound (2) may include the following compounds.

[Chemical Formula 56]

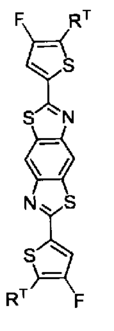

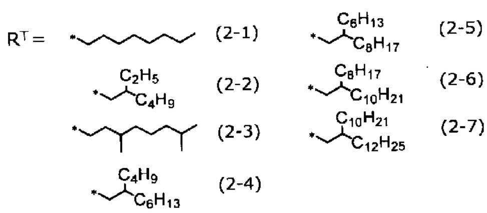

(2-1) (2-2) (2-3) (2-4) (2-5) (2-6) (2-7)

[Chemical Formula 57]

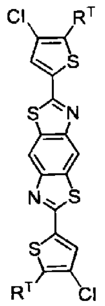

$R^T$ =

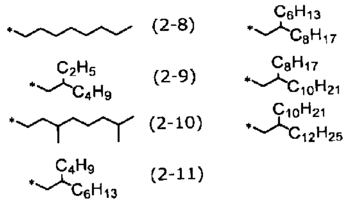

(2-8) (2-9) (2-10) (2-11) (2-12) (2-13) (2-14)

2-5. Compound represented by formula (20)

The present invention includes a thiophene compound represented by the following formula (20).

[Chemical Formula 8]

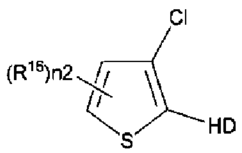

(20)

[in the formula (20),

HD represents a hexyldecyl group;
$R^{16}$s each independently represent a hydrogen atom, a hydrocarbon group with a carbon number of 6 to 30, a halogen atom, or a group represented by *-Sn($R^{18}$)$_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different;
n2 represents 1 or 2;
a plurality of $R^{16}$s may be same or different; and
asterisk symbol (*) represents a bond].

2-6. Compound represented by formula (21)

The present invention includes a thiophene compound represented by the following formula (21).

[Chemical Formula 9]

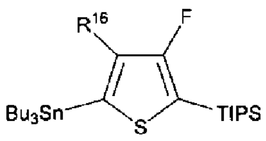

(21)

[in the formula (21),

TIPS represents a triisopropylsilyl group;
BusSn represents a tributyltin group;
$R^{16}$ represents a hydrogen atom, a hydrocarbon group with a carbon number of 6 to 30, a halogen atom, or a group represented by *-Sn($R^{18}$)$_3$;
$R^{18}$s each independently represent an aliphatic hydrocarbon group with a carbon number of 1 to 20 or an aromatic

hydrocarbon group with a carbon number of 6 to 10, and a plurality of $R^{18}$s may be same or different; and asterisk symbol (*) represents a bond].

3. Production method

The macromolecular compound represented by the formula (1) according to the present invention is produced by a production method which includes:

using one compound selected from the group consisting of 2,6-diiodobenzo[1,2-d:4,5-d']bisthiazole and 2,6-dibromobenzo[1,2-d:4,5-d']bisthiazole as starting materials; and
going through
a compound represented by the formula (2):

[Chemical Formula 7]

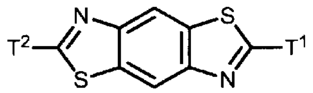

(2)

[in the formula (2),

$T^1$ and $T^2$ represent heteroaryl groups, and the heteroaryl groups are optionally each independently substituted by an organosilyl group, a halogen atom, or a hydrocarbon group];
a compound represented by the formula (3):

[Chemical Formula 6]

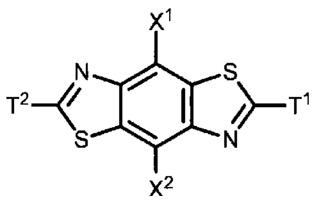

(3)

[in the formula (3),

$T^1$ and $T^2$ represent heteroaryl groups, and the heteroaryl groups are optionally each independently substituted by an organosilyl group, a halogen atom, or a hydrocarbon group; and
$X^1$ and $X^2$ each represent a halogen atom]; and
a compound represented by the formula (4):

[Chemical Formula 5]

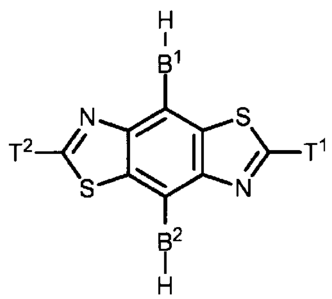

(4)

[in the formula (4),

$T^1$, $T^2$, $B^1$, and $B^2$ each represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

Preferably, the production method for the macromolecular compound according to the present invention further goes through a compound represented by the formula (5):

[Chemical Formula 4]

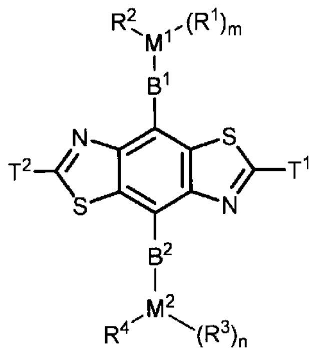

(5)

[in the formula (5),

$T^1$, $T^2$, $B^1$, $B^2$, $R^1$ to $R^4$, $M^1$, $M^2$, m, and n each represent the same groups as above. However, at least one of the heteroaryl groups represented by $T^1$, $T^2$, $B^1$, and $B^2$ is substituted by a halogen atom].

According to the production method according to the present invention, a variety of substituents can be introduced into a benzobisthiazole backbone, leading to material design with a high degree of freedom. As a result, the properties of a material (e.g. energy level, solubility, crystallinity, film formability and absorption wavelength) can be easily controlled.

3-1. First step

Preferably, the production method according to the present invention comprises the following first step.

First step: a step of reacting a compound represented by the formula (6) and/or the formula (7) with one compound selected from the group consisting of 2,6-diiodobenzo[1,2-d:4,5-d']bisthiazole and 2,6-dibromobenzo[1,2-d:4,5-d']bisthiazole in the presence of a metal catalyst to prepare a compound represented by the formula (22):
[Chemical Formula 12]

$T^1$-$R^5$ (6)

$T^2$-$R^6$ (7)

[in the formulae (6) and (7),

$T^1$ and $T^2$ each represent the same groups as above;
$R^5$ and $R^6$ each independently represent a hydrogen atom or *-$M^3(R^7)_kR^8$;
$R^7$ and $R^8$ each independently represent an aliphatic hydrocarbon group with a carbon number of 1 to 6, a hydroxy group, an alkoxy group with a carbon number of 1 to 6, or an aryloxy group with a carbon number of 6 to 10;
$M^3$ represents a boron atom or a tin atom;
asterisk symbol (*) represents a bond;
$R^7$ and $R^8$ may form a ring with $M^3$;
k represents an integer of 1 or 2; and
when k is 2, a plurality of $R^7$s may be same or different];

In the first step, the one compound (hereinafter, sometimes referred to as "2,6-dihalogenated benzobisthiazole") selected from the group consisting of 2,6-diiodobenzo[1,2-d:4,5-d']bisthiazole and 2,6-dibromobenzo[1,2-d:4,5-d']bisthiazole is preferably 2,6-diiodobenzo[1,2-d:4,5-d']bisthiazole.

The compound represented by the formula (6) and/or formula (7) (hereinafter, sometimes referred to as a "compound (6)" and/or "compound (7)"), which is reacted with 2,6-dihaloganated benzobisthiazole, is preferably a com-

pound in which $T^1$ and $T^2$ are each a group similar to one described above, and $R^5$ and $R^6$ are each a hydrogen atom or $*\text{-}M^3(R^7)_kR^8$. Asterisk symbol (*) represents a bond.

When $R^5$ and $R^6$ each represent $M^3(R^7)_kR^8$, the carbon number of the aliphatic hydrocarbon groups of $R^7$ and $R^8$ is preferably 1 to 5, more preferably 1 to 4. Examples of the aliphatic hydrocarbon groups of $R^7$ and $R^8$ include methyl group, ethyl group, propyl group and butyl group. The carbon number of $R^7$ and $R^8$ is preferably 1 to 3, more preferably 1 or 2. The alkoxy groups of $R^7$ and $R^8$ are each preferably methoxy group, ethoxy group, propoxy group or the like, more preferably methoxy group or ethoxy group. The carbon number of the aryloxy groups of $R^7$ and $R^8$ is preferably 6 to 9, more preferably 6 to 8. Examples of the aryloxy groups of $R^7$ and $R^8$ include phenyloxy group, benzyloxy group and phenylenebis(methyleneoxy) group.

When $R^5$ and $R^6$ are $*\text{-}M^3(R^7)_kR^8$, and $M^3$ is a boron atom, $R^7$ and $R^8$ are each preferably hydroxyl group, an alkoxy group with a carbon number of 1 to 6, or an aryloxy group with a carbon number of 6 to 10, and k is preferably 1. When $M^3$ is a boron atom, examples of $*\text{-}M^3(R^7)_kR^8$ include groups represented by the following formulae. Asterisk symbol (*) represents a bond.

[Chemical Formula 24]

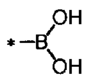 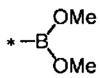 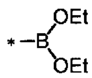 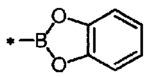 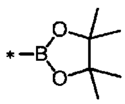 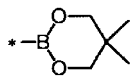

When $R^5$ and $R^6$ are $*\text{-}M^3(R^7)_kR^8$, and $M^3$ is tin atom, $R^7$ and $R^8$ are each preferably an aliphatic hydrocarbon group with a carbon number of 1 to 6, and k is preferably 2. When $M^3$ is tin atom, examples of $*\text{-}M^3(R^7)_kR^8$ include groups represented by the following formulae. Asterisk symbol (*) represents a bond.

[Chemical Formula 25]

$$*-Sn(Me)_3 \qquad *-Sn(Bu)_3$$

$R^5$ and $R^6$ are each preferably a group represented by $*\text{-}M^3(R^7)_kR^8$, more preferably a group represented by $*\text{-}Sn(R^7)_2R^8$.

Examples of the compounds (6) and (7) include compounds represented by the following formulae.

[Chemical Formula 58]

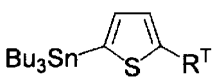

$R^T$ =

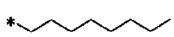 (6-1) 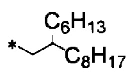 (6-5)

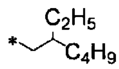 (6-2) 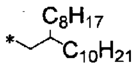 (6-6)

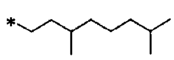 (6-3) 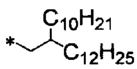 (6-7)

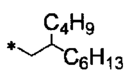 (6-4)

[Chemical Formula 59]

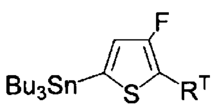

$R^T$ =

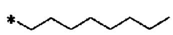 (6-8) 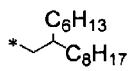 (6-12)

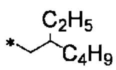 (6-9) 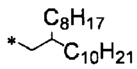 (6-13)

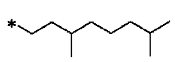 (6-10) 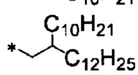 (6-14)

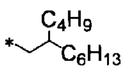 (6-11)

[Chemical Formula 60]

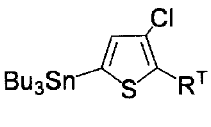

$R^T$ =

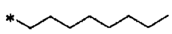 (6-15)

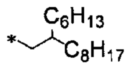 (6-19)

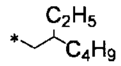 (6-16)

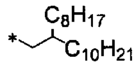 (6-20)

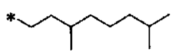 (6-17)

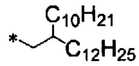 (6-21)

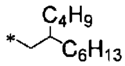 (6-18)

Among the formulae (6) and (7), the formula (6-2), (6-5), or (6-19) is preferable.

The compounds (6) and (7) may be same or different depending on an intended compound, and they are preferably the same for suppressing generation of byproducts.

In the first step, the molar ratio of 2,6-dihalogenated benzobisthiazole and the total of the compounds (6) and (7) (2,6-dihalogenated benzobisthiazole : total of compounds (6) and (7)) is not particularly limited, and is generally about 1 : 1 to 1 : 10, and it is preferably 1 : 1.5 to 1 : 8, more preferably 1 : 2 to 1 : 6, further preferably 1 : 2 to 1 : 5 from the viewpoint of the yield and reaction efficiency.

Examples of the metal catalyst to be used in the reaction of 2,6-dihalogenated benzobisthiazole with the compound (6) and/or compound (7) in the first step include transition metal catalysts such as palladium-based catalysts, nickel-based catalysts, iron-based catalysts, copper-based catalysts, rhodium-based catalysts and ruthenium-based catalysts. Among them, copper-based catalysts and palladium-based catalysts are preferable.

The valence number of palladium is not particularly limited, and palladium may be zero-valent or divalent.

Examples of the palladium-based catalyst include palladium acetate (II), palladium chloride (II), palladium bromide (II), palladium iodide (II), palladium oxide (II), palladium sulfide (II), palladium telluride (II), palladium hydroxide (II), palladium selenide (II), palladium cyanide (II), palladium acetate (II), palladium trifluoroacetate (II), palladium acetylacetonate (II), diacetatebis(triphenylphosphine)palladium (II), tetrakis(triphenylphosphine)palladium (0), dichlorobis(triphenylphosphine)palladium (II), dichlorobis(acetonitrile)palladium (II), dichlorobis(benzonitrile)palladium (II), dichloro[1,2-bis(diphenylphosphino)ethane]palladium (II), dichloro[1,3-bis(diphenylphosphino)propane]palladium (II), dichloro[1,4-bis(diphenylphosphino)butane]palladium (II), dichloro[1,1-bis(diphenylphosphinoferrocene)]palladium (II), dichloro[1,1-bis(diphenylphosphino)ferrocene]palladium (II) dichloromethane adducts, bis(dibenzylideneacetone)palladium (0), tris(dibenzylideneacetone)dipalladium (0), tris(dibenzylideneacetone)dipalladium (0) chloroform adducts, tris(dibenzylideneacetone)dipalladium (0)-xdba adducts, dichloro[1,3-bis(2,6-diisopropylphenyl)imidazole-2-ylidene](3-chloropyridyl)palladium (II), bis(tri-tert-butylphosphine)palladium (0), dichloro[2,5-norbornadiene]palladium (II), dichlorobis(ethylenediamine)palladium (II), dichloro(1,5-cyclooctadiene)palladium (II) and dichlorobis(methyldiphenylphosphine)palladium (II). One of these catalysts may be used alone, or two or more of these catalysts may be used in combination.

Examples of the copper-based catalyst include copper, copper halide compounds such as copper fluoride (I), copper chloride (I), copper bromide (I), copper iodide (I), copper fluoride (II), copper chloride (II), copper bromide (II) and copper iodide (II); copper oxide (I), copper sulfide (I), copper oxide (II), copper sulfide (II), copper acetate (I), copper acetate (II) and copper sulfate (II).

The metal catalyst is preferably a palladium-based catalyst, especially preferably palladium acetate (II), dichlorobis(triphenylphosphine)palladium (II), tris(dibenzylideneacetone)dipalladium (0) or a tris(dibenzylideneacetone)dipalladium (0) chloroform adduct.

In the first step, the molar ratio of 2,6-dihalogenated benzobisthiazole and the metal catalyst (2,6-dihalogenated benzobisthiazole : metal catalyst) is not particularly limited, and is generally about 1 : 0.0001 to 1 : 0.5, and it is preferably 1 : 0.001 to 1 : 0.4, more preferably 1 : 0.005 to 1 : 0.3, further preferably 1 : 0.01 to 1 : 0.2 from the viewpoint of the yield and reaction efficiency.

In the first step, a specific ligand may be coordinated to the metal catalyst such as a copper-based catalyst or a palladium-based catalyst. Examples of the ligand include trimethylphosphine, triethylphosphine, tri(n-butyl)phosphine, tri(isopropyl)phosphine, tri(tert-butyl)phosphine, tri-tert-butylphosphonium tetrafluoroborate, bis(tert-butyl)methylphosphine, tricyclohexylphosphine, diphenyl(methyl)phosphine, triphenylphosphine, tris(o-tolyl)phosphine, tris(m-tolyl)phosphine, tris(p-tolyl)phosphine, tris(2-furyl)phosphine, tris(2-methoxyphenyl)phosphine, tris(3-methoxyphenyl)phosphine, tris(4-methoxyphenyl)phosphine, 2-dicyclohexylphosphinobiphenyl, 2-dicyclohexylphosphino-2'-methylbiphenyl, 2-dicyclohexylphosphino-2',4',6'-triisopropyl-1,1'-biphenyl, 2-dicyclohexylphosphino -2',6'-diisopropoxy-1,1'-biphenyl, 2-dicyclohexylphosphino-2',6'-dimethoxy-1,1'-biphenyl, 2-dicyclohexylphosphino-2'-(N,N'-dimethylamino)biphenyl, 2-diphenylphosphino-2'-(N,N'-dimethylamino)biphenyl, 2-(di-tert-butyl)phosphino-2'-(N,N'-dimethylamino)biphenyl,2-(di-tert-butyl)phosphinobiphenyl, 2-(di-tert-butyl)phosphino-2'-methylbiphenyl, 1,2-bis(diphenylphosphino)ethane, 1,3-bis(diphenylphosphino)propane, 1,4-bis(diphenylphosphino)butane, 1,2-bis(dicyclohexylphosphino)ethane, 1,3-bis(dicyclohexylphosphino)propane, 1,4-bis(dicyclohexylphosphino)butane, 1,2-bisdiphenylphosphinoethylene, 1,1'-bis(diphenylphosphino)ferrocene, 1,2-ethylenediamine, N,N,N',N'-tetramethylethylenediamine, 2,2'-bipyridyl, 1,3-diphenyldihydroimidazolylidene, 1,3-dimethyldihydroimidazolylidene, diethyldihydroimidazolylidene, 1,3-bis(2,4,6-trimethylphenyl)dihydroimidazolylidene, 1,3-bis(2,6-diisopropylphenyl)dihydroimidazolylidene, 1,10-phenanthroline, 5,6-dimethyl-1,10-phenanthroline and bathophenanthroline. Only one ligand may be used, or two or more ligands may be used. Among them, 2-dicyclohexylphosphinobiphenyl, 2-dicyclohexylphosphino-2'-methylbiphenyl, 2-dicyclohexylphosphino -2',4',6'-triisopropyl-1,1'-biphenyl, 2-dicyclohexylphosphino - 2',6'-diisopropoxy-1,1'-biphenyl, triphenylphosphine, tris(o-tolyl)phosphine, tris(2-furyl)phosphine are preferable.

When a ligand is coordinated to the metal catalyst in the first step, the molar ratio of the metal catalyst and the ligand (metal catalyst : ligand) is not particularly limited, and is generally about 1 : 0.5 to 1 : 10, and it is preferably 1 : 1 to 1 : 8, more preferably 1 : 1 to 1 : 7, further preferably 1 : 1 to 1 : 5 from the viewpoint of the yield and reaction efficiency.

In the first step, a base may coexist depending on the type of $R^5$ and $R^6$ in the reaction of the compound (6) and/or compound (7) with 2,6-dihalogenated benzobisthiazole in the presence of the metal catalyst. For example, when $R^5$ and $R^6$ are each a group represented by $*-M^3(R^7)_kR^8$, it is preferable that a base coexists when $M^3$ is a boron atom, and a base is not required to coexist when $M^3$ is a tin atom.

Examples of the base include alkali metal salt compounds such as lithium hydride, sodium hydroxide, potassium hydroxide, cesium hydroxide, lithium carbonate, sodium carbonate, potassium carbonate, cesium carbonate and potassium phosphate; alkali earth metal salt compounds such as magnesium hydroxide, calcium hydroxide, barium hydroxide, magnesium carbonate, calcium carbonate and barium carbonate; alkoxy alkali metal compounds such as lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-amylalkoxide, sodium tert-amylalkoxide and potassium tert-amylalkoxide; and metal hydride compounds such as lithium hydride, sodium hydride and potassium hydride. Particularly, the base is preferably an alkoxy alkali metal compound, more preferably lithium tert-butoxide, sodium tert-butoxide, potassium tert-butoxide, sodium carbonate, potassium carbonate, cesium carbonate or potassium phosphate.

In the first step, the molar ratio of 2,6-dihalogenated benzobisthiazole and the base (2,6-dihalogenated benzobisthiazole : base) is not particularly limited, and is generally about 1 : 1 to 1 : 10, and it is preferably 1 : 1.5 to 1 : 8, more preferably 1 : 1.8 to 1 : 6, further preferably 1 : 2 to 1 : 5 from the viewpoint of the yield and reaction efficiency.

In the first step, the solvent in which the compound (6) and/or compound (7) are reacted with 2,6-dihalogenated benzobisthiazole in the presence of the metal catalyst is not particularly limited as long as the reaction is not affected, and an ether-based solvent, an aromatic-based solvent, an ester-based solvent, a hydrocarbon-based solvent, a halogen-based solvent, a ketone-based solvent, an amide-based solvent or the like may be used. Examples of the ether-based solvent include diethyl ether, dipropyl ether, diisopropyl ether, dibutyl ether, tetrahydrofuran, methyltetrahydrofuran, dimethoxyethane, cyclopentyl methyl ether, t-butyl methyl ether and dioxane. Examples of the aromatic-based solvent include benzene, toluene, xylene, mesitylene, chlorobenzene and dichlorobenzene. Examples of the ester-based solvent include methyl acetate, ethyl acetate, propyl acetate, isopropyl acetate and butyl acetate. Examples of the hydrocarbon-based solvent include pentane, hexane and heptane. Examples of the halogen-based solvent include dichloromethane, chloroform, dichloroethane and dichloropropane. Examples of the ketone-based solvent include acetone, methyl ethyl ketone and methyl isobutyl ketone. Examples of the amide-based solvent include N,N-dimethylformamide, N,N-dimethylacetamide, 1,3-dimethyl-2-imidazolidinone and 1,3-dimethyl3,4,5,6-tetrahydro-(1H)-pyrimidine. Nitrile-based solvents such as acetonitrile, sulfoxide-based solvents such as dimethylsulfoxide and sulfone-based solvents such as sulfolane may be used.

Among them, tetrahydrofuran, dioxane and N,N-dimethylformamide are especially preferable.

In the first step, the use amount of the solvent based on 1 g of 2,6-dihalogenated benzobisthiazole is not particularly limited, and is generally about 1 mL or more and about 50 mL or less, and it is preferably 5 mL or more and 40 mL or less, more preferably 8 mL or more and 35 mL or less, further preferably 10 mL or more and 30 mL or less from the viewpoint of the yield and reaction efficiency.

In the first step, the reaction temperature is not particularly limited, and it is preferably 0°C or higher and 200°C or lower, more preferably 30°C or higher and 180°C or lower, further preferably 40°C or higher and 150°C or lower from the viewpoint of improving the reaction yield.

3.2. Second step

Preferably, the production method according to the present invention includes the following second step. There are two routes for the second step: Route A, which includes the second step (a), and Route B, which includes the second step (b-1) and the second step (b-2).

3-2-1. Second step (a)

Second step (a) : a step of reacting a base and a halogenation reagent with a compound represented by the formula (22) to prepare a compound represented by the formula (3).
Examples of the base to be reacted with the compound (22) in the second step (a) include alkyllithiums, alkyl metal amides, alkylmagnesiums and magnesium complexes, and alkali metal hydrides.
Examples of the alkyllithium include n-butyllithium, sec-butyllithium and tert-butyllithium. Examples of the alkyl metal amide include lithium diisopropylamide, lithium diethylamide, lithium bis(trimethylsilyl)amide, sodium bis(trimethylsilyl)amide, potassium bis(trimethylsilyl)amide, lithium-2,2,6,6-tetramethylpiperidide, lithium amide, sodium amide and potassium amide. Examples of the alkylmagnesium and magnesium complex include tert-butylmagnesium chloride, ethylmagnesium chloride, 2,2,6,6-tetramethylpiperidinylmagnesium chloride and lithium chloride complexes. Examples of the alkali metal hydride include lithium hydride, sodium hydride and potassium hydride. Among them, alkyllithium or alkyl metal amide is preferable, and n-butyllithium and lithium diisopropylamide are especially preferable from the viewpoint of position selectivity.
In the second step (a), the molar ratio of the compound (22) and the base (compound (22) : base) is not particularly limited, and is generally about 1 : 1.8 to 1 : 3.0, and it is preferably 1 : 1.9 to 1 : 2.6, more preferably 1 : 2.0 to 1 : 2.4, further preferably 1 : 2.0 to 1 : 2.2 from the viewpoint of the yield and reaction efficiency.
In the second step (a), examples of the halogenation reagent to be reacted with the compound (22) together with the base include halogen molecules and N-halogenated succinimides. Examples of the halogen molecule include chlorine, bromine and iodine. Examples of the N-halogenated succinimide include N-chlorosuccinimide, N-bromosuccinimide and N-iodosuccinimide. From the viewpoint of availability and reactivity, a halogen molecule is preferable, and iodine is especially preferable.
In the second step (a), the molar ratio of the compound (22) and the halogenation reagent (compound (22) : halogenation reagent) is not particularly limited, and is generally about 1 : 1.5 to 1 : 20.0, and it is preferably 1 : 1.7 to 1 : 17.0, more preferably 1 : 1.9 to 1 : 15.0, further preferably 1 : 2.0 to 1 : 10.0 from the viewpoint of the yield and reaction efficiency.
The molar ratio of the base and the halogenation reagent (base : halogenation reagent) is, for example, about 1 : 0.5 to 1 : 2.0, preferably 1 : 0.6 to 1 : 1.7, more preferably 1 : 0.7 to 1 : 1.5, further preferably 1 : 0.8 to 1 : 1.2.
In the second step (a), the base and the halogenation reagent may be simultaneously reacted, and from the viewpoint of reaction efficiency, it is preferable that the basic compound is first reacted, and the halogenation reagent is then reacted.
In the second step (a), the solvent in which the compound (22) is reacted with the base and the halogenation reagent is not particularly limited, and ether-based solvents and hydrocarbon-based solvents may be used. Examples of the ether-based solvent include diethyl ether, dipropyl ether, diisopropyl ether, dibutyl ether, tetrahydrofuran, methyltetrahydrofuran, dimethoxyethane, cyclopentyl methyl ether, t-butyl methyl ether and dioxane. Examples of the hydrocarbon-based solvent include pentane, hexane, heptane, benzene, toluene and xylene. Among them, ether-based solvents are preferable, and tetrahydrofuran is especially preferable. The one solvent may be used alone, or two or more solvents may be used in combination.
In the second step (a), the use amount of the solvent based on 1 g of the compound (22) is not particularly limited, and is generally 3 mL or more and about 150 mL, and it is preferably 5 mL or more and 120 mL or less, more preferably 8 mL or more and 100 mL or less, further preferably 10 mL or more and 80 mL or less from the viewpoint of the yield and reaction efficiency.
In the second step (a), the temperature at which the base and the halogenation reagent are reacted with the compound (22) is preferably equal to or less than room temperature, more preferably -30°C or lower, further preferably -35°C or lower for suppressing generation of byproducts.

3-2-2. Second step (b-1) and Second step (b-2)

Second step (b-1): a step of reacting a base and an organosilylation reagent with the compound represented by the formula (22);
Second step (b-2): a step of reacting a base and a halogenation reagent with the product resulting from the second step (b-1) to prepare a compound represented by the formula (23).
That is, these are steps of reacting a base and an organosilylation reagent with the compound represented by the formula (22), and then, reacting a base and a halogenation reagent to prepare a compound represented by the formula (23).
Examples of the base to be reacted with the compound (22) in the second step (b-1) include alkyllithiums, alkyl metal amides, alkylmagnesiums and magnesium complexes, and alkali metal hydrides.
Examples of the alkyllithium include n-butyllithium, sec-butyllithium and tert-butyllithium. Examples of the alkyl

metal amide include lithium diisopropylamide, lithium diethylamide, lithium bis(trimethylsilyl)amide, sodium bis(trimethylsilyl)amide, potassium bis(trimethylsilyl)amide, lithium-2,2,6,6-tetramethylpiperidide, lithium amide, sodium amide and potassium amide. Examples of the alkylmagnesium and magnesium complex include tert-butylmagnesium chloride, ethylmagnesium chloride, 2,2,6,6-tetramethylpiperidinylmagnesium chloride and lithium chloride complexes. Examples of the alkali metal hydride include lithium hydride, sodium hydride and potassium hydride. Among them, alkyllithium or alkyl metal amide is preferable, and n-butyllithium and lithium diisopropylamide are especially preferable from the viewpoint of position selectivity.

In the second step (b-1), the molar ratio of the compound (22) and the base (compound (22) : base) is not particularly limited, and is generally about 1 : 1.8 to 1 : 3.0, and it is preferably 1 : 1.9 to 1 : 2.6, more preferably 1 : 2.0 to 1 : 2.4, further preferably 1 : 2.0 to 1 : 2.2 from the viewpoint of the yield and reaction efficiency.

Examples of the organosilylation reagent to be reacted with the compound (22) together with the base in the second step (b-1) include organosilyl chlorides and organosilyl triflates. Examples of the organosilyl chlorides include trimethylsilyl chloride, triethylsilyl chloride, triisopropylsilyl chloride, t-butyldimethylsilyl chloride, and t-butyldiphenylsilyl chloride. Examples of the organosilyl triflates include trimethylsilyl triflate, triethylsilyl triflate, triisopropylsilyl triflate, t-butyldimethylsilyl triflate, and t-butyldiphenylsilyl triflate. From the viewpoint of availability, reactivity, and the like, organosilyl chlorides are preferable, and trimethylsilyl chloride is particularly preferable.

In the second step (b-1), the molar ratio of the compound (22) and the organosilylation reagent (compound (22) : organosilylation reagent) is not particularly limited, and is generally about 1 : 1.5 to 1 : 20.0, and it is preferably 1 : 1.7 to 1 : 17.0, more preferably 1 : 1.9 to 1 : 15.0, further preferably 1 : 2.0 to 1 : 10.0 from the viewpoint of the yield and reaction efficiency.

The molar ratio of the base and the organosilylation reagent (base : organosilylation reagent) is, for example, about 1 : 0.5 to 1 : 2.0, preferably 1 : 0.6 to 1 : 1.7, more preferably 1 : 0.7 to 1 : 1.5, further preferably 1 : 0.8 to 1 : 1.2.

In the second step (b-1), the base and the halogenation reagent may be simultaneously reacted, and from the viewpoint of reaction efficiency, it is preferable that the basic compound is first reacted, and the halogenation reagent is then reacted.

In the second step (b-1), the solvent in which the compound (22) is reacted with the base and the halogenation reagent is not particularly limited, and ether-based solvents and hydrocarbon-based solvents may be used. Examples of the ether-based solvent include diethyl ether, dipropyl ether, diisopropyl ether, dibutyl ether, tetrahydrofuran, methyltetrahydrofuran, dimethoxyethane, cyclopentyl methyl ether, t-butyl methyl ether and dioxane. Examples of the hydrocarbon-based solvent include pentane, hexane, heptane, benzene, toluene and xylene. Among them, ether-based solvents are preferable, and tetrahydrofuran is especially preferable. The one solvent may be used alone, or two or more solvents may be used in combination.

In the second step (b-1), the use amount of the solvent based on 1 g of the compound (22) is not particularly limited, and is generally 3 mL or more and about 150 mL, and it is preferably 5 mL or more and 120 mL or less, more preferably 8 mL or more and 100 mL or less, further preferably 10 mL or more and 80 mL or less from the viewpoint of the yield and reaction efficiency.

In the second step (b-1), the temperature at which the base and the halogenation reagent are reacted with the compound (22) is preferably equal to or less than room temperature, more preferably -30°C or lower, further preferably -35°C or lower for suppressing generation of byproducts.

Examples of the base to be reacted with a product obtained by the second step (b-2) in the second step (b-2) include alkyllithiums, alkyl metal amides, alkylmagnesiums and magnesium complexes, and alkali metal hydrides.

Examples of the alkyllithium include n-butyllithium, sec-butyllithium and tert-butyllithium. Examples of the alkyl metal amide include lithium diisopropylamide, lithium diethylamide, lithium bis(trimethylsilyl)amide, sodium bis(trimethylsilyl)amide, potassium bis(trimethylsilyl)amide, lithium-2,2,6,6-tetramethylpiperidide, lithium amide, sodium amide and potassium amide. Examples of the alkylmagnesium and magnesium complex include tert-butylmagnesium chloride, ethylmagnesium chloride, 2,2,6,6-tetramethylpiperidinylmagnesium chloride and lithium chloride complexes. Examples of the alkali metal hydride include lithium hydride, sodium hydride and potassium hydride. Among them, alkyllithium or alkyl metal amide is preferable, and n-butyllithium and lithium diisopropylamide are especially preferable from the viewpoint of position selectivity.

In the second step (b-2), the molar ratio of the product obtained by the second step (b-1) and the base (compound (22) : base) is not particularly limited, and is generally about 1 : 1.8 to 1 : 3.0, and it is preferably 1 : 1.9 to 1 : 2.6, more preferably 1 : 2.0 to 1 : 2.4, further preferably 1 : 2.0 to 1 : 2.2 from the viewpoint of the yield and reaction efficiency.

In the second step (b-2), examples of the halogenation reagent to be reacted with the product obtained by the second step (b-1) together with the base include halogen molecules and N-halogenated succinimides. Examples of the halogen molecule include chlorine, bromine and iodine. Examples of the N-halogenated succinimide include N-chlorosuccinimide, N-bromosuccinimide and N-iodosuccinimide. From the viewpoint of availability and reactivity, a halogen molecule is preferable, and iodine is especially preferable.

In the second step (b-2), the molar ratio of the product obtained by the second step (b-1) and the halogenation

reagent (the product obtained by the second step (b-1)) : halogenation reagent) is not particularly limited, and is generally about 1 : 1.5 to 1 : 20.0, and it is preferably 1 : 1.7 to 1 : 17.0, more preferably 1 : 1.9 to 1 : 15.0, further preferably 1 : 2.0 to 1 : 10.0 from the viewpoint of the yield and reaction efficiency.

The molar ratio of the base and the halogenation reagent (base : halogenation reagent) is, for example, about 1 : 0.5 to 1 : 2.0, preferably 1 : 0.6 to 1 : 1.7, more preferably 1 : 0.7 to 1 : 1.5, further preferably 1 : 0.8 to 1 : 1.2.

In the second step (b-2), the base and the halogenation reagent may be simultaneously reacted, and from the viewpoint of reaction efficiency, it is preferable that the basic compound is first reacted, and the halogenation reagent is then reacted.

In the second step (b-2), the solvent in which the product obtained by the second step (b-1) is reacted with the base and the halogenation reagent is not particularly limited, and ether-based solvents and hydrocarbon-based solvents may be used. Examples of the ether-based solvent include diethyl ether, dipropyl ether, diisopropyl ether, dibutyl ether, tetrahydrofuran, methyltetrahydrofuran, dimethoxyethane, cyclopentyl methyl ether, t-butyl methyl ether and dioxane. Examples of the hydrocarbon-based solvent include pentane, hexane, heptane, benzene, toluene and xylene. Among them, ether-based solvents are preferable, and tetrahydrofuran is especially preferable. The one solvent may be used alone, or two or more solvents may be used in combination.

In the second step (b-2), the use amount of the solvent based on 1 g of the product obtained by the second step (b-1) is not particularly limited, and is generally 3 mL or more and about 150 mL, and it is preferably 5 mL or more and 120 mL or less, more preferably 8 mL or more and 100 mL or less, further preferably 10 mL or more and 80 mL or less from the viewpoint of the yield and reaction efficiency.

In the second step (b-2), the temperature at which the base and the halogenation reagent are reacted with the product obtained by the second step (b-1) is preferably equal to or less than room temperature, more preferably -30°C or lower, further preferably -35°C or lower for suppressing generation of byproducts.

3-3. Third step

Preferably, the production method according to the present invention includes the following third step.

The third step: a step of reacting a compound represented by the following formula (8) and/or formula (9) with the compound represented by the formula (23) in the presence of a metal catalyst to prepare a compound represented by the formula (4):

[Chemical Formula 13]

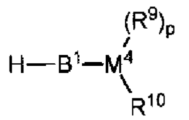

(8)

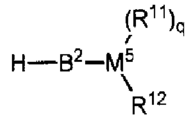

(9)

[in the formulae (8) and (9),

$B^1$ and $B^2$ represent the same groups as above or may include organosilyl groups. Alkylsilyl groups are preferred as organosilyl groups, and trimethylsilyl and triisopropylsilyl groups are particularly preferred.

$R^9$ to $R^{12}$ each independently represent an aliphatic hydrocarbon group with a carbon number of 1 to 6, a hydroxy group, an alkoxy group with a carbon number of 1 to 6, an aryl group with a carbon number of 6 to 10, or an aryloxy group with a carbon number of 6 to 10.

$M^4$ and $M^5$ each represent a boron atom, or a tin atom.

$R^9$ and $R^{10}$ may form a ring with $M^4$, and $R^{11}$ and $R^{12}$ may form a ring with $M^5$.

p and q each represent an integer of 1 or 2.

When p is 2, a plurality of $R^9$s may be same or different, and when q is 2, a plurality of $R^{11}$s may be same or different.].

In the third step, the compound represented by the formula (8) and/or formula (9) (hereinafter, sometimes referred to as a "compound (8)" and/or "compound (9)"), which is reacted with the compound (23), is preferably a compound in which $B^1$ and $B^2$ are each a group similar to one described above. As *-$M^4(R^9)_pR^{10}$ and *-$M^5(R^{11})_qR^{12}$ in

the compounds (8) and (9), groups similar to the groups shown as examples when $R^5$ and $R^6$ in the compounds (6) and (7) to be used in the first step are *-$M^3(R^{13})_kR^{14}$ and the groups shown as examples when $R^{15}$ in the formulae (t1) is *-$Si(R^{18})_3$ may be shown as preferred examples.

Particularly, as *-$M^4(R^9)_pR^{10}$ and *-$M^5(R^{11})_qR^{12}$ when $M^4$ and $M^5$ are boron atoms, for example, groups represented by the following formulae may be preferably used. Asterisk symbol (*) represents a bond.

[Chemical Formula 24]

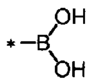 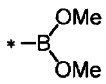 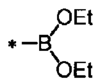 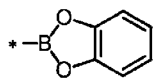 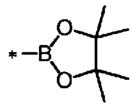 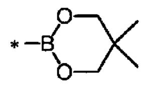

When $M^4$ and $M^5$ are tin atoms, *-$M^4(R^9)_pR^{10}$ and *-$M^5(R^{11})_qR^{12}$ are each preferably groups represented by the following formulae. Asterisk symbol (*) represents a bond.

[Chemical Formula 25] *-$Sn(Me)_3$ *-$Sn(Bu)_3$

Examples of the compounds (8) and (9) include compounds represented by the following formulae. In the formulae (8-1) to (8-5), TIPS represents a triisopropylsilyl group, and BusSn represents a tributyltin group.

[Chemical Formula 27]

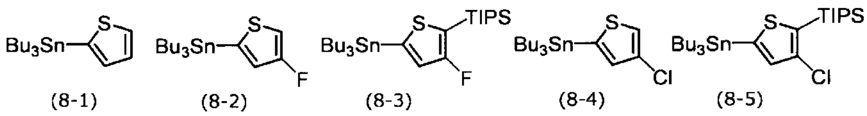

(8-1) (8-2) (8-3) (8-4) (8-5)

The compounds (8) and (9) may be same or different depending on an intended compound, and they are preferably the same from the viewpoint of suppressing generation of byproducts.

In the third step, the molar ratio of the compound (23) and the total of the compounds (8) and (9) (compound (23) : total of compounds (8) and (9)) is not particularly limited, and is generally about 1 : 1 to 1 : 10, and it is preferably 1 : 1.5 to 1 : 8, more preferably 1 : 2 to 1 : 6, further preferably 1 : 2 to 1 : 5 from the viewpoint of the yield and reaction efficiency.

As the metal catalyst to be used in the reaction of the compound (23) with the compound (8) and/or compound (9) in the third step, metal catalysts similar to those shown as examples of the metal catalyst to be used in the first step may be preferably used. Examples thereof include transition metal catalysts such as palladium-based catalysts, nickel-based catalysts, iron-based catalysts, copper-based catalysts, rhodium-based catalysts and ruthenium-based catalysts. The metal catalyst to be used in the third step is preferably a palladium-based catalyst, especially preferably palladium acetate, dichlorobis(triphenylphosphine)palladium (II), tris(dibenzylideneacetone)dipalladium (0) or a tris(dibenzylideneacetone)dipalladium (0) chloroform adduct.

In the third step, the molar ratio of the compound (23) and the metal catalyst (compound (23) : metal catalyst) is not particularly limited, and is generally about 1 : 0.0001 to 1 : 0.5, and it is preferably 1 : 0.001 to 1 : 0.4, more preferably 1 : 0.005 to 1 : 0.3, further preferably 1 : 0.01 to 1 : 0.2 from the viewpoint of the yield and reaction efficiency.

In the third step, a specific ligand may be coordinated to the metal catalyst such as a copper-based catalyst or a palladium-based catalyst. As the ligand, one similar to the ligand to be used in the first step may be preferably used, and triphenylphosphine, tris(o-tolyl)phosphine and tris(2-furyl)phosphine are preferable. When the ligand is coordinated to the metal catalyst in the third step, the molar ratio of the metal catalyst and the ligand (metal catalyst: ligand) is not particularly limited, and is generally about 1 : 0.5 to 1 : 10, and it is preferably 1 : 1 to 1 : 8, more preferably 1 : 1 to 1 : 7, further preferably 1 : 1 to 1 : 5 from the viewpoint of the yield and reaction efficiency.

In the third step, a base may coexist in the reaction of the compound (8) and/or compound (9) with the compound (23) in the presence of the metal catalyst, and whether or not a base should coexist can be determined according to the type of $M^4$ and $M^5$. For example, it is preferable that a base coexists when $M^4$ and $M^5$ are boron atoms, and a base is not required to coexist when $M^4$ and $M^5$ are tin atoms.

As the base, bases similar to those shown as examples of the base to be used in the first step may be preferably used. Examples of the base include, in addition to the bases to be used in the first step, amines such as tertiary amines such as trimethylamine, triethylamine, tripropylamine, diisopropylethylamine, tributylamine, tripentylamine, trihexylamine, trioctylamine, triallylamine, pyridine, 2-methylpyridine, 3-methylpyridine, 4-methylpyridine, N-methylmorpholine,

N,N-dimethylcyclohexylamine, N,N-dimethylaniline, N-methylimidazole, 1,4-diazabicyclo[2.2.2]octane and 1,8-diazabicyclo[5.4.0]undece-7-ene; secondary amines such as dimethylamine, diethylamine, dipropylamine, diisopropylamine, dibutylamine, dipentylamine, dihexylamine, dicyclohexylamine, dioctylamine and diallylamine; and primary amines such as methylamine, ethylamine, propylamine, isopropylamine, butylamine, pentylamine, hexylamine, cyclohexylamine, octylamine and allylamine.

The base may be selected according to the type of $M^4$ and $M^5$, and when $M^4$ and $M^5$ are boron atoms, bases shown as examples of the base to be used in the first step are preferable, alkoxy alkali metal compounds are more preferable, and lithium tert-butoxide, sodium tert-butoxide, potassium tert-butoxide, sodium carbonate, potassium carbonate, cesium carbonate and potassium phosphate are further preferable. As the base when $M^4$ and $M^5$ are silicon atoms, amines are preferable, secondary amines and tertiary amines are more preferable, and diisopropylamine, diisopropylethylamine and triethylamine are especially preferable.

In the third step, the molar ratio of the compound (23) and the base (compound (23) : base) is not particularly limited, and is generally about 1 : 1 to 1 : 50, and it is preferably 1 : 1.5 to 1 : 40, more preferably 1 : 1.8 to 1 : 35, further preferably 1 : 2 to 1 : 30 from the viewpoint of the yield and reaction efficiency. When $M^4$ and $M^5$ are boron atoms, the molar ratio of the compound (23) and the base (compound (23) : base) is preferably 1 : 1 to 1 : 10, more preferably 1 : 1.5 to 1 : 8, further preferably 1 : 1.8 to 1 : 6, especially preferably 1 : 2 to 1 : 5. When $M^4$ and $M^5$ are silicon atoms, the molar ratio of the compound (23) and the base (compound (23) : base) is preferably 1 : 1 to 1 : 50, more preferably 1 : 5 to 1 : 40, further preferably 1 : 8 to 1 : 35, especially preferably 1 : 10 to : 35.

As a solvent in which the compound (8) and/or compound (9) is reacted with the compound (23) in the presence of the metal catalyst in the third step, one similar to the solvent to be used in the first step may be preferably used. In particular, toluene, tetrahydrofuran, dioxane and N,N-dimethylformamide are especially preferable.

In the third step, the use amount of the solvent based on 1 g of the compound (23) is not particularly limited, and is generally about 1 mL or more and about 50 mL or less, and it is preferably 5 mL or more and 40 mL or less, more preferably 8 mL or more and 35 mL or less, further preferably 10 mL or more and 30 mL or less from the viewpoint of the yield and reaction efficiency.

In the third step, the reaction temperature is not particularly limited, and it is preferably 0°C or higher and 200°C or lower, more preferably 30°C or higher and 180°C or lower, further preferably 40°C or higher and 150°C or lower for improving the reaction yield.

3-4. Fourth step

Preferably, the production method according to the present invention includes the following fourth step.

The fourth step: a step of reacting a base and a tin halide compound with the compound represented by the formula (4) to prepare a compound represented by the formula (5).

As the base to be reacted with the compound (4) in the fourth step, any of the bases shown as examples in the second step may be used, and among them, alkyl metal amides are preferable, and lithium diisopropylamide is especially preferable.

In the fourth step, the molar ratio of the compound (4) and the base (compound (4) : base) is not particularly limited, and is generally about 1 : 1 to 1 : 5, and it is preferably 1 : 1.1 to 1 : 4, more preferably 1 : 1.5 to 1 : 3, further preferably 1 : 1.8 to 1 : 2.5 from the viewpoint of the yield and reaction efficiency.

Examples of tin halide compound to be reacted with the compound (4) together with the base in the fourth step include alkyltin halide compounds, cycloalkyltin halide compounds and aryltin halide compounds. Examples of the alkyltin halide compound include triethyltin chloride, tripropyltin chloride, tributyltin chloride, trimethyltin bromide, triethyltin bromide, tripropyltin bromide and tributyltin bromide. Examples of the cycloalkyltin halide compound include tricyclohexyltin chloride and tricyclohexyltin bromide. Examples of the aryltin halide compound include triphenyltin chloride, tribenzyltin chloride, triphenyltin bromide and tribenzyltin bromide. Among them, alkyltin halide compounds are preferable, and trimethyltin chloride and tributyltin chloride are more preferable.

In the fourth step, the molar ratio of the compound (4) and the halogenated tin compound (compound (4) : halogenated tin compound) is not particularly limited, and is generally about 1 : 1 to 1 : 5, and it is preferably 1 : 1.1 to 1 : 4, more preferably 1 : 1.5 to 1 : 3, further preferably 1 : 1.8 to 1 : 2.5 from the viewpoint of the yield and reaction efficiency.

The molar ratio of the base and the tin halide compound (base : tin halide compound) is, for example, about 1 : 0.5 to 1 : 2.0, preferably 1 : 0.6 to 1 : 1.7, more preferably 1 : 0.7 to 1 : 1.5, further preferably 1 : 0.8 to 1 : 1.2.

The base and the tin halide compound may be simultaneously reacted with the compound (4), and from the viewpoint of the reaction yield, it is preferable that the base is first reacted with the compound (4), and the tin halide compound is then reacted. In the fourth step, the temperature at which the compound (4) is reacted with the base, and the tin halide compound is then added is preferably equal to or less than room temperature, more preferably 0°C or lower from the viewpoint of suppressing generation of byproducts.

In the fourth step, the solvent in which the base and the tin halide compound are reacted with the compound

(4) is not particularly limited, and ether-based solvents, hydrocarbon-based solvents and so on may be used. Examples of the ether-based solvent include diethyl ether, dipropyl ether, diisopropyl ether, dibutyl ether, tetrahydrofuran, methyltetrahydrofuran, dimethoxyethane, cyclopentyl methyl ether, t-butyl methyl ether and dioxane. Examples of the hydrocarbon-based solvent include pentane, hexane, heptane, benzene, toluene and xylene. Among them, tetrahydrofuran is preferable. One solvent may be used alone, or two or more solvents may be used in combination.

In the fourth step, the use amount of the solvent based on 1 g of the compound (4) is not particularly limited, and is generally about 1 mL or more and about 70 mL or less, and it is preferably 5 mL or more and 60 mL or less, more preferably 10 mL or more and 50 mL, further preferably 20 mL or more and 45 mL or less from the viewpoint of the yield and reaction efficiency.

3-5. Coupling reaction

Further, using a coupling reaction, structural units according to the present invention and structural units which are combined with the structural units according to the present invention to form a donor-acceptor-type macromolecular compound can be alternately arranged to produce a macromolecular compound according to the present invention.

The coupling reaction can be carried out by reacting the compound (5) with any of compounds represented by the following formulae (C1) to (C34) in the presence of a metal catalyst.

[Chemical Formula 23]

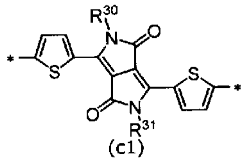
(c1)
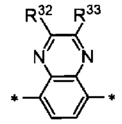
(c2)
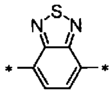
(c3)
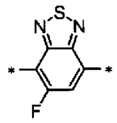
(c4)
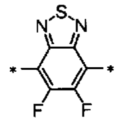
(c5)
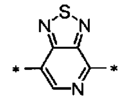
(c6)
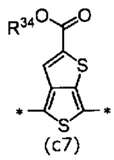
(c7)
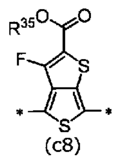
(c8)
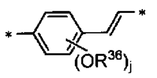
(c9)
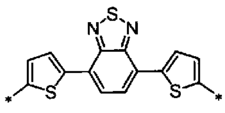
(c10)
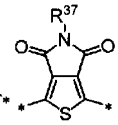
(c11)
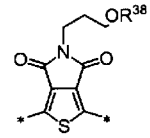
(c12)
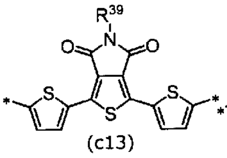
(c13)
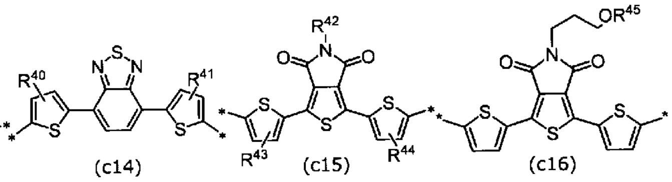
(c14) (c15) (c16)
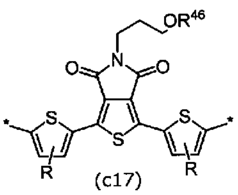
(c17)
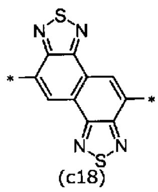
(c18)
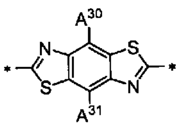
(c19)
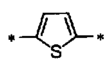
(c20)
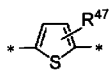
(c21)
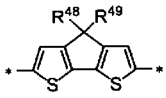
(c22)
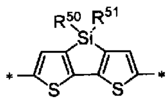
(c23)
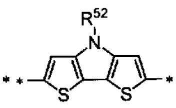
(c24)
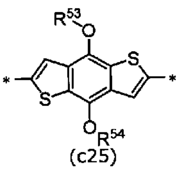
(c25)
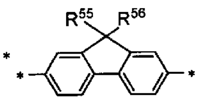
(c26)
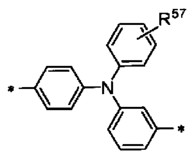
(c27)
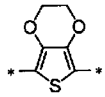
(c28)
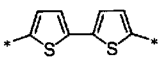
(c29)
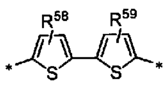
(c30)
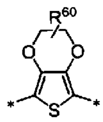
(c31)
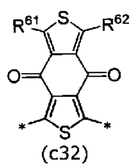
(c32)
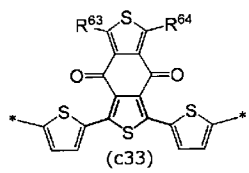
(c33)
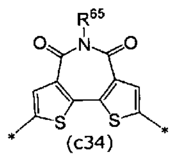
(c34)

[in the formulae (C1) to (C34), R and $R^{30}$ to $R^{65}$ each independently represent the same group as $R^{15}$; $A^{30}$ and $A^{31}$ each independently represent an alkoxy group, a thioalkoxy group, a thiophene ring optionally substituted by a hydrocarbon group or an organosilyl group, a thiazole ring optionally substituted by a hydrocarbon group or an organosilyl group, or a phenyl group optionally substituted by a hydrocarbon group, an alkoxy group, a thioalkoxy group, an organosilyl group, a halogen atom, or a trifluoromethyl group; and j represents an integer of 0 to 4; Y represents a halogen atom.].

The groups represented by the above formulae (C1) to (C18) and (C32) to (C34) are groups which serve as an acceptor unit, and the groups represented by the formulae (C20) to (C31) are groups which serve as a donor unit. The group represented by the formula (C19) may serve as an acceptor unit or serve as a donor unit depending on the type of $A^{30}$ and $A^{31}$.

Among the compounds represented by formulas (C1) to (C34), it is preferably a structure resulting from fusing heteroaryl groups and it is further preferably a group that acts as an acceptor unit. Among them, a structure resulting from binding and/or fusing thiophene rings is more preferable, and a structure represented by the formula (C1), (C7), (C8), (C10) to (C17), or (C32) to (C34) is particularly preferable.

The molar ratio of the compound (5) and any one of compounds represented by the formulae (C1) to (C34) is preferably in the range of 1 : 99 to 99 : 1, preferably in the range of 20 : 80 to 80 : 20, preferably in the range of 40 : 60 to 60 : 40.

The metal catalyst for coupling is preferably a transition metal catalyst, and examples of the transition metal catalyst include palladium-based catalyst, nickel-based catalysts, iron-based catalysts, copper-based catalysts, rhodium-based catalysts and ruthenium-based catalysts. Among them, palladium-based catalysts are preferable. Palladium of the palladium-based catalyst may be zero-valent or divalent.

As the palladium-based catalyst, any of the palladium-based catalysts shown as examples in the first step may be used, and one of these catalysts may be used alone, or two or more of these catalysts may be used in combination. Among them, dichlorobis(triphenylphosphine)palladium (II), tris(dibenzylideneacetone)dipalladium (0), tris(dibenzylideneacetone)dipalladium (0) chloroform adducts and tris(dibenzylideneacetone)dipalladium (0)-xdba adducts are especially preferable.

In the coupling step, the molar ratio of the compound represented by the formula (5) and the metal catalyst (compound (4) : metal catalyst) is not particularly limited, and is generally about 1 : 0.0001 to 1 : 0.5, and it is preferably 1 : 0.001 to 1 : 0.3, more preferably 1 : 0.005 to 1 : 0.2, further preferably 1 : 0.01 to 1 : 0.1 from the viewpoint of the yield and reaction efficiency.

In the coupling reaction, a specific ligand may be coordinated to the metal catalyst. As the ligand, any of the ligands shown as examples in the first step may be used, and a catalyst to which any one of these ligands is coordinated may be used in the reaction. One ligand may be used alone, or two or more ligands may be used in combination. Among them, triphenylphosphine, tris(o-tolyl)phosphine and tris(2-methoxyphenyl)phosphine are preferable.

When a ligand is coordinated to the metal catalyst in the coupling step, the molar ratio of the metal catalyst and the ligand (metal catalyst : ligand) is not particularly limited, and is generally about 1 : 0.5 to 1 : 10, and it is preferably 1 : 1 to 1 : 8, more preferably 1 : 1 to 1 : 7, further preferably 1 : 1 to 1 : 5 from the viewpoint of the yield and reaction efficiency.

The solvent in which the compound (5) is reacted with any one of compounds represented by the formulae (c1) to (c34) in the coupling reaction is not particularly limited as long as the reaction is not affected, and previously known solvents, for example ether-based solvents, aromatic-based solvents, ester-based solvents, hydrocarbon-based solvents, halogen-based solvents, ketone-based solvents, amide-based solvents and so on may be used. Examples of the ether-based solvent include diethyl ether, dipropyl ether, diisopropyl ether, dibutyl ether, tetrahydrofuran, methyltetrahydrofuran, dimethoxyethane, cyclopentyl methyl ether, tert-butyl methyl ether and dioxane. Examples of the aromatic-based solvent include benzene, toluene, xylene, mesitylene, chlorobenzene, dichlorobenzene and tetralin. Examples of the ester-based solvent include methyl acetate, ethyl acetate, propyl acetate, isopropyl acetate and butyl acetate. Examples of the hydrocarbon-based solvent include pentane, hexane, heptane, octane and decalin. Examples of the halogen-based solvent include dichloromethane, chloroform, dichloroethane and dichloropropane. Examples of the ketone-based solvent include acetone, methyl ethyl ketone and methyl isobutyl ketone. Examples of the amide-based solvent include N,N-dimethylformamide, N,N-dimethylacetamide, 1,3-dimethyl-2-imidazolidinone and 1,3-dimethyl-3,4,5,6-tetrahydro-(1H)-pyrimidinone. In addition, nitrile-based solvents such as acetonitrile, sulfoxide-based solvents such as dimethylsulfoxide, and sulfone-based solvents such as sulfolane may be used. Among them, tetrahydrofuran, toluene, xylene, chlorobenzene and N,N-dimethylformamide are preferable, and chlorobenzene is especially preferable. One solvent may be used alone, or two or more solvents may be used in combination.

In the coupling step, the use amount of the solvent based on 1 g of the total of the compound (5) and any one of compounds represented by the formulae (C1) to (C34) is not particularly limited, and is generally about 1 mL or more and about 150 mL or less, and it is preferably 5 mL or more and 100 mL or less, more preferably 8 mL or more and 90 mL or less, further preferably 10 mL or more and 80 mL or less from the viewpoint of the yield and reaction efficiency.

3-6. Organosilyl group removing step

The production method according to the present invention preferably includes, as necessary, an organosilyl group removing step. The organosilyl group removing step is preferably the following organosilyl group removing step 1 or organosilyl group removing step 2.

Organosilyl group removing step 1: a step of reacting a base with the compound having the organosilyl group in an alcohol-based solvent to remove the organosilyl group.

Organosilyl group removing step 2: a step of reacting a fluorine compound with the compound having the organosilyl group to remove the organosilyl group.

In the production method according to the present invention, the organosilyl group removing step may be performed on any of the compounds prepared in the above first to fourth steps or may be performed on the macromolecular compound prepared in the above coupling step. That is, the organosilyl group removing step may be performed between the first step and the second step, may be performed between the second step and the third step, may be performed

between the third step and the fourth step, may be performed between the fourth step and the coupling step, or may be performed after the coupling step.

Organosilyl group removing step 1

Examples of the base to be used in the organosilyl group removing step 1 include alkali metal salt compounds such as sodium hydroxide, cesium hydroxide, lithium carbonate, sodium carbonate, potassium carbonate and cesium carbonate; alkali earth metal salt compounds such as magnesium hydroxide, calcium hydroxide, barium hydroxide, magnesium carbonate, calcium carbonate and barium carbonate; and alkoxy alkali metal compounds such as lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-amylalkoxide, sodium tert-amylalkoxide and potassium tert-amylalkoxide. Particularly, the base is preferably an alkoxy alkali metal compound, more preferably sodium carbonate, potassium carbonate or cesium carbonate.
onate.

In the organosilyl group removing step 1, the molar ratio of the compound prepared having the organosilyl group and the base (compound : base) is not particularly limited, and is generally about 1 : 0.01 to 1 : 10, and it is preferably 1 : 0.03 to 1 : 8, more preferably 1 : 0.05 to 1 : 6, further preferably 1 : 0.1 to 1 : 5 from the viewpoint of the yield and reaction efficiency. The reaction proceeds even when the molar amount of the base is equivalent to that of the catalyst.

Examples of the alcohol-based solvent to be used in the organosilyl group removing step 1 include methanol, ethanol, propanol, isopropyl alcohol, butanol, isobutyl alcohol, tert-butanol, pentanol, hexanol, cyclohexanol, heptanol, octanol and 2-ethylhexanol, and methanol and ethanol are especially preferable.

In the organosilyl group removing step 1, the use amount of the alcohol-based solvent based on 1 g of the compound having the organosilyl group is not particularly limited, and is generally about 1 mL or more and about 50 mL or less, and it is preferably 5 mL or more and 40 mL or less, more preferably 8 mL or more and 35 mL or less, further preferably 10 mL or more and 30 mL or less from the viewpoint of the yield and reaction efficiency.

In the organosilyl group removing step 1, the reaction temperature is not particularly limited, and it is preferably 0°C or higher and 200°C or lower, more preferably 10°C or higher and 180°C or lower, further preferably 20°C or higher and 150°C or lower for improving the reaction yield.

Organosilyl group removing step 2

Examples of the fluorine compound to be used in the organosilyl group removing step 2 include lithium fluoride, sodium fluoride, potassium fluoride, cesium fluoride, magnesium fluoride, calcium fluoride, barium fluoride, ammonium fluoride, tetramethylammonium fluoride, tetraethylammonium fluoride, tetrapropylammonium fluoride, tetrabutylammonium fluoride, tetrapentylammonium fluoride and tetrahexylammonium fluoride, and sodium fluoride, potassium fluoride, tetraethylammonium fluoride and tetrabutylammonium fluoride are preferable.
e are preferable.

In the organosilyl group removing step 2, the molar ratio of the compound having an organosilyl group and the fluorine compound (compound : fluorine compound) is not particularly limited, and is generally about 1 : 1 to 1 : 10, and it is preferably 1 : 1.5 to 1 : 8, more preferably 1 : 1.8 to 1 : 6, further preferably 1 : 2 to 1 : 5 from the viewpoint of the yield and reaction efficiency.

In the case of causing an organosilyl group removing reaction with respect to any of the compounds each having the organosilyl group obtained in the above first to fourth steps, ones similar to the solvents used in the first to fourth steps may each be preferably used as a solvent to be used in the organosilyl group removing step 2. Among them, tetrahydrofuran, dioxane, toluene, and N,N-dimethylformamide are particularly preferable. In the case of causing an organosilyl group removing reaction with respect to the macromolecular compound obtained in the coupling step, ones similar to the solvents used in the coupling step may be preferably used. Among them, chlorobenzene, ortho-dichlorobenzene, toluene, and xylene are particularly preferable.

In the organosilyl group removing step 2, the use amount of the solvent based on 1 g of the compound having the organosilyl group is not particularly limited, and is generally about 1 mL or more and about 50 mL or less, and it is preferably 5 mL or more and 40 mL or less, more preferably 8 mL or more and 35 mL or less, further preferably 10 mL or more and 30 mL or less from the viewpoint of the yield and reaction efficiency.

In the organosilyl group removing step 2, the reaction temperature is not particularly limited, and it is preferably 0°C or higher and 200°C or lower, more preferably 10°C or higher and 180°C or lower, further preferably 20°C or higher and 150°C or lower for improving the reaction yield.

EXAMPLES

Hereinafter, the present invention will be described more in detail by way of examples, and the present invention is not limited to the following examples. Of course, the present invention can be carried out while changes are appropriately made without departing from the spirit of the foregoing and following descriptions, and these changes are all encompassed in the technical scope of the present invention. In the following, "%" means "% by mass" unless otherwise specified.

Measurement methods used in examples are as described below.

(NMR spectrum measurement)

For the benzobisthiazole compound, an NMR spectrum measurement was made using an NMR spectrum measuring apparatus ("400 MR" manufactured by Agilent, Inc. (former Varian, Inc.).

(High-resolution mass spectrum measurement)

For the benzobisthiazole compound, a high-resolution mass spectrum measurement was made using a mass spectrometer ("MicrOTOF" manufactured by Bruker Daltnics K.K.).

(Gel permeation chromatography (GPC))

For the benzobisthiazole compound, a molecular weight measurement was made using gel permeation chromatography (GPC). In the measurement, the benzobisthiazole compound was dissolved in a mobile phase solvent (chloroform) in a concentration of 0.5 g/L, the measurement was made under the following conditions, and the measured value was calculated based on a calibration curve prepared with polystyrene as a standard sample to calculate the number average molecular weight and weight average molecular weight of the benzobisthiazole compound. GPC conditions in the measurement are as described below.

Mobile phase: o-dichlorobenzene (0.5% BHT)
Flow rate: 0.3 mL/min
Apparatus: HLC-8321GPC/HT (manufactured by TOSOH CORPORATION)
Column: Waters Styragel HT 3,4,6E

IR Spectrum

For the benzobisthiazole compound, an IR spectrum measurement was made using an infrared spectrometer ("FT/IR-6100" manufactured by JASCO Corporation).

Ultraviolet-visible absorption spectrum

The obtained benzobisthiazole compound was dissolved in chloroform in a concentration of 0.03 g/L, and an ultraviolet-visible absorption spectrum measurement was made using an ultraviolet/visible spectrometer ("UV-3600 iPlus" manufactured by Shimadzu Corporation) and a cell having an optical path length of 1 cm.

Melting point measurement

For the benzobisthiazole compound, a melting point measurement was made using a melting point measuring apparatus ("M-560" manufactured by Buchi, Inc.).

Ionization potential measurement

The benzobisthiazole compound was deposited to form a film with a thickness of 50 nm to 100 nm on a glass plate. For the film, an ionization potential measurement was made under a vacuum atmosphere using an ionization energy measurement system ("BIP-KV202GD" manufactured by Bunkoukeiki Co.,Ltd.)

Synthesis example 1

Synthesis of HDT-Cl

[Chemical Formula 61]

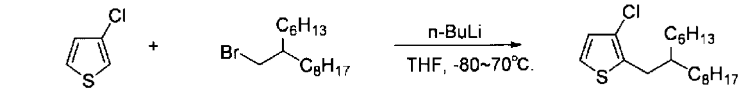


3-ChloroThiophene HDB HDT-Cl

3-chlorothiophene (2.2 g, 18.5 mmol) and tetrahydrofuran (44 mL) were added in a 200 mL flask and cooled to -80°C, and then n-butyllithium (1.6 M solution, 11.5 mL, 5.9 mmol) was added dropwise, and then the mixture was stirred for 30 minutes. Then, HDB (1.8 g, 5.9 mmol) was added, and the mixture was heated to 70°C and stirred for 15 hours. After the reaction was completed, water was added, and then the reaction product was subjected to extraction twice with hexane, and then the resulting organic layer was washed with water, and then, dried with anhydrous magnesium sulfate. Then, the organic layer was filtered and concentrated to obtain a crude product, and the crude product was purified by distillation to prepare 1.58 g of HDT-Cl as a transparent oil (yield: 78%).

Generation of an intended compound was confirmed by $^1$H-NMR measurement.

$^1$H NMR (400 MHz, $CDCl_3$): δ7.07 (d, J = 5.2 Hz, 1H), 6.82 (d, J = 5.2 Hz, 1H), 2.69 (d, J= 7.2 Hz, 2H), 1.70 (m, 1H), 1.25 (m, 24H), 0.85 (m, 6H)

Synthesis example 2

Synthesis of HDT-Cl-SB

[Chemical Formula 62]

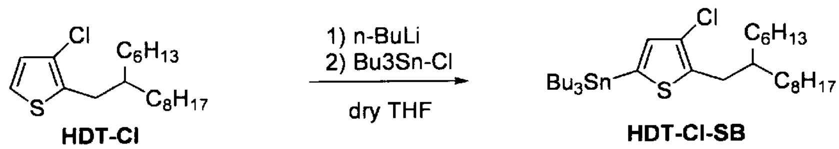


HDT-Cl HDT-Cl-SB

The HDT-Cl (3.8 g, 11.0 mmol) and tetrahydrofuran (32 mL) were added in a 200 mL flask and cooled to -80°C, and then n-butyllithium (1.6 M solution, 7.7 mL, 12.3 mmol) was added dropwise, and then the mixture was stirred for 1 hour. Then, tributyltin chloride (3.2 mL, 11.8 mmol) was added, and the mixture was heated to 70°C and stirred for 15 hours. After the reaction was completed, water was added, and then the reaction product was subjected to extraction twice with toluene, and then the resulting organic layer was washed with water, and then, dried with anhydrous magnesium sulfate. Then, the organic layer was filtered and concentrated to obtain a crude product, and the crude product was purified by column chromatography (alumina, hexane) to prepare 6.45 g of HDT-Cl-SB as a transparent oil (yield: 92%).

Generation of an intended compound was confirmed by $^1$H-NMR measurement.

$^1$H NMR (400 MHz, $CDCl_3$): δ6.84 (s, 1H), 2.70 (d, J= 7.2 Hz, 2H), 1.70 (m, 1H), 1.25 (m, 30H), 0.85 (m, 15H)

Synthesis example 3

Synthesis of DBTH-HDTH-DC

[Chemical Formula 63]

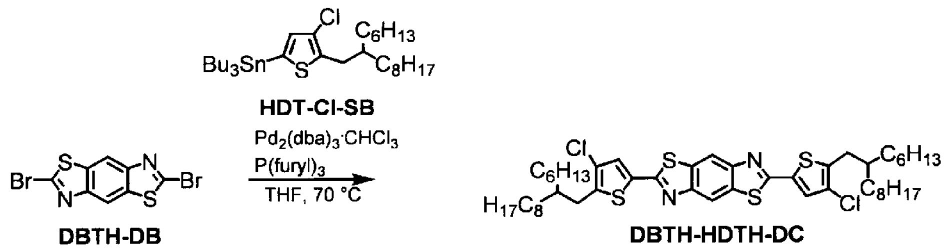


DBTH-DB (1.1 g, 3.14 mmol), the HDT-Cl-SB (4.7 g, 2.36 mmol), tris(2-furyl)phosphine (116 mg, 0.50 mmol), tris(dibenzylideneacetone)dipalladium (0)-chloroform adduct (131 mg, 0.13 mmol), and tetrahydrofuran (22 mL) were added in a 100 mL flask and reacted at 70°C for 14 hours. After the reaction was completed, the reaction product was cooled to room temperature, and then water was added, and then the mixture was subjected to extraction twice with chloroform, and then the resulting organic layer was washed with water, and then, dried with anhydrous magnesium sulfate. Then, the organic layer was filtered and concentrated to obtain a crude product, and the crude product was purified by column chromatography (silica gel, chloroform/hexane=1/1) to prepare 2.4 g of DBTH-HDTH-DC as a yellow green solid (yield: 89%).

Generation of an intended compound was confirmed by $^1$H-NMR measurement.

$^1$H NMR (400 MHz, $CDCl_3$): δ 8.40 (s, 2H), 7.44 (s, 2H), 2.77 (d, J= 7.2 Hz, 4H), 1.75 (m, 2H), 1.25 (m, 48H), 0.85 (m, 12H).

Synthesis example 4

Synthesis of DBTH-HDTH-DC-DTMS

[Chemical Formula 64]

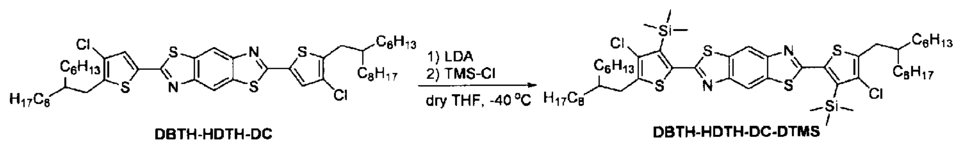


The DBTH-HDTH-DC (1.2 g, 1.34 mmol) and tetrahydrofuran (20 mL) were added in a 50 mL flask and were cooled to -40°C, and then lithium diisopropylamide (2 M solution, 1.55 mL, 3.1 mmol) was added dropwise, and then the mixture was stirred for 60 minutes. Then, trimethylsilyl chloride (0.53 mL, 4.20 mmol) was added, and then the mixture was caused to undergo a reaction at room temperature for 2 hours. After the reaction was completed, water was added, and then the reaction product was subjected to extraction with chloroform, and then the resulting organic layer was washed with saturated sodium bicarbonate water and then with a saturated saline solution, and then was dried with anhydrous magnesium sulfate. Then, the organic layer was filtered and concentrated to prepare 1.43 g of a crude product (crude yield: 102%) containing DBTH-HDTH-DC-DTMS as a main component.

Generation of an intended compound was confirmed by $^1$H-NMR measurement.

$^1$H NMR (400 MHz, $CDCl_3$): δ 8.52 (s, 2H), 2.78 (d, J= 7.2 Hz, 4H), 1.72 (m, 2H), 1.25 (m, 48H), 0.85 (m, 12H), 0.25 (m, 18H).

Synthesis example 5

Synthesis of DI-DBTH-HDTH-DC-DTMS

[Chemical Formula 65]

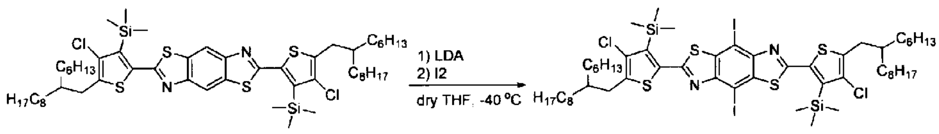


DBTH-HDTH-DC-DTMS

DI-DBTH-HDTH-DC-DTMS

The DBTH-HDTH-DC-DTMS (crude product: 1.43 g) and tetrahydrofuran (28 mL) were added in a 50 mL flask and cooled to -40°C, and then lithium diisopropylamide (2 M solution, 2.2 mL, 4.4 mmol) was added dropwise, and then the mixture was stirred for 30 minutes. Then, iodine (1.06 g, 4.18 mmol) was added, and then the mixture was caused to undergo a reaction at room temperature for 2 hours. After the reaction was completed, 10% sodium hydrogen sulfite was added, and then the reaction product was subjected to extraction with chloroform, and then the resulting organic layer was washed with saturated sodium bicarbonate water and then with a saturated saline solution, and then, dried with anhydrous magnesium sulfate. Then, the organic layer was filtered and concentrated to obtain a crude product, and then the crude product was purified by column chromatography (silica gel, chloroform/hexane=1/1) to prepare 1.19 g of DI-DBTH-HDTH-DC-DTMS as a yellow solid (two-step overall yield: 68%).

Generation of an intended compound was confirmed by $^1$H-NMR measurement.

$^1$H NMR (400 MHz, $CDCl_3$): δ2.78 (d, J= 7.2 Hz, 4H), 1.72 (m, 2H), 1.25 (m, 48H), 0.85 (m, 12H), 0.25 (m, 18H).

Synthesis example 6

Synthesis of DTH-DBTH-HDTH-DC-DTMS

[Chemical Formula 66]

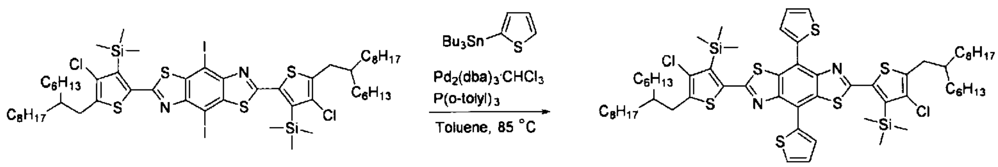


DI-DBTH-HDTH-DC-DTMS

DTH-DBTH-HDTH-DC-DTMS

The DI-DBTH-HDTH-DC-DTMS (800 mg, 0.63 mmol), tributylthiophen-2-ylstannane (0.59 g, 1.58 mmol), tris(2-tolyl)phosphine (31 mg, 0.10 mmol), a tris(dibenzylideneacetone)dipalladium (0)-chloroform adduct (26 mg, 0.025 mmol), and toluene (8 mL) were added in a 20 mL flask and reacted at 85°C for 14 hours. After the reaction was completed, the reaction product was cooled to room temperature, and then water was added, and then the mixture was subjected to extraction twice with chloroform, and then the resulting organic layer was washed with water, and then, dried with anhydrous magnesium sulfate. Then, the organic layer was filtered and concentrated to obtain a crude product, and the crude product was purified by column chromatography (silica gel, chloroform/hexane=1/1 - chloroform) to prepare 600 mg of DTH-DBTH-HDTH-DC-DTMS as a yellow solid (yield: 81%).

Generation of an intended compound was confirmed by $^1$H-NMR measurement.
$^1$H NMR (400 MHz, $CDCl_3$): δ7.86 (dd, J = 4.0, 0.8 Hz, 2H), 7.52 (dd, J = 5.2, 0.8 Hz, 2H), 7.21 (dd, J = 5.2, 4.0 Hz, 2H), 2.76 (d, J= 7.2 Hz, 4H), 1.70 (m, 2H), 1.25 (m, 48H), 0.85 (m, 12H), 0.25 (m, 18H)

Synthesis example 7

Synthesis of DTH-DBTH-HDTH-DC-DTMS-DSM

[Chemical Formula 67]

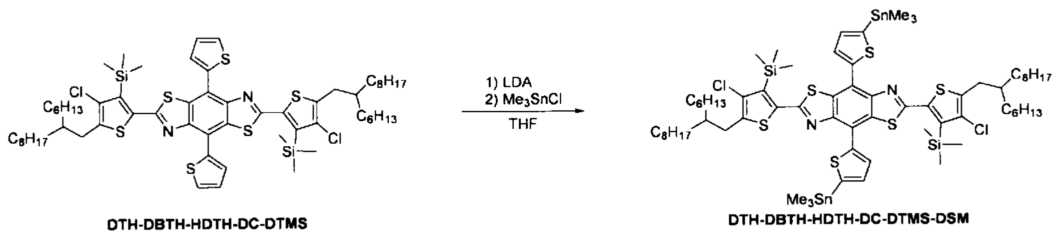


DTH-DBTH-HDTH-DC-DTMS

DTH-DBTH-HDTH-DC-DTMS-DSM

The DTH-DBTH-HDTH-DC-DTMS (216 mg, 0.18 mmol) and tetrahydrofuran (4.4 mL) were added in a 20 mL flask and cooled to -80°C, and then lithium diisopropylamide (2 M solution, 0.36 mL, 0.72 mmol) was added dropwise, and then the mixture was stirred for 30 minutes. Then, trimethyltin chloride (1 M solution, 720 μL, 0.72 mmol) was added, and then the mixture was heated to room temperature and stirred for 30 minutes. After the reaction was completed, water was added, and then the reaction product was subjected to extraction twice with toluene, and the resulting organic layer was washed with water, and then, dried with anhydrous magnesium sulfate. Then, the organic layer was filtered and concentrated to obtain a crude product, and then the crude product was purified by GPC-HPLC (JAIGEL-1H, 2H, chloroform) to prepare 136 mg of DTH-DBTH-HDTH-DC-DTMS-DSM as a yellow solid (yield: 50%).

Generation of an intended compound was confirmed by $^1$H-NMR measurement.
$^1$H NMR (400 MHz, $CDCl_3$): δ7.98 (d, J = 3.2 Hz, 2H), 7.28 (d, J = 3.2 Hz, 2H), 2.78 (d, J= 7.2 Hz, 4H), 1.70 (m, 2H), 1.25 (m, 48H), 0.85 (m, 12H), 0.40 (m, 18H), 0.25 (m, 18H)

Example 1

Synthesis of P-THDT-DC-DBTH-DMO-IMTH

[Chemical Formula 68]

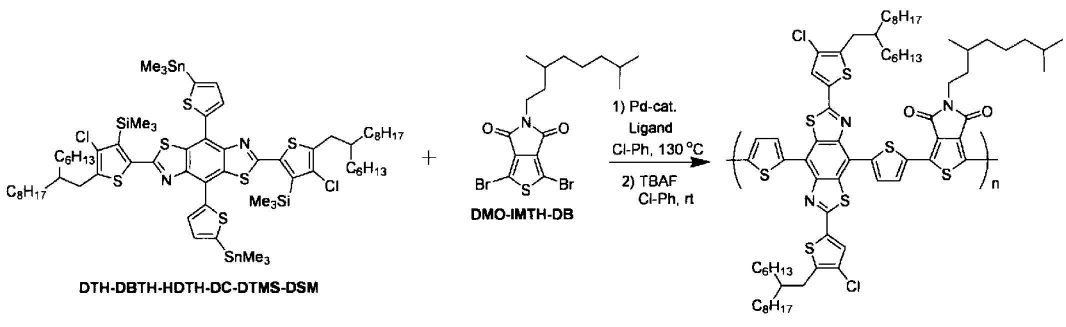


DTH-DBTH-HDTH-DC-DTMS-DSM

DMO-IMTH-DB

P-THDT-DC-DBTH-DMO-IMTH

The DTH-DBTH-HDTH-DC-DTMS-DSM (75 mg, 0.050 mmol), DMO-IMTH-DB (22 mg, 0.050 mmol), tris(dibenzylideneacetone)dipalladium (0)-xdba adduct (2.3 mg, 1.9 μmol), tris(2-methoxyphenyl)phosphine (2.8 mg, 7.9 μmol), and chlorobenzene (3 mL) were added in a 10 mL flask and reacted at 130°C for 23 hours. The reaction liquid was cooled to room temperature, and then tetrabutylammonium fluoride (a solution containing about 1 mol/L of tetrahydrofuran, 0.17 mL, 0.17 mmol) was added, and then the mixture was caused to undergo a reaction at room temperature for 4.5 hours. After the reaction was completed, water was added, and then the reaction product was subjected to extraction twice with chloroform, and then the resulting organic layer was washed with water. Then, the organic layer was filtered and concentrated to obtain a crude product, and then methanol (40 mL) was added to the crude product to precipitate a solid. The solid was obtained through filtering. The obtained solid was subjected to Soxhlet washing (methanol, acetone, and hexane). Then, the solid was subjected to Soxhlet extraction (chloroform) to prepare 51 mg (83%) of P-THDT-DC-DBTH-DMO-IMTH as a black solid.

Ionization potential: 5.43 eV (HOMO -5.43 eV)
GPC measurement result: Mw (weight average molecular weight): 24200
Mn (number average molecular weight): 10300

(Production of Mixed Solution 1 Made from p-Type Semiconductor Compound and n-Type Semiconductor Compound)

As a p-type semiconductor compound, a macromolecular compound having the structure of the P-THDT-DC-DBTH-DMO-IMTH prepared in the above Example 1 was used. As an n-type semiconductor compound, PC61BM (phenyl-C61-butyric acid methyl ester "NS-E100H" manufactured by Frontier Carbon Corporation) was used. The p-type semiconductor compound and the n-type semiconductor compound were, in a mass ratio of 1:1, dissolved in chlorobenzene. The total concentration of the p-type semiconductor compound and the n-type semiconductor compound was set to 2.0% by mass. The obtained solution was stirred and mixed at a temperature of 100°C for 2 hours or longer on a hot stirrer. The solution having been stirred and mixed was filtered with a 0.45 μm filter to produce a mixed solution 1 made from the p-type semiconductor compound and the n-type semiconductor compound.

(Production of Mixed Solution 2 Made from p-Type Semiconductor Compound and n-Type Semiconductor Compound)

As a p-type semiconductor compound, a macromolecular compound having the structure of the P-THDT-DC-DBTH-DMO-IMTH prepared in the above Example 1 was used. As an n-type semiconductor compound, IT4F ((3,9-bis(2-methylene-((3-1,1-dicyanomethylene)-6,7-difluoro)-indanone))-5,5,11,11-tetrakis(4-hexylphenyl)-dithieno[2,3-d:2'3'-d']-s-indaceno[1,2-b:5,6-b']dithiophene (manufactured by 1-Material)) was used. The p-type semiconductor compound and the n-type semiconductor compound were, in a mass ratio of 1:1, dissolved in chlorobenzene. The total concentration of the p-type semiconductor compound and the n-type semiconductor compound was set to 2.0% by mass. The obtained solution was stirred and mixed at a temperature of 100°C for 2 hours or longer on a hot stirrer. The solution having been stirred and mixed was filtered with a 0.45 μm filter to produce a mixed solution 2 made from the p-type semiconductor compound and the n-type semiconductor compound.

Synthesis example 8

Synthesis of 2Br-4F-5TIPS-TH (Known compound)

[Chemical Formula 69]

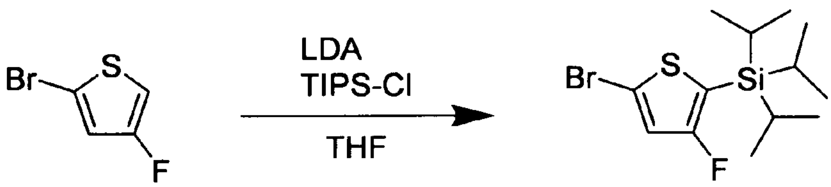


2Br-4F-TH 2Br-4F-5TIPS-TH

2Br-4F-TH (0.3 mL, 3.0 mmol) and tetrahydrofuran (1.5 mL) were added in a 20 mL flask and cooled to -80°C, and then lithium diisopropylamine (2 M solution, 1.57 mL, 3.1 mmol) was added dropwise, and then the mixture was stirred for 1 hour. Then, triisopropylsilyl chloride (0.95 mL, 4.5 mmol) was added, and then the mixture was heated to room temperature and stirred for 3 hours. After the reaction was completed, water was added, and then the reaction product was subjected to extraction twice with toluene, and then the resulting organic layer was washed with water, and then, dried with anhydrous magnesium sulfate. Then, the organic layer was filtered and concentrated to obtain a crude product, and the crude product was purified by column chromatography (silica gel, hexane) to prepare 0.89 g of 2Br-4F-5TIPS-TH as a transparent oil (yield: 88%).

Generation of an intended compound was confirmed by $^1$H-NMR measurement.
$^1$H NMR (400 MHz, $CDCl_3$): δ6.85 (d, J = 2.0 Hz, 1H), 1.32 (m, 3H), 1.11 (s, 18H)

Synthesis example 9

Synthesis of 2SB-4F-5TIPS-TH

[Chemical Formula 70]

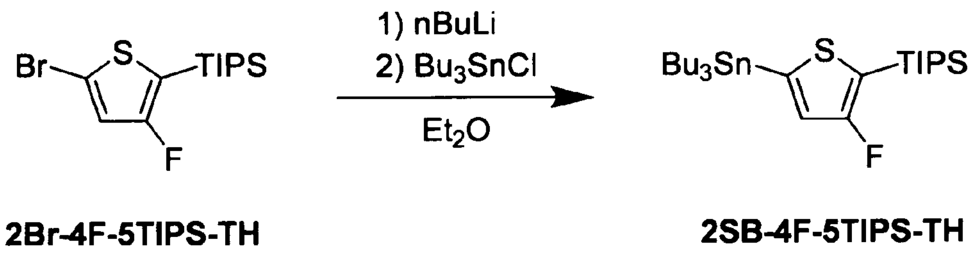


The 2Br-4F-5TIPS-TH (0.31 g, 0.93 mmol) and diethyl ether (4.7 mL) were added in a 20 mL flask and cooled to -70°C, and then n-butyllithium (1.6 M solution, 0.61 mL, 0.98 mmol) was added dropwise, and then the mixture was stirred for 1 hour. Then, tributyltin chloride (0.27 mL, 0.98 mmol) was added, and the mixture was heated to room temperature and stirred for 2 hours. After the reaction was completed, water was added, and then the reaction product was subjected to extraction twice with toluene, and then the resulting organic layer was washed with water, and then, dried with anhydrous magnesium sulfate. Then, the organic layer was filtered and concentrated to obtain a crude product, and the crude product was purified by distillation to prepare 0.19 g of 2SB-4F-5TIPS-TH as a transparent oil (yield: 37%).

Generation of an intended compound was confirmed by $^1$H-NMR measurement.

$^1$H NMR (400 MHz, $CDCl_3$): δ6.87 (d, J = 2.4 Hz, 1H), 1.55 (m, 6H), 1.25 (m, 9H), 1.11 (m, 24H) , 0.88 (m, 9H)

Synthesis example 10

Synthesis of DFTH-DBTH-HDTH

[Chemical Formula 71]

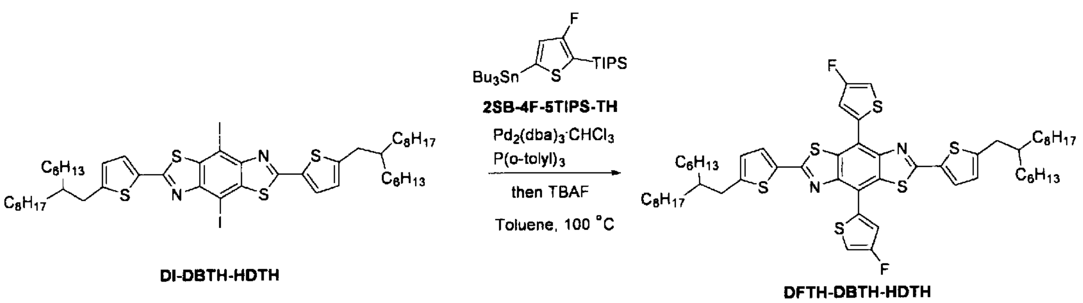


DI-DBTH-HDTH (130 mg, 0.12 mmol), the 2SB-4F-5TIPS-TH (0.17 g, 0.31 mmol), tris(2-tolyl)phosphine (3 mg, 0.01 mmol), tris(dibenzylideneacetone)dipalladium (0)-chloroform adduct (2.7 mg, 0.0024 mmol), and toluene (1.3 mL) were added in a 20 mL flask and reacted at 100°C for 17 hours. After the reaction was completed, the reaction product was cooled to room temperature, and then water was added, and then the mixture was subjected to extraction twice with toluene, and then the resulting organic layer was washed with water, and then, dried with anhydrous magnesium sulfate. Then, the organic layer was filtered and concentrated to obtain a crude product, and the crude product was purified by column chromatography (silica gel, chloroform/hexane=1/1 - chloroform) to prepare 64 mg of DFTH-DBTH-HDTH as a yellow solid (yield: 52%).

Generation of an intended compound was confirmed by $^1$H-NMR measurement.

$^1$H NMR (400 MHz, $CDCl_3$): δ7.75 (d, J = 1.6 Hz, 2H), 7.56 (d, J = 4.0 Hz, 2H), 6.93 (d, J = 1.6 Hz, 2H), 6.81 (d, J = 4.0 Hz, 2H), 2.80 (d, J= 7.2 Hz, 4H), 1.70 (m, 2H), 1.25 (m, 48H), 0.87 (m, 12H)

Synthesis example 11

Synthesis of DFTH-DBTH-HDTH-DSM

[Chemical Formula 72]

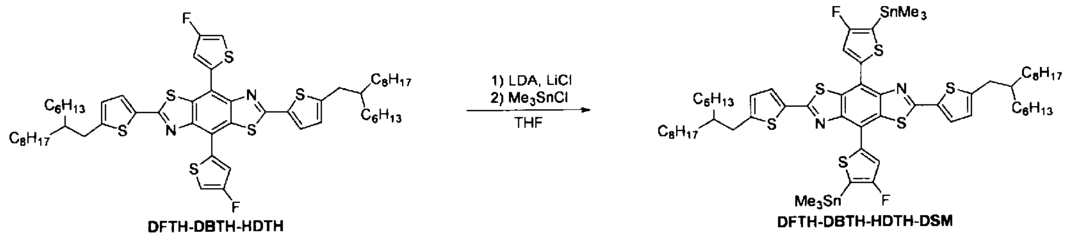


DFTH-DBTH-HDTH

DFTH-DBTH-HDTH-DSM

The DFTH-DBTH-HDTH (480 mg, 0.48 mmol) and tetrahydrofuran (9.6 mL) were added in a 30 mL flask and cooled to -80°C, and then lithium diisopropylamide (2 M solution, 0.76 mL, 1.5 mmol) was added dropwise, and then the mixture was stirred for 30 minutes. Then, trimethyltin chloride (1 M solution, 1.67 mL, 1.67 mmol) was added, and then the mixture was heated to room temperature and stirred for 30 minutes. After the reaction was completed, water was added, and then the reaction product was subjected to extraction twice with toluene, and then the resulting organic layer was washed with water, and then, dried with anhydrous magnesium sulfate. Then, the organic layer was filtered and concentrated to obtain a crude product, and the crude product was purified by GPC-HPLC (JAIGEL-1H, 2H, chloroform) to prepare 540 mg of DFTH-DBTH-HDTH-DSM as a yellow solid (yield: 85%).

Generation of an intended compound was confirmed by $^1$H-NMR measurement.

$^1$H NMR (400 MHz, $CDCl_3$): $\delta$7.86 (d, J = 1.6 Hz, 2H), 7.56 (d, J = 3.6 Hz, 2H), 6.81 (d, J = 3.6 Hz, 2H), 2.78 (d, J= 7.2 Hz, 4H), 1.70 (m, 2H), 1.25 (m, 48H), 0.85 (m, 12H), 0.40 (m, 18H)

Example 2

Synthesis of P-DFTH-THDH-DBTH-DMO-IMTH

[Chemical Formula 73]

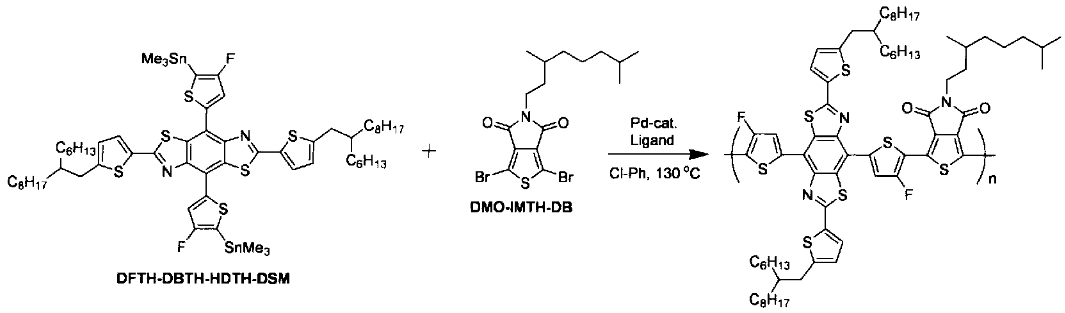


DFTH-DBTH-HDTH-DSM

DMO-IMTH-DB

P-DFTH-THDT-DBTH-DMO-IMTH

The DFTH-DBTH-HDTH-DSM (0.10 g, 0.075 mmol), DMO-IMTH-DB (34 mg, 0.075 mmol), tris(dibenzylideneacetone)dipalladium (0)-chloroform adduct (3 mg, 3.0 $\mu$mol), tris(2-methoxyphenyl)phosphine (4.4 mg, 8.5 $\mu$mol), and chlorobenzene (7 mL) were added in a 20 mL flask and reacted at 130°C for 23 hours. Methanol (40 mL) was added to the reaction liquid to precipitate a solid. The solid was obtained through filtering. The obtained solid was subjected to Soxhlet washing (methanol, acetone, and hexane). Then, the solid was subjected to Soxhlet extraction (chloroform) to prepare 78 mg (85%) of P-DFTH-THDH-DBTH-DMO-IMTH as a black solid.

Ionization potential: 5.16 eV (HOMO -5.16 eV)
GPC measurement result: Mw (weight average molecular weight): 21800
Mn (number average molecular weight): 11900

(Production of Mixed Solution 3 Made from p-Type Semiconductor Compound and n-Type Semiconductor Compound)

As a p-type semiconductor compound, a macromolecular compound having the structure of the P-DFTH-THDH-DBTH-HD-BTI prepared in the above Example 2 was used. As an n-type semiconductor compound, IT4F ((3,9-bis(2-methylene-((3-1,1-dicyanomethylene)-6,7-difluoro)-indanone))-5,5,11,11-tetrakis(4-hexylphenyl)-dithieno[2,3-d:2'3'-d']-s-indaceno[1,2-b:5,6-b']dithiophene (manufactured by 1-Material)) was used. The p-type semiconductor compound and the n-type semiconductor compound were, in a mass ratio of 1:1, dissolved in chlorobenzene. The total concentration of the p-type semiconductor compound and the n-type semiconductor compound was set to 2.0% by mass. The obtained solution was stirred and mixed at a temperature of 100°C for 2 hours or longer on a hot stirrer. The solution having been stirred and mixed was filtered with a 0.45 μm filter to produce a mixed solution 3 made from the p-type semiconductor compound and the n-type semiconductor compound.

Synthesis example 12

Synthesis of DFTH-DBTH-EHTH

[Chemical Formula 74]

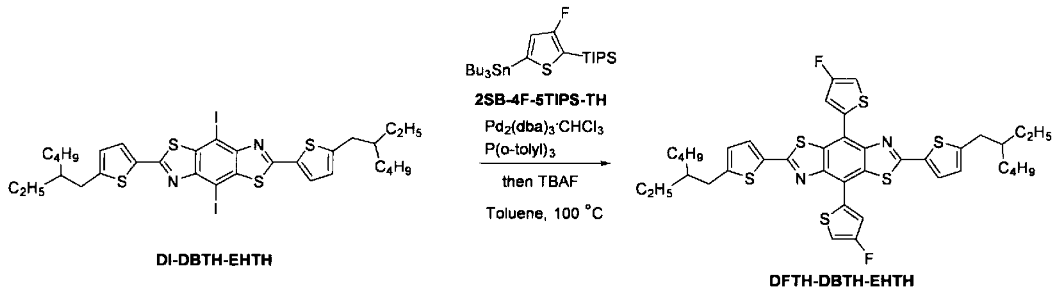


DI-DBTH-EHTH

DFTH-DBTH-EHTH

DI-DBTH-EHTH (100 mg, 0.12 mmol), 2SB-4F-5TIPS-TH (0.17 g, 0.31 mmol), tris(2-tolyl)phosphine (3 mg, 0.01 mmol), tris(dibenzylideneacetone)dipalladium (0)-chloroform adduct (2.7 mg, 0.0024 mmol), and toluene (1.3 mL) were added in a 20 mL flask and reacted at 100°C for 17 hours. After the reaction was completed, the reaction product was cooled to room temperature, and then water was added, and then the mixture was subjected to extraction twice with toluene, and then the resulting organic layer was washed with water, and then, dried with anhydrous magnesium sulfate. Then, the organic layer was filtered and concentrated to obtain a crude product, and the crude product was purified by column chromatography (silica gel, chloroform/hexane=1/1 - chloroform) to prepare 52 mg of DFTH-DBTH-EHTH as a yellow solid (yield: 55%).

Generation of an intended compound was confirmed by $^1$H-NMR measurement.
$^1$H NMR (400 MHz, $CDCl_3$): δ7.72 (d, J = 1.6 Hz, 2H), 7.56 (d, J = 4.0 Hz, 2H), 6.93 (d, J = 1.6 Hz, 2H), 6.80 (d, J = 4.0 Hz, 2H), 2.82 (m, 4H), 1.66 (m, 2H), 1.42 ? 1.30 (m, 16H), 0.90 (t J = 6.4 Hz, 6H), 0.88 (t, J = 6.4 Hz, 6H)

Synthesis example 13

Synthesis of DFTH-DBTH-EHTH-DSM

[Chemical Formula 75]

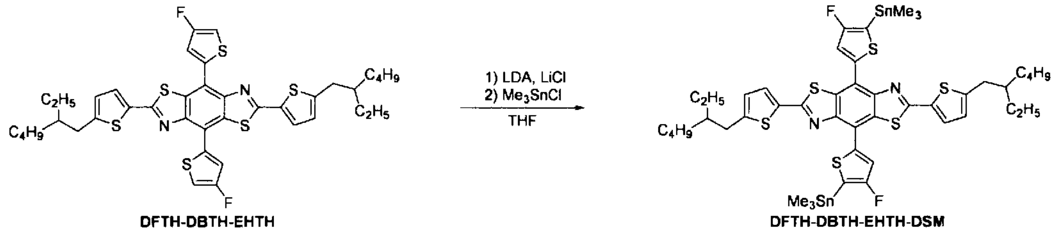

DFTH-DBTH-EHTH
DFTH-DBTH-EHTH-DSM

The DFTH-DBTH-EHTH (375 mg, 0.48 mmol) and tetrahydrofuran (9.6 mL) were added in a 30 mL flask and cooled to -80°C, and then lithium diisopropylamide (2 M solution, 0.76 mL, 1.5 mmol) was added dropwise, and then the mixture was stirred for 30 minutes. Then, trimethyltin chloride (1 M solution, 1.67 mL, 1.67 mmol) was added, and then the mixture was heated to room temperature and then stirred for 30 minutes. After the reaction was completed, water was added, and then the reaction product was subjected to extraction twice with toluene, and then the resulting organic layer was washed with water, and then, dried with anhydrous magnesium sulfate. Then, the organic layer was filtered and concentrated to obtain a crude product, and then the crude product was purified by GPC-HPLC (JAIGEL-1H, 2H, chloroform) to prepare 467 mg of DFTH-DBTH-EHTH-DSM as a yellow solid (yield: 88%).

Generation of an intended compound was confirmed by $^1$H-NMR measurement.

$^1$H NMR (400 MHz, $CDCl_3$): δ7.84 (d, J = 1.6 Hz, 2H), 7.52 (d, J = 3.6 Hz, 2H), 6.80 (d, J = 3.6 Hz, 2H), 2.80 (m, 4H), 1.66 (m, 2H), 1.42 ? 1.30 (m, 16H), 0.90 (t J = 6.4 Hz, 6H), 0.88 (t, J = 6.4 Hz, 6H), 0.40 (m, 18H)

Example 3

Synthesis of P-DFTH-TEHH-DBTH-DMO-IMTH

[Chemical Formula 76]

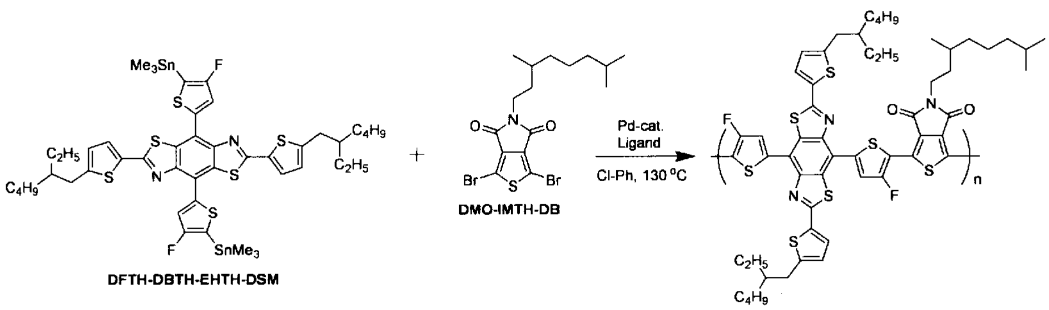

DFTH-DBTH-EHTH-DSM
DMO-IMTH-DB
P-DFTH-TEHT-DBTH-DMO-IMTH

The DFTH-DBTH-EHTH-DSM (0.083 g, 0.075 mmol, DMO-IMTH-DB (34 mg, 0.075 mmol), tris(dibenzylideneacetone)dipalladium (0)-chloroform adduct (3 mg, 3.0 μmol), tris(2-methoxyphenyl)phosphine (4.4 mg, 8.5 μmol), and chlorobenzene (7 mL) were added in a 20 mL flask and reacted at 130°C for 23 hours. Methanol (40 mL) was added to the reaction liquid to precipitate a solid. The solid was obtained through filtering. The obtained solid was subjected to Soxhlet washing (methanol, acetone, and hexane). Then, the solid was subjected to Soxhlet extraction (chloroform) to prepare 58 mg (70%) of P-DFTH-TEHH-DBTH-DMO-IMTH as a black solid.

Ionization potential: 5.18 eV (HOMO -5.18 eV)
GPC measurement result: Mw (weight average molecular weight): 23200
Mn (number average molecular weight): 12500

(Production of Mixed Solution 4 Made from p-Type Semiconductor Compound and n-Type Semiconductor Compound)

As a p-type semiconductor compound, a macromolecular compound having the structure of the P-DFTH-TEHH-DBTH-DMO-IMTH prepared in the above Example 3 was used. As an n-type semiconductor compound, IT4F ((3,9-bis(2-methylene-((3-1,1-dicyanomethylene)-6,7-difluoro)-indanone))-5,5,11,11-tetrakis(4-hexylphenyl)-dithieno[2,3-d:2'3'-d']-s-indaceno[1,2-b:5,6-b']dithiophene (manufactured by 1-Material) was used. The p-type semiconductor compound and the n-type semiconductor compound were, in a mass ratio of 1:1, dissolved in chlorobenzene. The total concentration of the p-type semiconductor compound and the n-type semiconductor compound was set to 2.0% by mass. The obtained solution was stirred and mixed at a temperature of 100°C for 2 hours or longer on a hot stirrer. The solution having been stirred and mixed was filtered with a 0.45 μm filter to produce a mixed solution 4 made from the p-type semiconductor compound and the n-type semiconductor compound.

Example 4

Synthesis of P-DFTH-THDH-DBTH-HD-BTI

[Chemical Formula 77]

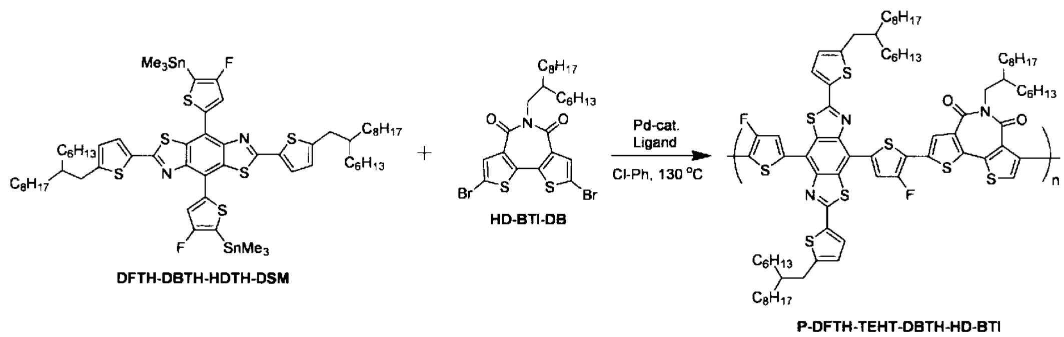


DFTH-DBTH-HDTH-DSM

HD-BTI-DB

P-DFTH-TEHT-DBTH-HD-BTI

The DFTH-DBTH-HDTH-DSM (0.10 g, 0.075 mmol), HD-BTI-DB (46 mg, 0.075 mmol), tris(dibenzylideneacetone)dipalladium (0)-chloroform adduct (3 mg, 3.0 μmol), tris(2-methoxyphenyl)phosphine (4.4 mg, 8.5 μmol), and chlorobenzene (7 mL) were added in a 20 mL flask and reacted at 130°C for 23 hours. Methanol (40 mL) was added to the reaction liquid to precipitate a solid. The solid was obtained through filtering. The obtained solid was subjected to Soxhlet washing (methanol, acetone, and hexane). Then, the solid was subjected to Soxhlet extraction (chloroform) to prepare 87 mg (79%) of P-DFTH-THDH-DBTH-HD-BTI as a black solid.

Ionization potential: 5.36 eV (HOMO -5.36 eV)
GPC measurement result: Mw (weight average molecular weight): 45200
Mn (number average molecular weight): 23500

(Production of Mixed Solution 5 Made from p-Type Semiconductor Compound and n-Type Semiconductor Compound)

As a p-type semiconductor compound, a macromolecular compound having the structure of the P-DFTH-THDH-DBTH-HD-BTI prepared in the above Example 4 was used. As an n-type semiconductor compound, IT4F ((3,9-bis(2-methylene-((3-1,1-dicyanomethylene)-6,7-difluoro)-indanone))-5,5,11,11-tetrakis(4-hexylphenyl)-dithieno[2,3-d:2'3'-d']-s-indaceno[1,2-b:5,6-b']dithiophene (manufactured by 1-Material)) was used. The p-type semiconductor compound and the n-type semiconductor compound were, in a mass ratio of 1:1, dissolved in chlorobenzene. The total concentration of the p-type semiconductor compound and the n-type semiconductor compound was set to 2.0% by mass. The obtained solution was stirred and mixed at a temperature of 100°C for 2 hours or longer on a hot stirrer. The solution having been stirred and mixed was filtered with a 0.45 μm filter to produce a mixed solution 5 made from the p-type semiconductor compound and the n-type semiconductor compound.

Example 5

Synthesis of P-THDT-DC-DBTH-HD-BTI

[Chemical Formula 78]

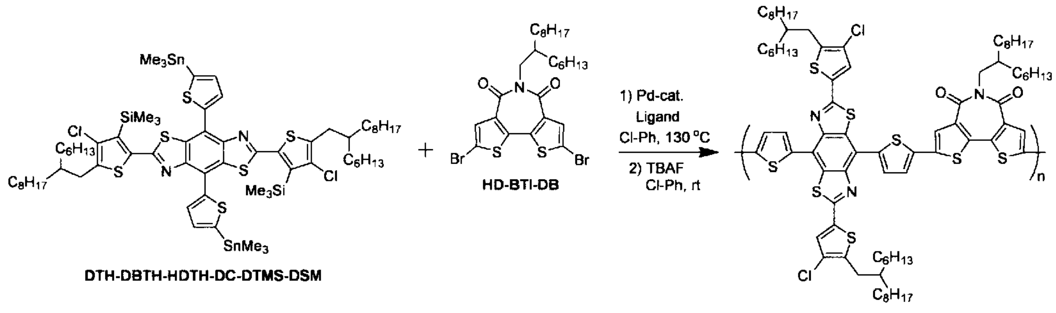


DTH-DBTH-HDTH-DC-DTMS-DSM

HD-BTI-DB

P-THDT-DC-DBTH-HD-BTI

The DTH-DBTH-HDTH-DC-DTMS-DSM (70 mg, 0.046 mmol), HD-BTI-DB (29 mg, 0.046 mmol), tris(dibenzylideneacetone)dipalladium (0)-xdba adduct (2.3 mg, 1.9 μmol), tris(2-methoxyphenyl)phosphine (2.8 mg, 7.9 μmol), and chlorobenzene (3 mL) were added in a 10 mL flask and reacted at 130°C for 23 hours. The reaction liquid was cooled to room temperature, and then tetrabutylammonium fluoride (a solution containing about 1 mol/L of tetrahydrofuran, 0.16 mL, 0.16 mmol) was added, and then the mixture was caused to undergo a reaction at room temperature for 4.5 hours. After the reaction was completed, water was added, and then the reaction product was subjected to extraction twice with chloroform, and then the resulting organic layer was washed with water. Then, the organic layer was filtered and concentrated to obtain a crude product, and then methanol (40 mL) was added to the crude product to precipitate a solid. The solid was obtained through filtering. The obtained solid was subjected to Soxhlet washing (methanol, acetone, and hexane). Then, the solid was subjected to Soxhlet extraction (chloroform) to prepare 38 mg (66%) of P-THDT-DC-DBTH-HD-BTI as a black solid.

Ionization potential: 5.56 eV (HOMO -5.56 eV)
GPC measurement result: Mw (weight average molecular weight): 55700
Mn (number average molecular weight): 21700

(Production of Mixed Solution 6 Made from p-Type Semiconductor Compound and n-Type Semiconductor Compound)

As a p-type semiconductor compound, a macromolecular compound having the structure of the P-THDT-DC-DBTH-HD-BTI prepared in the above Example 5 was used. As an n-type semiconductor compound, IT4F ((3,9-bis(2-methylene-((3-1,1-dicyanomethylene)-6,7-difluoro)-indanone))-5,5,11,11-tetrakis(4-hexylphenyl)-dithieno[2,3-d:2'3'-d']-s-indaceno[1,2-b:5,6-b']dithiophene (manufactured by 1-Material)) was used. The p-type semiconductor compound and the n-type semiconductor compound were, in a mass ratio of 1:1, dissolved in chlorobenzene. The total concentration of the p-type semiconductor compound and the n-type semiconductor compound was set to 2.0% by mass. The obtained solution was stirred and mixed at a temperature of 100°C for 2 hours or longer on a hot stirrer. The solution having been stirred and mixed was filtered with a 0.45 μm filter to produce a mixed solution 6 made from the p-type semiconductor compound and the n-type semiconductor compound.

(Production of Photoelectric Conversion Element)

A glass substrate (manufactured by GEOMATEC Co., Ltd.) patterned with an indium tin oxide (ITO) transparent conductive film (cathode) as an electrode was ultrasonically cleaned with acetone, then ultrasonically cleaned with ethanol, and then, dried through nitrogen blowing. The dried glass substrate was subjected to UV-ozone treatment, and then an electron transport layer was formed. The electron transport layer was formed by applying a 0.5 M zinc acetate-0.5 M aminoethanol/2-methoxyethanol solution onto the glass substrate with use of a spin coater (3000 rpm, 40 seconds) and performing annealing at 175°C for 30 minutes. The glass substrate on which the electron transport layer was formed was sent into a glove box and, in an inert gas atmosphere, spin-coated with the mixed solution 1, the mixed solution 2, the mixed solution 3, the mixed solution 4, or the mixed solution 5 made from the corresponding p-type semiconductor compound and n-type semiconductor compound, and then the mixed solution was annealed or dried under a reduced pressure on a hot plate to form an active layer. Next, a molybdenum oxide as a hole transport layer was vapor-deposited by using a vapor deposition machine. Then, silver as an electrode (anode) was vapor-deposited to produce an inverted-configuration device.

Regarding the obtained inverted-configuration device, photoelectric conversion element evaluation was made through the following procedure by using a solar simulator.

(Method for Evaluating Photoelectric Conversion Element)

The photoelectric conversion element was equipped with a 0.05027-mm square metal mask, and then a solar simulator (OTENTO-SUN III (with an AM 1.5 G filter) having an irradiation intensity of 100 mW/cm$^2$ and manufactured by Bunkoukeiki Co., Ltd.) was used as an irradiation light source, and current-voltage characteristics between the ITO electrode and the silver electrode were measured by using SourceMeter (model 2400 manufactured by Keithley Instruments). From the result of the measurement, a short-circuit current density Jsc (mA/cm$^2$), an open circuit voltage Voc (V), a fill factor FF, the value of Voc×FF, and a conversion efficiency (PCE) (%) were calculated. The short-circuit current density Jsc is a current density at which the voltage value is 0 V. The open circuit voltage Voc is a voltage value at which the current value is 0 mA/cm$^2$. The fill factor FF is a factor indicating an internal resistance and is expressed according to the following expression where Pmax represents the maximum output.

$$FF = Pmax/(Voc \times Jsc)$$

The conversion efficiency (PCE) is expressed according to the following expression.

$$PCE = Jsc \times Voc \times FF$$

As a result, the inverted-configuration device produced by using the above mixed solution 1 had a short-circuit current density (Jsc) of 9.59 mA/cm$^2$, an open circuit voltage (Voc) of 0.92 V, a fill factor (FF) of 0.60, and a conversion efficiency PCE of 5.34%. On the other hand, the inverted-configuration device produced by using the above mixed solution 2 had a short-circuit current density (Jsc) of 16.00 mA/cm$^2$, an open circuit voltage (Voc) of 0.89 V, a fill factor (FF) of 0.55, and a conversion efficiency PCE of 7.87%. On the other hand, the inverted-configuration device produced by using the above mixed solution 3 had a short-circuit current density (Jsc) of 10.47 mA/cm$^2$, an open circuit voltage (Voc) of 0.76 V, a fill factor (FF) of 0.49, and a conversion efficiency PCE of 3.91%. On the other hand, the inverted-configuration device produced by using the above mixed solution 4 had a short-circuit current density (Jsc) of 9.45 mA/cm$^2$, an open circuit voltage (Voc) of 0.75 V, a fill factor (FF) of 0.51, and a conversion efficiency PCE of 3.61%. On the other hand, the inverted-configuration device produced by using the above mixed solution 5 had a short-circuit current density (Jsc) of 11.50 mA/cm$^2$, an open circuit voltage (Voc) of 0.75 V, a fill factor (FF) of 0.50, and a conversion efficiency PCE of 4.31%. On the other hand, the inverted-configuration device produced by using the above mixed solution 6 had a short-circuit current density (Jsc) of 8.23 mA/cm$^2$, an open circuit voltage (Voc) of 1.06 V, a fill factor (FF) of 0.55, and a conversion efficiency PCE of 4.78%.

INDUSTRIAL APPLICABILITY

When an organic semiconductor material including the macromolecular compound according to the present invention is used for an organic electronic device, the open circuit voltage (Voc) of the organic electronic device can be particularly increased, and thus the conversion efficiency PCE thereof can be increased. Therefore, the conversion efficiencies PCE of organic electronic devices such as an organic thin-film solar cell can be increased. Furthermore, the macromolecular compound according to the present invention has a high conversion efficiency PCE, and thus, is useful for, for example, organic electronic devices such as an organic electroluminescence element and an organic thin-film transistor element.

## Claims

**1.** A macromolecular compound comprising a benzobisthiazole structural unit represented by the formula (1):

[Chemical Formula 1]

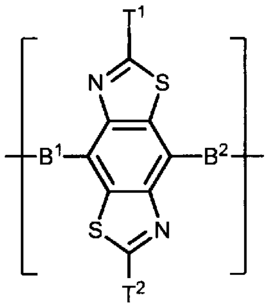

(1)

[in the formula (1),

$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

**2.** The macromolecular compound according to claim 1, wherein $T^1$ and $T^2$ are each a group represented by the following formula (t1):

[Chemical Formula 2]

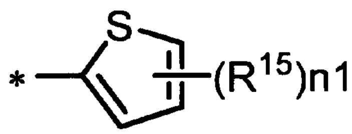

(t1)

[in the formula (t1),

$R^{15}$ represents a hydrogen atom, a hydrocarbon group with a carbon number of 6 to 30, a halogen atom, or a group represented by *-Si($R^{18}$)$_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different;
n1 represents an integer of 1 to 3;
a plurality of $R^{15}$s may be same or different; and
asterisk symbol (*) represents a bond].

**3.** The macromolecular compound according to claim 1 or 2, wherein $B^1$ and $B^2$ are each a group represented by the following formula (b1):

[Chemical Formula 3]

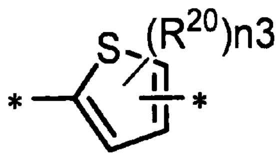

(b1)

[in the formula (b1),

$R^{20}$ represents a halogen atom or a hydrocarbon group with a carbon number of 6 to 30;
n3 represents an integer of 0 to 2;
a plurality of $R^{20}$s may be same or different; and
asterisk symbols (*) each represent a bond, and in particular, asterisk symbol (*) on the left side represents a bond to a benzene ring of a benzobisthiazole compound].

**4.** The macromolecular compound according to any one of claims 1 to 3, wherein the heteroaryl groups represented by $B^1$ and $B^2$ are each substituted by one or more halogen atoms.

**5.** The macromolecular compound according to any one of claims 1 to 4, wherein the heteroaryl groups represented by $T^1$ and $T^2$ are each substituted by one or more halogen atoms.

**6.** The macromolecular compound according to any one of claims 1 to 5, which is a donor-acceptor-type semiconductor polymer.

**7.** An organic semiconductor material comprising the macromolecular compound according to any one of claims 1 to 6.

**8.** A benzobisthiazole compound represented by the formula (5):

[Chemical Formula 4]

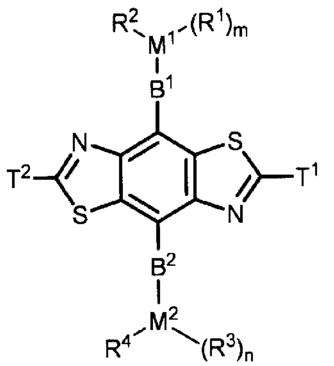

(5)

[in the formula (5),

$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom;
$R^1$ to $R^4$ each independently represent an aliphatic hydrocarbon group with a carbon number of 1 to 6, a hydroxy group, an alkoxy group with a carbon number of 1 to 6, or an aryloxy group with a carbon number of 6 to 10;
$M^1$ and $M^2$ each independently represent a boron atom or a tin atom;
$R^1$ and $R^2$ may form a ring with $M^1$, and $R^3$ and $R^4$ may form a ring with $M^2$;
m and n each represent an integer of 1 or 2; and
when m is 2, a plurality of $R^1$s may be same or different, and when n is 2, a plurality of $R^3$s may be same or different].

**9.** A benzobisthiazole compound represented by the formula (4):

[Chemical Formula 5]

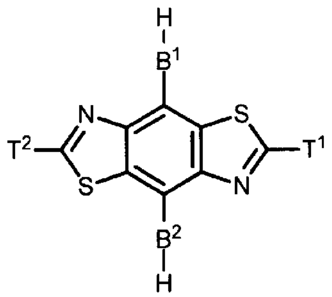

(4)

[in the formula (4),
$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

**10.** A benzobisthiazole compound represented by the formula (3):

[Chemical Formula 6]

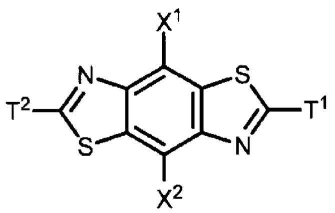

(3)

[in the formula (3),

$T^1$ and $T^2$ represent heteroaryl groups, the heteroaryl groups are optionally each independently substituted by an organosilyl group, a halogen atom, or a hydrocarbon group, and at least one of the heteroaryl groups represented by $T^1$ and $T^2$ is substituted by a halogen atom; and
$X^1$ and $X^2$ each represent a halogen atom].

**11.** A benzobisthiazole compound represented by the formula (2):

[Chemical Formula 7]

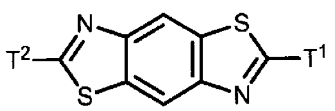

(2)

[in the formula (2),
$T^1$ and $T^2$ represent heteroaryl groups, the heteroaryl groups are optionally each independently substituted by an organosilyl group, a halogen atom, or a hydrocarbon group, and at least one of the heteroaryl groups represented by $T^1$ and $T^2$ is substituted by a halogen atom].

**12.** A thiophene compound represented by the formula (20):

[Chemical Formula 8]

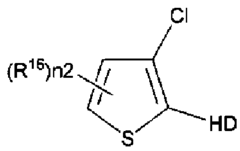

(20)

[in the formula (20),

HD represents a hexyldecyl group;
$R^{16}$s each independently represent a hydrogen atom, a hydrocarbon group with a carbon number of 6 to 30, a halogen atom, or a group represented by $*\text{-Sn}(R^{18})_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different;
n2 represents 1 or 2;
a plurality of $R^{16}$s may be same or different; and
asterisk symbol (*) represents a bond].

**13.** A thiophene compound represented by the formula (21):

[Chemical Formula 9]

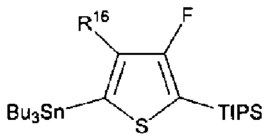

(21)

[in the formula (21),

TIPS represents a triisopropylsilyl group;
BusSn represents a tributyltin group;
$R^{16}$ represents a hydrogen atom, a hydrocarbon group with a carbon number of 6 to 30, a halogen atom, or a group represented by $*\text{-Sn}(R^{18})_3$;
$R^{18}$s each independently represent an aliphatic hydrocarbon group with a carbon number of 1 to 20 or an aromatic hydrocarbon group with a carbon number of 6 to 10, and a plurality of $R^{18}$s may be same or different; and
asterisk symbol (*) represents a bond].

**14.** A production method for the macromolecular compound according to any one of claims 1 to 6, the production method comprising:

using one or more compounds selected from the group consisting of 2,6-diiodobenzo[1,2-d:4,5-d']bisthiazole and 2,6-dibromobenzo[1,2-d:4,5-d']bisthiazole as starting materials; and
going through
a compound represented by the formula (22):

[Chemical Formula 10]

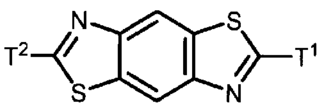

(22)

[in the formula (22),

$T^1$ and $T^2$ represent heteroaryl groups, and the heteroaryl groups are optionally each independently substituted by an organosilyl group, a halogen atom, or a hydrocarbon group];
a compound represented by the formula (23):

[Chemical Formula 11]

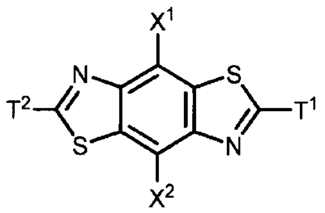

(23)

[in the formula (23),
$T^1$ and $T^2$ represent heteroaryl groups, and the heteroaryl groups are optionally each independently substituted by an organosilyl group, a halogen atom, or a hydrocarbon group; and
$X^1$ and $X^2$ each represent a halogen atom]; and
a compound represented by the formula (4):

[Chemical Formula 5]

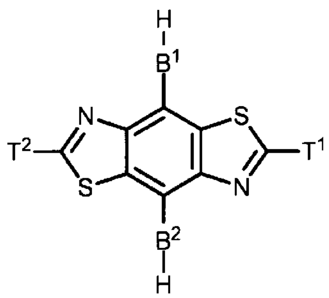

(4)

[in the formula (4),
$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom].

**15.** The production method according to claim 14, comprising the following first step, second step (a), and third step:

the first step: a step of reacting a compound represented by the formula (6) and/or the formula (7):

[Chemical Formula 12]

$$T^1—R^5 \quad (6)$$

$$T^2—R^6 \quad (7)$$

[in the formulae (6) and (7),
$T^1$ and $T^2$ represent heteroaryl groups, and the heteroaryl groups are optionally each independently substituted by an organosilyl group, a halogen atom, or a hydrocarbon group;
$R^5$ and $R^6$ each independently represent a hydrogen atom or $*\text{-}M^3(R^7)_kR^8$;
$R^7$ and $R^8$ each independently represent an aliphatic hydrocarbon group with a carbon number of 1 to 6, a hydroxy group, an alkoxy group with a carbon number of 1 to 6, or an aryloxy group with a carbon number of 6 to 10;
$M^3$ represents a boron atom or a tin atom;

asterisk symbol (*) represents a bond;
$R^7$ and $R^8$ may form a ring with $M^3$;
k represents an integer of 1 or 2; and
when k is 2, a plurality of $R^7$s may be same or different]
with one or more compounds selected from the group consisting of 2,6-diiodobenzo[1,2-d:4,5-d']bisthiazole and 2,6-dibromobenzo[1,2-d:4,5-d']bisthiazole in the presence of a metal catalyst to prepare a compound represented by the formula (22);
the second step (a): a step of reacting a base and a halogenation reagent with the compound represented by the formula (22) to prepare a compound represented by the formula (23); and
the third step: a step of reacting a compound represented by the following formula (8) and/or formula (9) with the compound represented by the formula (23) in the presence of a metal catalyst to prepare a compound represented by the formula (4):

[Chemical Formula 13]

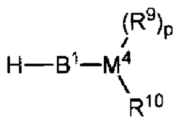
(8)

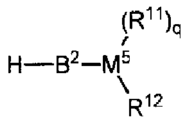
(9)

[in the formulae (8) and (9),
$B^1$ and $B^2$ represent heteroaryl groups, and the heteroaryl groups are optionally each independently substituted by an organosilyl group, a halogen atom, or a hydrocarbon group;
$R^9$ to $R^{12}$ each independently represent an aliphatic hydrocarbon group with a carbon number of 1 to 6, a hydroxy group, an alkoxy group with a carbon number of 1 to 6, an aryl group with a carbon number of 6 to 10, or an aryloxy group with a carbon number of 6 to 10;
$M^4$ and $M^5$ each represent a boron atom, a tin atom, or a silicon atom;
$R^9$ and $R^{10}$ may form a ring with $M^4$, and $R^{11}$ and $R^{12}$ may form a ring with $M^5$;
p and q each represent an integer of 1 or 2; and
when p is 2, a plurality of $R^9$s may be same or different, and when q is 2, a plurality of $R^{11}$s may be same or different].

**16.** The production method according to claim 14, comprising the following first step, second step (b-1), second step (b-2), and third step:

the first step: the same first step as that described above;
the second step (b-1): a step of reacting a base and an organosilylation reagent with the compound represented by the formula (22);
the second step (b-2): a step of reacting a base and a halogenation reagent with the product resulting from the second step (b-1) to prepare a compound represented by the formula (23); and
the third step: the same third step as that described above.

**17.** The production method according to any one of claims 14 to 16, further comprising going through a compound represented by the formula (5):

[Chemical Formula 4]

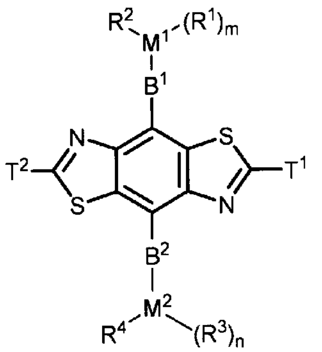

(5)

[in the formula (5),

$T^1$, $T^2$, $B^1$, and $B^2$ represent heteroaryl groups, the heteroaryl groups are optionally each independently substituted by an organosilyl group, a halogen atom, or a hydrocarbon group, and at least one of the heteroaryl groups represented by $T^1$, $T^2$, $B^1$, and $B^2$ is substituted by a halogen atom;
R' to $R^4$ each independently represent an aliphatic hydrocarbon group with a carbon number of 1 to 6, a hydroxy group, an alkoxy group with a carbon number of 1 to 6, or an aryloxy group with a carbon number of 6 to 10;
$M^1$ and $M^2$ each independently represent a boron atom or a tin atom;
$R^1$ and $R^2$ may form a ring with $M^1$, and $R^3$ and $R^4$ may form a ring with $M^2$;
m and n each represent an integer of 1 or 2; and
when m is 2, a plurality of $R^1$s may be same or different, and when n is 2, a plurality of $R^3$s may be same or different].

**18.** The production method according to any one of claims 14 to 17, further comprising the following fourth step:
the fourth step: a step of reacting a base and a tin halide compound with the compound represented by the formula (4) to prepare a compound represented by the formula (5).

**19.** An organic electronic device comprising the organic semiconductor material according to claim 7.

**20.** The organic electronic device according to claim 19, which is an organic thin-film solar cell.

**21.** A solar cell module comprising the organic electronic device according to claim 20.

[FIG. 1]

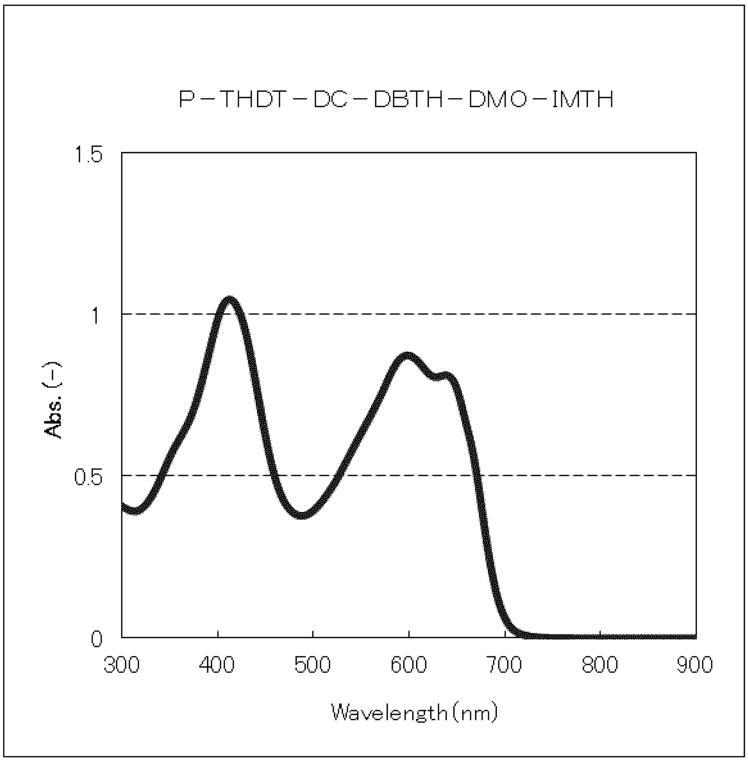
P－THDT－DC－DBTH－DMO－IMTH
1.5
1
0.5
0
Abs. (-)
300
400
500
600
700
800
900
Wavelength (nm)

[FIG. 2]

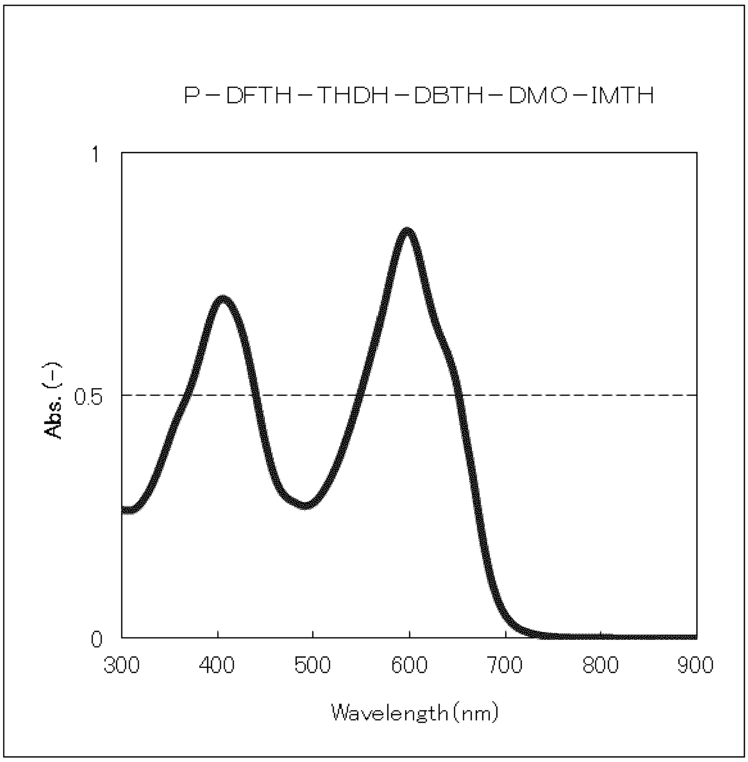
P－DFTH－THDH－DBTH－DMO－IMTH
1
0.5
0
Abs. (-)
300
400
500
600
700
800
900
Wavelength (nm)

[FIG. 3]

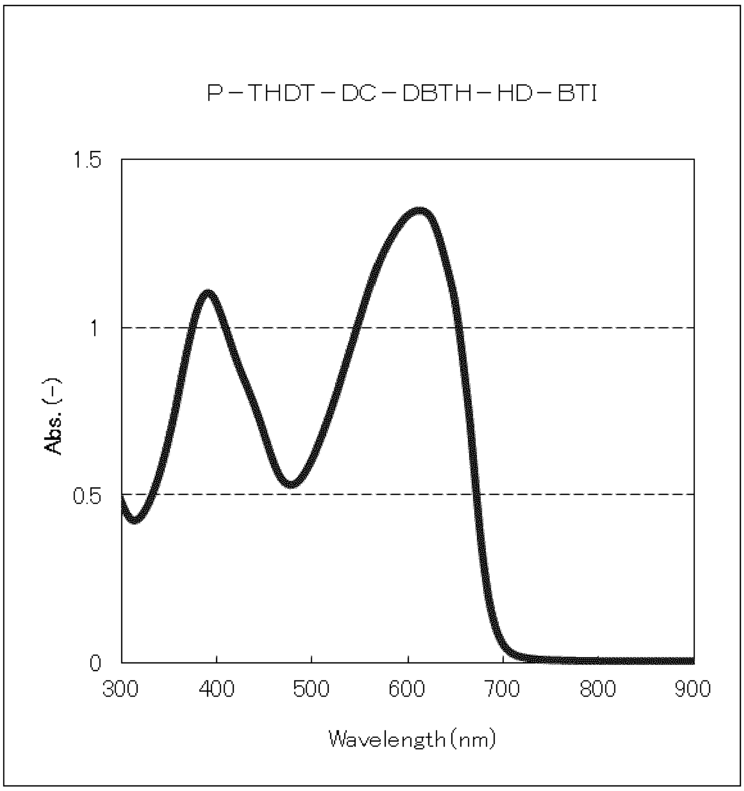
P－THDT－DC－DBTH－HD－BTI
1.5
1
0.5
0
Abs. (-)
300
400
500
600
700
800
900
Wavelength (nm)

**INTERNATIONAL SEARCH REPORT**

International application No. **PCT/JP2022/036770**

**A. CLASSIFICATION OF SUBJECT MATTER**

***C08G 61/12***(2006.01)i; ***C07B 61/00***(2006.01)i; ***C07D 513/04***(2006.01)i; ***C07F 7/10***(2006.01)i; ***C07F 7/22***(2006.01)i; ***H10K 30/50***(2023.01)i

FI: C08G61/12; C07F7/22 N; C07F7/10 V; H01L31/04 152B; H01L31/04 152G; C07D513/04 325; C07B61/00 300; C07F7/22 Q CSP

According to International Patent Classification (IPC) or to both national classification and IPC

**B. FIELDS SEARCHED**

Minimum documentation searched (classification system followed by classification symbols)

C08G61/12; C07B61/00; C07D513/04; C07F7/10; C07F7/22; H01L51/46

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2016/121589 A1 (TOYOBO CO., LTD.) 04 August 2016 (2016-08-04) paragraphs [0099], [0103], [0116] | 9 |
| Y | paragraphs [0055], [0056] | 13 |
| A | | 1-8, 10-12, 14-21 |
| X | DESSI, A. et al. Organic Chromophores Based on a Fused Bis-Thiazole Core and Their Application in Dye-Sensitized Solar Cells. European Journal of Organic Chemistry. 2013, vol. 2013, no. 10, pp. 1916-1928 p. 1919, scheme 2 | 11 |
| A | | 1-10, 12-21 |
| X | JP 2019-135274 A (SOKEN CHEM. & ENG. CO., LTD.) 15 August 2019 (2019-08-15) paragraphs [0059], [0060] | 11 |
| A | | 1-10, 12-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 November 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

INTERNATIONAL SEARCH REPORT

International application No.
**PCT/JP2022/036770**

**C. DOCUMENTS CONSIDERED TO BE RELEVANT**

| Category* | Citation of document, with indication, where appropriate, of the relevant passages | Relevant to claim No. |
|---|---|---|
| X<br><br>A | JP 2020-98899 A (TOYO INK SC HOLDINGS CO., LTD.) 25 June 2020 (2020-06-25)<br>paragraphs [0095], [0096] | 11<br><br>1-10, 12-21 |
| X<br><br>A | WO 2020/187867 A1 (RAYNERGY TEK INC.) 24 September 2020 (2020-09-24)<br>pages 124, 125, example 7 | 12<br><br>1-11, 13-21 |
| Y<br><br><br><br>A | FAN, B. et al. Tailoring Regioisomeric Structures of π-Conjugated Polymers Containing Monofluorinated π-Bridges for Highly Efficient Polymer Solar Cells. ACS Energy Letters. 2020, vol. 5, no. 6, pp. 2087-2094<br>p. 2088, scheme 1 | 13<br><br><br><br>1-12, 14-21 |
| A | JP 2019-43878 A (TOYOBO CO., LTD.) 22 March 2019 (2019-03-22)<br>entire text | 1-21 |

Form PCT/ISA/210 (second sheet) (January 2015)

**INTERNATIONAL SEARCH REPORT**
**Information on patent family members**

International application No.
**PCT/JP2022/036770**

| Patent document cited in search report | Publication date (day/month/year) | Patent family member(s) | Publication date (day/month/year) |
|---|---|---|---|
| WO 2016/121589 A1 | 04 August 2016 | (Family: none) | |
| JP 2019-135274 A | 15 August 2019 | TW 201811857 A | |
| JP 2020-98899 A | 25 June 2020 | EP 3902021 A1<br>tables 36, 37<br>CN 113228318 A<br>TW 202033724 A | |
| WO 2020/187867 A1 | 24 September 2020 | JP 2022-525907 A<br>paragraph [0283]<br>US 2022/0173321 A1<br>paragraph [0383]<br>CN 113631627 A | |
| JP 2019-43878 A | 22 March 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2017157782 A